(19) Europäisches Patentamt
European Patent Office
Office européen des brevets

(11) EP 0 663 912 B1

(12) **EUROPEAN PATENT SPECIFICATION**

(45) Date of publication and mention of the grant of the patent:
**12.12.2001 Bulletin 2001/50**

(21) Application number: **93923826.7**

(22) Date of filing: **08.10.1993**

(51) Int Cl.⁷: **C07D 401/12**, C07D 403/12, C07D 411/12, A61K 31/425, A61K 31/44, A61K 31/445, A61K 31/47, A61K 31/495, A61K 31/50, A61K 31/505, C07D 417/12, C07D 487/04, C07D 453/02

(86) International application number:
**PCT/US93/09662**

(87) International publication number:
**WO 94/08992 (28.04.1994 Gazette 1994/10)**

(54) **HETEROCYCLIC ETHER COMPOUNDS THAT ENHANCE COGNITIVE FUNCTION**
HETEROCYCLISCHE ETHER-DERIVATE ZUR VERSTÄRKUNG DER COGNITIVEN FUNKTIONEN
COMPOSES HETEROCYCLIQUES D'ETHER AMELIORANT LA FONCTION COGNITIVE

(84) Designated Contracting States:
**AT BE CH DE DK ES FR GB GR IE IT LI LU NL PT SE**

(30) Priority: **09.10.1992 US 959005**

(43) Date of publication of application:
**26.07.1995 Bulletin 1995/30**

(73) Proprietor: **ABBOTT LABORATORIES**
**Abbott Park, Illinois 60064-3500 (US)**

(72) Inventors:
- **ABREO, Melwyn A.**
**Imperial Beach, CA 91932 (US)**
- **GUNN, David E.**
**Waukegan, Illinois 60087 (US)**
- **LIN, Nan-Horng**
**Mundelein, IL 60060 (US)**
- **ELLIOTT, Richard L.**
**Grayslake, IL 60030 (US)**
- **GARVEY, David S.**
**Lake Forest, IL 60045 (US)**
- **LEBOLD, Suzanne A.**
**Chicago, IL 60614 (US)**
- **WASICAK, James T.**
**Waterford, WI 53185 (US)**

(74) Representative: **Modiano, Guido, Dr.-Ing. et al**
**Modiano, Josif, Pisanty & Staub,**
**Baaderstrasse 3**
**80469 München (DE)**

(56) References cited:
**US-A- 4 643 995**
**US-A- 4 929 625**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 663 912 B1

## Description

This application is a continuation-in-part of copending U.S. patent application Serial No. 07/959,005, filed October 9, 1992.

### TECHNICAL FIELD

This invention relates to heterocyclic ether compounds and pharmaceutical compositions thereof which are cholinergic ligands selective for neuronal nicotinic cholinergic channel receptors; to methods for preparing these compounds; and to the use of these compounds: in treating cognitive, neurological and mental disorders, such as dementias and anxiety, which are characterized by decreased cholinergic function, in treating attention-deficit disorder, in treating or preventing withdrawal symptoms caused by the cessation of chronic or long term use of tobacco products, and in ameliorating the symptoms of anxiety and frustration associated with withdrawal of other addictive substances such as, for example, cocaine, diazepam or alcohol.

### BACKGROUND OF THE INVENTION

Dementia has been widely recognized as a very serious health problem. Alzheimer's disease, which has been identified by the National Institutes of Aging as accounting for more than 50% of dementia in the elderly, is also the fourth or fifth leading cause of death in Americans over 65 years of age. Four million Americans, 40% of Americans over age 85 (the fastest growing segment of the U.S. population), have Alzheimer's disease. Twenty-five percent of all patients with Parkinson's disease also suffer from Alzheimer's disease-like dementia. And in about 15% of patients with dementia, Alzheimer's disease and multi-infarct dementia coexist The third most common cause of dementia, after Alzheimer's disease and vascular dementia, is cognitive impairment due to organic brain disease related directly to alcoholism, which occurs in about 10% of alcoholics.

The precise molecular lesion(s) that contribute to the morphological and functional deficits associated with dementia is unclear despite intensive research efforts over the last decade. However, the most consistent abnormality for Alzheimer's disease, as well as for vascular dementia and cognitive impairment due to organic brain disease related to alcoholism, is the degeneration of the cholinergic system arising from the basal forebrain (BF) to both the cortex and hippocampus (Bigl *et al.*, in Brain Cholinergic Systems, M. Steriade and D. Biesold, eds., Oxford University Press, Oxford, 1990, pp. 364-386). In particular, neurochemical evidence from the brains of patients afflicted with Alzheimefs disease has revealed reliable decreases in markers of cholinergic neuronal function (Perry *et al., Br. Med. J*. **1978**, *2*: 1457; Reisine *et al., Brain Res*. **1978,** *159*:477; Coyle *et al.*, *Science* **1983,** *219*:1184; and McGeer *et al.*, *Neurology* **1984**, *34*:741). While there are a number of other neurotransmitter systems affected by Aizheimer's disease (Davies, *Med. Res. Rev*. **1983**, *3*:221), the relative occurrence of such abnormalities is less consistent or the effect is less profound than the decreases in these cholinergic neuronal function markers. More specifically, substantial reductions (30-50%) in nicotinic cholinergic channel receptors have been consistently reported in the brains of patients with Alzheimer's disease or Parkinson's disease (Kellar *et al., Brain Res.,* **1987,** *436*:62; and Whitehouse *et al*., *Neurol.* **1988**, *38*:720), whereas changes in muscarinic cholinergic receptors are less remarkable and more dependent on receptor subtype.

However, degeneration of the cholinergic neurotransmitter system is not limited to individuals suffering from dementia. It has also been seen in otherwise healthy aged adults and rats. Decreases in cholinergic markers in the basal forebrain, decreases in cortical activities of the biosynthetic and degradative enzymes for acetylcholine, decreases in the ability to release acetylcholine from tissue slices, and decreases in numbers of cortical nicotinic cholinergic channel receptors have all been reported in such otherwise healthy aged individuals (for a review, see Giacobini, *J. Neurosci. Res.* **1990,** *27*:548). Moreover, for those cholinergic neurons that remain, aging may cause a decrease in the temporal fidelity of existing impulse flow from the basal forebrain to the cortex (Aston-Jones *et al*., *Brain Res.* **1985,** *325*:271). Consistent with these findings are pharmacological studies suggesting that cholinergic mechanisms are, at least in part, responsible for the memory disturbances in aged animals and humans not suffering from Alzheimer's disease (Drachman and Leavitt, *Arch. Neurol*. **1974,** *30*:113; Bartus *et al*., *Science* **1982,** *217*:408).

Other clinical correlates associated with the neurodegenerative process of Alzheimer's disease include decreases in regional cerebral blood flow and cerebral glucose utilization, in regions which largely parallel the areas where cholinergic deficits occur (Ingvar and Risberg, *Exp. Brain Res.,* **1962,** 3:195; Ingvar *et al.,* Aging: Alzheimer's Disease, Senile Dementia and Related Disorders, Vol. 7 , R. Katzman, R.D. Terry, and K.L Bick, eds., Raven Press, 1978, p. 203; and Dastur, *J. Cerebral Blood Flow & Metabol.,* **1985,** *5*:1). In fact, it has been suggested that routine measurement of cerebral blood flow may be a useful procedure in evaluating patients suspected of having dementia, and of having Alzheimer's disease in particular.

Conflicting reports exist regarding the effect of aging on resting cerebral blood flow and cerebral glucose

utilization in "normal, healthy" aged humans (Dastur, *J. Cerebral Blood Flow & Metabol*. **1985,** *5*:1) and rats (Smith *et al., Brain* **, 1980,** *103*:351; and Buchweitz-Milton and Weiss, *Neurobiol. Aging,* **1987,** *8*:55). Although decreases in cerebral blood flow and cerebral glucose utilization are generally reported in aged populations, it has been suggested that these decreases are secondary to other ongoing cerebral dysfunctions. Nonetheless, deficiencies in metabolic and cerebrovascular responses to pharmacologic and physiologic perturbation are consistently reported. Of particular interest is the recent finding in rats that increases in cerebral blood flow elicited by electrical stimulation of the basal forebrain shows age-related impairments (Linville and Arneric, *Soc. Neurosci. Abstract,* **1989,** *15*:17.5). Indeed, studies that compare the degree of learning impairment with the degree of reduced cortical cerebral blood flow in aged rats show a good correlation (Berman *et al., Neurobiol. Aging,* **1988,** *9*:691).

Recent clinical evidence suggests that the characteristic perfusion abnormality observed in Alzheimer's disease patients reflects regional nicotinic cholinergic deficits (Prohovnik, *Neurobiol. Aging*, **1990**, *11*:262). In particular, mecamylamine, a centrally-acting nicotinic receptor antagonist, reduces resting cortical perfusion in the parietotemporal cortex of humans, the area of the cortex most consistently found to be impaired in functional brain imaging of Alzheimer's disease patients. In agreement with this finding, regulation of cerebral blood flow in the frontoparietal cortex, governed by the basal forebrain, is also dependent upon nicotinic mechanisms in the rat (Arneric, *J. Cerebral Blood Flow & Metabol*., **1989,** *9* (Suppl. 1): S502).

Chronic alcoholism, more particularly, the resultant organic brain disease, like Alzheimer's disease and normal aging, is also characterized by diffuse reductions in cortical cerebral blood flow in those brain regions where cholinergic neurons arise (basal forebrain) and to which they project (cerebral cortex) (Lofti & Meyer, *Cerebrovasc. and Brain Metab. Rev,* **1989,** *1:2*). Moreover, of all the neurotransmitter systems studied, the neurotoxic effects of alcohol on the cholinergic system are thought to be the most important.

Intuitively, regardless of specific etiologic process, therapies directed towards enhancing cognitive processing would be contingent upon maintaining a well-regulated balance between adequate cerebral blood flow, cerebral glucose utilization and cholinergic neurotransmission arising from the basal forebrain.

Pilot clinical studies suggest that nicotine may be useful for the acute treatment of deficits in attention and information processing associated with Alzheimer's disease (Sahakian *et al., Brit. J. Psych*., **1989,** *154*:797; Newhouse *et al*., *Psychopharmacol*., **1988,** *95*:171). It has been shown that both acutely- and chronically-administered nicotine enhances cognitive function in rats (Levin *et al*., *Behav. Neural Biol.,* **1990**, *53*:269), an effect that is also observed in aged animals (Cregan *et al*., *Soc. Neurosci. Abstract*, **1989**, *15*: 2952). Anecdotal evidence suggests a negative correlation between smoking by an individual and the likelihood of the individual acquiring Alzheimer's disease. These findings are supported by additional animal studies demonstrating a neuroregenerative/neuroprotective action of chronically-administered nicotine on both neuronal and vascular functions following hemitransection or MPTP-induced destruction of the nigro-striatal dopamine system (Janson *et al*., *Prog. Brain Res*., **1989**, *79*:257; and Owman *et al*., *Prog. Brain Res*., **1989**, *79*:267). Interestingly, in contrast to the classical down-regulation of receptors typically seen with receptor agonists, chronic nicotine administration up-regulates (50-100%) the number of receptors without affecting affinity (Benwell *et al*.,*J. Neurochem*., **1988**, *50*:1243). This effect occurs both in humans and in smaller animals such as rats (Lapchack *et al*., *J. Neurochem*., **1989**, *52*:483).

Existing cholinergic channel agonists, however, are therapeutically sub-optimal. This is due to unfavorable pharmacokinetics (e.g., with arecoline and nicotine), poor potency and lack of selectivity (e.g., with RS-86), poor CNS penetration (e.g., with carbachol) or poor oral bioavailability (e.g., with nicotine). RS-86, for example, has similar affinity for cholinergic receptors located in the heart and in cortical tissues and is a full agonist at cardiac receptors, whereas it is only a partial agonist at cortical receptors (S.B. Freedman, *British Journal of Pharmacology,* **1986,** *87*: 29P). In addition, known agents have many unwanted central agonist actions, including hypothermia, hypolocomotion and tremor and peripheral side effects, including miosis, lacrimation, defecation and tachycardia (Benowitz *et al*., in: Nicotine Psychopharmacology, S. Wonnacott, M.A.H. Russell, & I.P. Stolerman, eds., Oxford University Press, Oxford, **1990,** pp. 112-157; and M. Davidson, *et al*., in Current Research in Alzheimer Therapy, E. Giacobini and R. Becker, ed.; Taylor & Francis: New York, **1988;** pp 333-336).

In addition to treating decline in cognitive ability by improving cholinergic function and cerebral blood flow, it is also desirable to symptomatically treat the other mental disorders accompanying the earlier stages of Alzheimer's disease. Anxiolytics have been used to treat the severe agitation that most Alzheimer's patients experience with the initial loss of memory (INPHARMA, March 16, **1991,** pg 20). In fact, the use of anxiolytics has become an important aspect of treatment strategies for Alzheimer's disease (Schmidt *et al*., *Drug Dev. Res*., **1988,** *14*:251). Nicotine is known to have anxiolytic properties (Pomerieau *et al*., *Addictive Behaviors,* **1984**, *9*:265) and, therefore, nicotine or selective nicotine agonists (*i.e*., activators) may be useful in the treatment of the anxiety associated with dementias, such as Alzheimer's disease.

Other situations where beneficial therapeutic outcome may be achieved or improved through administration of nicotine or a cholinergic channel activator, because of the anxiolytic properties of these agents, include attentional deficit disorder and drug withdrawal.

Attention-deficit disorder (ADD), with or without hyperactivity, is a behavioral disorder characterized by distractibility and impulsiveness. Children with this disorder are handicapped by their inability to concentrate and control their impulsivity, especially in settings requiring sustained attention, for example, in school. While a cure for this disorder has not been found, stimulants, such as pemoline, have been used successfully in management of the behavioral manifestations of ADD. Nicotine, because of its ability to improve concentration and task performance (F.T. Etscorn, U.S. Patent 4,597,961, issued July 1, 1986; and D.M. Warburton and K. Wesnes in Smoking Behavior, R.E. Thornton, ed., Churchill-Livingston, Edinburgh, **1978,** pp. 19-43) is also potentially useful in treating ADD.

Tobacco use, especially cigarette smoking, has long been recognized as a major factor leading to disease and death. Approximately 4,000 by-products of combustion, many of which are known carcinogens, have been found in cigarette smoke. Of the three most-studied constituents of cigarette smoke, two, tars and carbon monoxide, have been found to cause or exacerbate numerous life-threatening disorders. Tars are most often implicated in the induction of lung, larynx, oral cavity, esophageal and other cancers, and are also thought to be responsible for respiratory diseases, including pulmonary emphysema, chronic bronchitis and smokers' respiratory syndrome. Carbon monoxide, on the other hand, combines with hemoglobin in the blood thereby decreasing the ability of the blood to carry oxygen, and it has also been implicated as a causative agent in the development of coronary artery disease and arteriosclerosis. The third highly-studied, and the most pharmacologically-active substance, in tobacco products is nicotine, which is the reinforcing agent responsible for maintaining tobacco dependency and therefore exposing smokers to other threats of tobacco use (J.H. Jaffe in Nicotine Pharmacology: Molecular, Cellular and Behavioral Aspects, S.Wonnacott, M.A. H. Russell and I.P. Stolerman, eds., Oxford Science Publications, Oxford, 1990, pp. 1-37).

The nicotine withdrawal syndrome associated with smoking cessation is characterized by craving for nicotine, irritability, frustration or anger, anxiety, difficulty concentrating, restlessness, decreased heart rate and increased appetite and weight gain. Nicotine has, not surprisingly, been found to ease the withdrawal experienced by those attempting to break tobacco dependencies. As early as 1942, Johnston reported (L Johnston, *Lancet*, **1942,** *2*:742) that injections of nicotine relieved the withdrawal symptoms experienced by cigarette smokers when they stopped smoking. More recently, in double-blind studies, nicotine was far superior to a placebo in suppressing or preventing the appearance of many of the signs and symptoms of withdrawal (J.R. Hughes *et al., Psychopharmacology,* **1984,** *83*:82-7; N. G. Schneider *et al., Addictive Behavior,* **1984,** *9*:149-56; R.J. West *et al*., *Journal of Addiction*, **1984,** *79*:215-9; K.O. Fagerstrom in Nicotine Replacement: a Critical Evaluation , O.F. Pomperieau and C.S. Pomperleau, eds., Alan R. Liss, Inc., New York, 1988, pp. 109-28, ; and J.E. Henningfield and D.R. Jasinski, *ibid,* pp.35-61). Irritability and impatience were shown to have been reduced in at least five independent controlled studies, while anxiety and difficulty concentrating were shown to have been reduced in at least two studies. Other smoking-withdrawal symptoms for which nicotine was shown to have been significantly more effective than a placebo in relieving the condition in at least one study include depression, hunger, somatic complaints, and sociability. Nicotine has also been found to be effective in reducing anger, irritability, frustration and feelings of tension, while increasing the ability to focus upon the completion of tasks, without causing general response depression, drowsiness or sedation (R.R. Hutchinson *et al.,* U.S. Patent 3,879,794, issued March 11, 1975).

One approach to alleviating the symptoms of tobacco withdrawal has been to develop more efficient methods of delivering nicotine, itself, for example, in transdermal patches (F.T. Etscorn, U.S. Patent 4,597,961, issued July 1, 1986). The major problem with this approach is the non-selective effects of nicotine and in particular, the stimulant effects of increasing cardiac workload and oxygen demand that nicotine has on the heart . A selective cholinergic channel activator would be expected to be equally efficacious in relieving withdrawal symptoms with fewer cardiovascular liabilities.

Withdrawal from addictive substances in general, regardless of which particular agent is withdrawn, is a traumatic experience characterized by anxiety and frustration. These emotional disturbances contribute to failure in therapy and, consequently, to a return to substance dependence. Although ameliorating these symptoms, including reducing anger, irritability, frustration and feelings of tension, does not eliminate the craving for the withdrawn drug, any agent improving the individual's ability to cope and to concentrate should vastly improve the chances of successfully completing withdrawal treatment.

It has now been discovered that compounds according to this invention are selective and potent cholinergic channel activators or ligands useful in treating these problems.

Various heterocyclic compounds with analgesic and hypotensive activities are encompassed by the generic disclosure of Scheffler *et al*. (U.S. Patent 4,643,995), including a 2-pyrrolidine compound substituted with a 2-pyridineoxymethylene group, specifically,

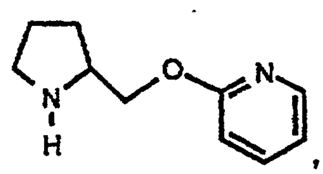

which is the 2-pyridyl analog of a 3-pyridyl compound of the instant invenlian. Tomioka *et al.* (*Chem. Pharm. Bull.,* **1990,** 38:2133-5) were the first to actually prepare this compound (CAS RN=130894-09-6), but they disclose a use of the compound only as a ligand for chiral hydroxylation reactions.

Certain other 2-pyridyl compounds are disclosed *inter alia* by Engel *et al.* in U.S. Patent 4,946,836 as having analgesic activity and possessing the structure:

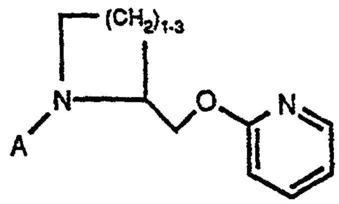

wherein A is $-CO-CHR^3-NH_2$, in which $R^3$ may be, among many other larger groups, hydrogen or a $C_1$-$C_6$-alkyl group. However, compounds which differ by their 2-pyridine attachment as compared with the 3-pyridine attachment of the compounds of the present invention, namely,

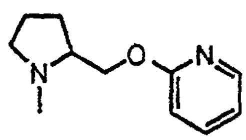

and

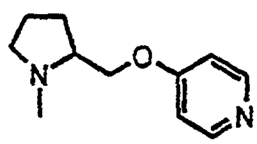

have $K_i$'s in binding assays of 504 nM and >1000 nM, respectively, well below the binding of the 3-pyridine analog of the present invention. The positional isomers according to the present invention possess unexpected properties and utilities from both the compounds of Engel *et al*. and the other adjoining positional isomer, and are not disclosed or in any way suggested by the Engel *et al*. reference.

Various other compounds having a pyrrolidine or azetidine substituted in the 3-position with a heterocycloxy group have been disclosed (cf., U.S.Patents 4,592,866 to A.D. Cale; 4,705,853 to A.D. Gale; 4,956,359 to Taylor *et al*.; and 5,037,841 to Schoehe *et al*.).

SUMMARY OF THE INVENTION

The present invention is directed to novel heterocyclic ether compounds of the formula:

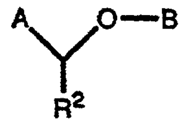

and pharmaceutically-acceptable salts or prodrugs thereof, wherein A, B and $R^2$ are specifically defined, to pharmaceutical compositions comprising a therapeutically-effective amount of these compounds and a pharmaceutically-acceptable carrier or diluent, as well as a method of treating cognitive, neurological and mental disorders characterized by decreased cholinergic function, such as, for example, attention-deficit disorder, dementias, and anxiety associated with cognitive impairment and substance abuse withdrawal.

DETAILED DESCRIPTION OF THE INVENTION

The selective and potent novel heterocyclic ether compounds of the present invention, which are selective and potent ligands at cholinergic channel receptors, are alkyl compounds substituted at the 1-position with a heteroaryloxy group and also substituted at the 1-position with an azacycloalkyl or azabicycloalkyl group, wherein the attachment of the azacycloalkyl or azabicycloalkyl group to the alkyl center is at a position alpha to the nitrogen atom of the azacycloalkyl or azabicycloalkyl group, and are represented by formula (I):

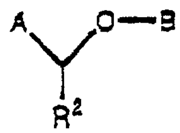 (I),

wherein:

A is selected from the group consisting of:

(i) 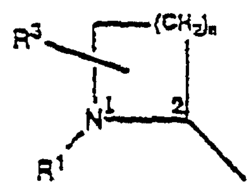 ,

wherein n is 1, 2. or 3;

$R^1$ is H or $C_1$-$C_6$-alkyl; and
$R^3$ is H or, when n is 2, $R^3$ is any of H,
one substituent at the 4-position of the N- containing ring and is O-W, wherein W is H, $C_1$-$C_3$-alkyl, $CH_2OH$, $CH_2O$-methyl, Br, Cl, or F, or
two substituents, one of which being a substituent at the 4-position of the N- containing ring and being O-W wherein W is as defined above, and the other one being a substituent at the 5-position of the N-containing ring and being $C_1$-$C_3$-alkyl.

(ii) 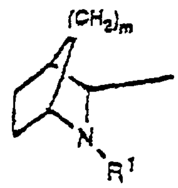,

wherein m is 1 or 2 and $R^1$ is as defined above:

(iii) 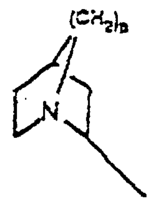,

wherein p is 1 or 2;

(iv) 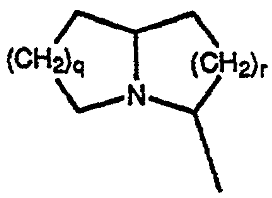,

wherein q and r are independently 0, 1 or 2, except that q and r may not concurrently be 0; and

(v) 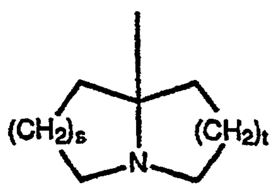,

wherein s and t are independently 0, 1 or 2, except that s and t may not concurrently be 0;

$R^2$ is H or $C_1$-$C_6$-alkyl; and
B is selected from the group consisting of:

(i) 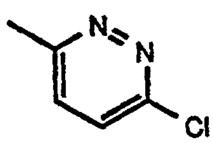;

(ii) 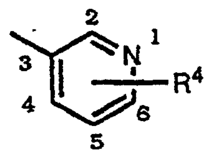 ,

wherein $R^4$ is H or is mono-substituted at the 2-, 4-, 5- or 6-position with hydroxyl, $C_1$-$C_3$-alkyl or $C_1$-$C_3$-alkoxy, as defined below; or is mono-substituted at the 4-, 5- or 6-position with Br, Cl or F;

(iii) 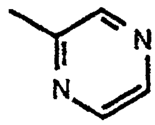 ;

(iv) 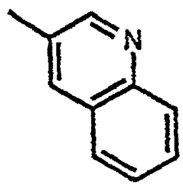 ;

(v) 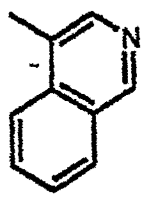 ;

(vi) 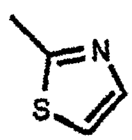 ;

and

(vii) 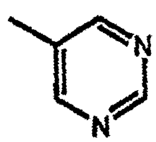 ;

with the provisos that when A is selected from option (iii), then B must be selected from options (ii) and (vi); or when B is selected from option (ii) with $R^4$ being 2-methyl, and A is selected from option (i), then $R^1$ must be H; or a pharmaceutically-acceptable salt or

prodrug thereof.

Preferred embodiments of the present invention are represented by compounds of formula (I), wherein

A is

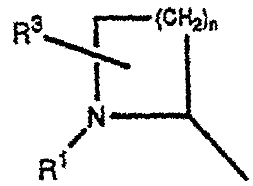

,

wherein n is 1 or 2, and $R^1$ and $R^3$ are as defined above;

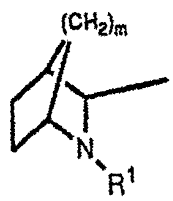

,

wherein m and $R^1$ are as defined above; or

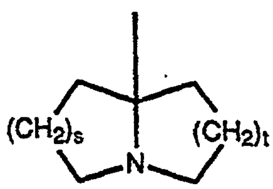

,

wherein s and t are as defined above; and

B is

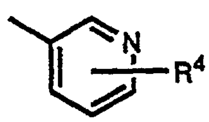

,

wherein $R^4$ is as defined above, or

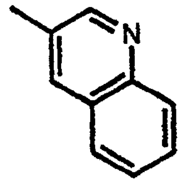
;

and

$R^1$ and $R^2$ are H, or $R^1$ is methyl and $R^2$ is H;

or a pharmaceutically-acceptable salt or prodrug thereof.

More preferred embodiments of the present invention are represented by compounds of formula (I), wherein

A is

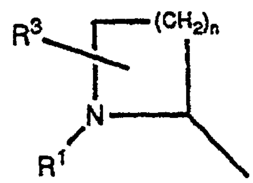
,

wherein n is 1 or 2, $R^3$ is H, and $R^1$ and $R^2$ are H, or $R^1$ is methyl and $R^2$ is H; and
B is

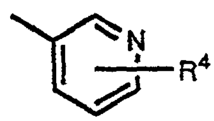
,

wherein $R^4$ is as defined above; and

or a pharmaceutically-acceptable salt or prodrug thereof.

The following are representative of the novel compounds of the present invention:

3-(1-Methy(-2-(R)-pyrrolidinylmethyloxy)pyridine;
3-(2-(R)-Pyrrolidinylmethyloxy)pyridine;
2-(1-Methyl-2-(S)-pyrrolidinylmethyloxy)pyrazine;
3-(1-Methyl-2-(S)-pyrrolidinylmethyloxy)pyridine;
2-(1-Methyl-2-(R)-pyrrolidinylmethyloxy)-6-chloropyridazine;
1-(Methyl)-2-(S)-(1-(3'-pyridyloxy)ethyl)pyrrolidine;
2-(2-(S)-Azetidinylmethyloxy)pyrazine;
2-(1-Methyl-2-(S)-azetidinylmethyloxy)pyrazine;
3-(2-(S)-Azetidinylmethyloxy)pyridine;
3-(1-Methyl-2-(S)-azetidinylmethyloxy)pyridine;
2-(1-Methyl-2-(S)-pyrrolidinylmethyloxy)thiazole;
2-(1-Methyl-2-(S)-pyrrolidinylmethyloxy)-6-chloropyridazine;
6-Chloro-3-(1-Methyl-2(S)-methyloxyazetidinyl)pyridazine;
3-(2-(S)-Pyrrolidinylmethyloxy)pyridine;
5-Chloro-3-(2-(S)-pyrrolidinylmethyloxy)pyridine;
5-Chloro-3-(1-methyl-2-(S)-pyrrolidinylmethyloxy)pyridine;

2-Methyl-3-(2-(S)-pyrrolidinylmethyloxy)pyridine;
6-Methyl-3-(2-(S)-pyrrolidinylmethyloxy)pyridine;
6-Methyl-3-(1-methyl-2-(S)-pyrrolidinylmethyloxy)pyridine;
4-Bromo-3-(2-(S)-pyrrolidinylmethyloxy)pyridine;
4-Bromo-3-(1-methyl-2-(S)-pyrrolidinylmethyloxy)pyridine;
3-(1-Methyl-5-methyl-2-(S)-pyrrolidinylmethyloxy)pyridine;
3-(*trans*-1-Methyl-4-hydroxy-2(S)-pyrrolidinylmethyloxy)pyridine;
3-(*trans*-1,4-Dimethyl-2(S)-pyrrolidinylmethyloxy)pyridine;
3-(*trans*-1-Methyl-4-ethyl-2(S)-pyrrolidinylmethyloxy)pyridine;
3-(1-Methyl-2-pipecolinylmethyloxy)pyridine;
4-Methyl-3-(2-(S)-pyrrolidinylmethyloxy)pyridine;
5-Bromo-3-(1-methyl-2-(S)-pyrrolidinylmethyloxy)pyridine;
2-Methyl-3-(2-(S)-azetidinylmethyioxy)pyridine;
3-(1-Methyl-2-(S)-pyrrolidinylmethyloxy)quinoline;
4-(1-Methyl-2-(S)-pyrrolidinylmethyloxy)isoquinoline;
6-Chloro-3-(1-(8-pyrrolizidinyl)methyloxy)pyridazine;
3-(1 -(8-Pyrrolizidinyl)methyloxy)pyrazine;
2-(1-(8-Pyrrolizidinyl)methyloxy)thiazole;
(1R, 4S)-3-(R)-(2-Thiazoloxymethyl)-N-methyl-2-azabicyclo[2.2.1]heptane;
(1S,4R)-3-(S)-(2-Thiazoloxymethyl)-N-methyl-2-azabicyclo[2.2.1]heptane;
(1S,4R)-3-(S)-(3-Pyridyloxymethyl)-N-methyl-2-azabicyclo[2.2.1]heptane;
(1R,4S)-3-(R)-(3-Pyridyloxymethyl)-N-methyl-2-azabicyclo[2.2.1]heptane;
5-Chloro-3-(2-(R)-pyrrolidinylmethyloxy)pyridine;
5-Chloro-3-(1-methyl-2-(R)-pyrrolidinylmethyloxy)pyridine;
2-Methyl-3-(2-(R)-pyrrolidinylmethyloxy)pyridine;
6-Methyl-3-(1-methyl-2-(R)-pyrrolidinylmethyloxy)pyridine;
6-Methyl-3-(2-(R)-pyrrolidinylmethyloxy)pyridine;
3-(1-Ethyl-2(S)-pyrrolidinylmethyloxy)pyridine;
5-Chloro-3-(2-(S)-azetidinylmethyloxy)pyridine;
4-Methyl-3-(2-(S)-azetidinylmethyloxy)pyridine;
2-Methyl-3-(2-(R)-azetidinylmethyloxy)pyridine; and
3-(1-Methyl-2-(R)-pipecolinylmethyloxy) pyridine;

or a pharmaceutically-acceptable salt or prodrug thereof.

The following are representative of the preferred novel compounds of the present invention:

3-(1-Methyl-2-(R)-pyrrolidinylmethyloxy)pyridine;
3-(2-(R)-Pyrrolidinylmethyloxy)pyridine;
3-(1-Methyl-2-(S)-pyrrolidinylmethyloxy)pyridine;
1-(Methyl)-2-(S)-(1-(3'-pyridyloxy)ethyl)pyrrolidine;
3-(2-(S)-Azetidinylmethyloxy)pyridine;
3-(1-Methyl-2-(S)-azetidinylmethyloxy)pyridine;
3-(2-(S)-Pyrrolidinylmethyloxy)pyridine;
5-Chloro-3-(2-(S)-pyrrolidinylmethyloxy)pyridine;
5-Chloro-3-(1-methyl-2-(S)-pyrrolidinylmethyloxy)pyridine;
2-Methyl-3-(2-(S)-pyrrolidinylmethyloxy)pyridine;
2-Methyl-3-(1-methyl-2-(S)-pyrrolidinylmethyloxy)pyridine ;
6-Methyl-3-(2-(S)-pyrrolidinylmethyloxy)pyridine;
6-Methyl-3-(1-methyl-2-(S)-pyrrolidinylmethyloxy)pyridine;
4-Bromo-3-(1-methyl-2-(S)-pyrrolidinylmethyloxy)pyridine;
3-(1-Methyl-5-methyl-2-(S)-pyrrolidinyimethyloxy)pyridine;
3-(*trans*-1,4-Dimethyl-2(S)-pyrrolidinylmethyloxy)pyridine;
3-(*trans*-1-Methyl-4-ethyl-2(S)-pyrrolidinylmethyloxy)pyridine;
4-Methyl-3-(2-(S)-pyrrolidinylmethyloxy)pyridine;
5-Bromo-3-(1-methyl-2-(S)-pyrrolidinylmethyloxy)pyridine;
2-Methyl-3-(2-(S)-azetidinylmethyloxy)pyridine;
3-(1-Methyl-2-(S)-pyrrolidinylmethyloxy)quinoline;
(1S,4R)-3-(S)-(3-Pyridyloxymethyl)-N-methyl-2-azabicyclo[2.2.1]heptane;

(1R,4S)-3-(R)-(3-Pyridyloxymethyl)-N-methyl-2-azabicyclo[2.2.1]heptane;
5-Chloro-3-(2-(R)-pyrrolidinylmethyloxy)pyridine;
5-Chloro-3-(1-methyl-2-(R)-pyrrolidinylmethyloxy)pyridine;
6-Methyl-3-(1-methyl-2-(R)-pyrrolidinylmethyloxy)pyridine;
6-Methyl-3-(2-(R)-pyrrolidinylmethyloxy)pyridine;
3-(1-Ethyl-2(S)-pyrrolidinylmethyloxy)pyridine; and
5-Chloro-3-(2-(S)-azetidinylmethyloxy)pyridine;

or a pharmaceutically-acceptable salt or prodrug thereof.

The following are representative of the more preferred novel compounds of the present invention:

3-(2-(R)-Pyrrolidinylmethyloxy)pyridine;
3-(1-Methyl-2-(S)-pyrrolidinylmethyloxy)pyridine;
3-(2-(S)-Azetidinylmethyloxy)pyridine;
3-(1-Methyl-2-(S)-azetidinylmethyloxy)pyridine;
3-(2-(S)-Pyrrolidinylmethyloxy)pyridine;
5-Chloro-3-(2-(S)-pyrrolidinylmethyloxy)pyridine;
5-Chloro-3-(1-methyl-2-(S)-pyrrolidinylmethyloxy)pyridine;
6-Methyl-3-(2-(S)-pyrrolidinylmethyloxy)pyridine;
6-Methyl-3-(1-methyl-2-(S)-pyrrolidinylmethyloxy)pyridine;
5-Bromo-3-(1-methyl-2-(S)-pyrrolidinylmethyloxy)pyridine;
2-Methyl-3-(2-(S)-azetidinylmethyloxy)pyridine;
5-Chloro-3-(2-(R)-pyrrolidinylmethyloxy)pyridine;
6-Methyl-3-(2-(R)-pyrrolidinylmethyloxy)pyridine; and
5-Chloro-3-(2-(S)-azetidinylmethyloxy)pyridine;

or a pharmaceutically-acceptable salt or prodrug thereof.

The present invention is also directed to pharmaceutical compositions comprising a therapeutically-effective amount of a compound of formula (I) and a pharmaceutically-acceptable carrier or diluent.

In yet another aspect of the present invention is provided a method of treating cognitive, neurological and mental disorders, which are characterized by decreased cholinergic function in humans and lower mammals, by administration of a compound of formula (I).

"$C_1$-$C_3$-alkyl" and "$C_1$-$C_6$-alkyl" refer to branched or straight-chain, unsubstituted alkyl groups comprising one-to-three or one-to-six carbon atoms, including, but not limited to, methyl, ethyl, n-propyl, isopropyl, n-butyl, t-butyl, sec-butyl or isobutyl, or additionally, for $C_1$-$C_6$-alkyl, neopentyl or n-hexyl and the like.

"$C_1$-$C_3$-alkoxyl" refers to branched or straight-chain, unsubstituted alkyl groups comprising one-to-three or one-to-six carbon atoms, including, but not limited to, methyl, ethyl, n-propyl, isopropyl, n-butyl, t-butyl, sec-butyl or isobutyl, attached to an oxygen linking atom.

"Azacycloalkyl", as used herein, refers to a saturated monocyciic compound having one nitrogen atom and 3-to-5 carbon atoms in the ring, including azetidine, pyrrolidine and piperidine.

"Azabicycloalkyl", as used herein, refers to a saturated bicyciic compound possessing one nitrogen atom common to both rings and also possessing 4-to-5 carbon atoms atoms in each ring, including, but not limited to 1-azabicyclo[2.2.1]heptane, 2-azabicyclo[2.2.1]-heptane, and pyrrolizidine.

"Heteroaryloxy". as used herein, refers to aromatic compounds containing 1 or 2 nitrogen atoms and optionally one additional oxygen or sulfur atom and from 3-to-9 carbon atoms, including, but not limited to pyridine, pyridazine, pyrazine, quinoline, isoquinoline, pyrimidine, and isothiazole.

The term, "prodrug", refers to compounds that are rapidly transformed *in vivo* to yield the parent compounds of Formula (I), as for example, by hydrolysis in blood. T. Higuchi and V. Stella provide a thorough discussion of the prodrug concept in Prodrugs as Novel Delivery Systems, Vol. 14 of the A.C.S. Symposium Series, American Chemical Society (1975). Examples of esters useful as prodrugs for compounds containing carboxyl groups may be found on pages 14-21 of Bioreversible Carriers in Drug Design: Theory and Application, edited by E.B. Roche, Pergamon Press (1987).

The term, "prodrug ester group", refers to any of several ester-forming groups that are hydrolyzed under physiological conditions. Examples of prodrug ester groups include pivoyloxymethyl, acetoxymethyl, phthalidyl, indanyl and methoxymethyl, as well as other such groups known in the art.

The term, "administration", of the cholinergic agent or composition, as used herein, refers to systemic use as when taken orally, parenterally, by inhalation spray, by nasal, rectal or buccal routes, or topically as ointments, pastes, creams, lotions, gels, powders, solutions, sprays, inhalants or transdermal patches in dosage form unit formulations

containing conventional nontoxic pharmaceutically acceptable carriers, adjuvants and vehicles as desired.

The term "parenteral", as used herein, includes intravenous, intramuscular, intraperitoneal. intrasternal, subcutaneous and intraarticular injection as well as via infusion techniques.

By "pharmaceutically-acceptable", it is meant those salts, amides and esters which are, within the scope of sound medical judgement, suitable for use in contact with the tissues of humans and animals without undue toxicity, irritation, allergic response and the like, and are commensurate with a reasonable benefit/risk ratio, effective for their intended use in the treatment of psychological, neurological, cardiovascular and addictive behavior disorders. Pharmaceutically-acceptable salts are well known in the art. For example, S. M. Berge, *et al*. describe pharmaceutically-acceptable salts in detail in J. Pharmaceutical Sciences, **66**: 1-19, **1977**. The salts may be prepared *in situ* during the final isolation and purification of the compounds of Formula (I), or separately by reacting the free base function with a suitable organic acid. Representative acid addition salts include hydrochloride, hydrobromide, sulfate, bisulfate, acetate, oxalate, valerate, oleate, palmitate, stearate, laurate, borate, benzoate, lactate, phosphate, toluenesulfonate, methanesulfonate, citrate, maleate, fumarate, succinate, tartrate, ascorbate, glucoheptonate, lactobionate, lauryl sulfate salts and the like. Representative alkali or alkaline earth metal salts include sodium, calcium, potassium, magnesium salts and the like. Examples of pharmaceutically-acceptable, nontoxic amides of the compounds of Formula I include amides derived from $C_1$-$C_6$-alkyl carboxylic acids wherein the alkyl groups are straight- or branched-chain, aromatic carboxylic acids such as derivatives of benzoic acid and heterocyclic carboxylic acids, including furan-2-carboxylic acid or nicotinic acid. Amides of the compounds of Formula I may be prepared according to conventional methods and include amino acid and polypeptide derivatives of the amines of Formula 1.

As used herein, the term, "pharmaceutically-acceptable carriers", means a non-toxic, inert solid, semi-solid or liquid filler, diluent, encapsulating material or formulation auxiliary of any type. Some examples of the materials that may serve as pharmaceutically-acceptable carriers are sugars, such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols, such as propylene glycol; polyols such as glycerin, sorbitol, mannitol and polyethylene glycol; esters such as ethyl oleate and ethyl laurate; agar; buffering agents such as magnesium hydroxide and aluminum hydroxide; alginic acid; pyrogen-free water; isotonic saline; Ringers solution; ethyl alcohol and phosphate buffer solutions, as well as other non-toxic compatible substances used in pharmaceutical formulations. Wetting agents, emulsifiers and lubricants such as sodium lauryl sulfate and magnesium stearate, as well as coloring agents, releasing agents, coating agents, sweetening, flavoring and perfuming agents, preservatives and antioxidants may also be present in the composition, according to the judgement of the formulator. Examples of pharmaceutically-acceptable antioxidants include water-soluble antioxidants, such as ascorbic acid, cysteine hydrochloride, sodium bisulfite, sodium metabisulfite, sodium sulfite, and the like; oil-soluble antioxidants, such as ascorbyl palmitate, butylated hydroxyanisole (BHA), butylated hydroxytoluene (BHT), lecithin, propyl gallate, alpha-tocopherol and the like; and the metal chelating agents such as citric acid, ethylenediamine tetraacetic acid (EDTA), sorbitol, tartaric acid, phosphoric acid and the like.

By a "therapeutically-effective amount" of the cholinergic channel ligand agent, is meant a sufficient amount of the compound to treat cholinergically-related disorders at a reasonable benefit/risk ratio applicable to obtain a desired therapeutic response. It will be understood, however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidentally with the specific compound employed; and like factors well known in the medical arts. Total daily dose of the compounds of this invention administered to a host in single or divided doses may be in amounts as determined by the attending physician, typically, for example, in amounts of from about 0.001 to 100 mg/kg body weight daily and preferably 0.01 to 10 mg/kg/day. Dosage unit compositions may contain such amounts of submultiples thereof to make up the daily dose.

The present invention includes one or more of the compounds of formula (I) prepared and formulated with one or more non-toxic pharmaceutically-acceptable compositions, as described below.

Compositions suitable for parenteral injection may comprise pharmaceutically-acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethylene glycol, glycerol, and the like), suitable mixtures thereof, vegetable oils (such as olive oil) and injectable organic esters such as ethyl oleate. Proper fluidity may be maintained, for example, by the use of a coating such as lecithin, by the maintenance of the required particle size in the case of dispersions, and by the use of surfactants.

These compositions may also contain adjuvants such as preserving, wetting, emulsifying, and dispersing agents. Prevention of the action of microorganisms may be ensured by various antibacterial and antifungal agents, for example, parabens, chlorobutanol, phenol, sorbic acid, and the like. It may also be desirable to include isotonic agents, for example, sugars, sodium chloride and the like. Prolonged absorption of the injectable pharmaceutical form may be brought about by the use of agents delaying absorption, for example, aluminum monostearate and gelatin.

If desired, and for more effective distribution, the compounds may be incorporated into slow-release or targeted-delivery systems, such as polymer matrices, liposomes, and microspheres. They may be sterilized, for example, by filtration through a bacteria-retaining filter, or by incorporating sterilizing agents in the form of sterile solid compositions, which may be dissolved in sterile water, or some other sterile injectable medium immediately before use.

Solid dosage forms for oral administration may include capsules, tablets, pills, powders, and granules. In such solid dosage forms, the active compound is admixed with at least one inert customary excipient (or carrier), such as sodium citrate or dicalcium phosphate, and additionally (a) fillers or extenders, as for example, starches, lactose, sucrose, glucose, mannitol and silicic acid; (b) binders, as for example, carboxymethylcellulose, alginates, gelatin, polyvinylpyrrolidone, sucrose and acacia; (c) humectants, as for example, glycerol; (d) disintegrating agents, as for example, agar-agar, calcium carbonate, potato or tapioca starch, alginic acid, certain complex silicates and sodium carbonate; (e) solution retarders, as for example paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate or mixtures thereof. In the case of capsules, tablets and pills, the dosage forms may also comprise buffering agents.

Solid compositions of a similar type may also be employed as fillers in soft and hard-filled gelatin capsules, using such excipients as lactose or milk sugar, as well as high molecular weight polyethylene glycols, and the like.

Solid dosage forms such as tablets, dragees, capsules, pills and granules may be prepared with coatings and shells, such as enteric coatings and others well known in this art. They may contain pacifying agents, and may also be of such composition that they release the active compound or compounds in a certain part of the intestinal tract in a delayed manner. Examples of embedding compositions which may be used are polymeric substances and waxes.

The active compounds may also be in micro-encapsulated form, if appropriate, with one or more of the above-mentioned excipients.

Liquid dosage forms for oral administration include pharmaceutically-acceptable emulsions, solutions, suspensions, syrups and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil and sesame oil, glycerol, tetrahydrofurfuryl alcohol, polyethylene glycols and fatty acid esters of sorbitan or mixtures of these substances, and the like.

Besides such inert diluents, these liquid dosage forms may also include adjuvants, such as wetting agents, emulsifying and suspending agents, sweetening, flavoring and perfuming agents.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal or vaginal administrations are preferably suppositories which can be prepared by mixing the compounds of this invention with suitable non-irritating excipients or carriers such as cocoa butter, polyethylene glycol or a suppository wax, which are solid at ordinary temperatures but liquid at body temperature and therefore, melt in the rectum or vaginal cavity and release the active component.

Dosage forms for topical or transdermal administration of a compound of this invention further include ointments, pastes, creams, lotions, gels, powders, solutions, sprays, inhalants or transdermal patches. Transdermal administration via a transdermal patch is a particularly effective and preferred dosage form of the present invention. The active component is admixed under sterile conditions with a pharmaceutically-acceptable carrier and any needed preservative, buffers or propellants as may be required. It is known that some agents may require special handling in the preparation of transdermal patch formulations. For example, compounds that are volatile in nature may require admixture with special formulating agents or with special packaging materials to assure proper dosage delivery. In addition, compounds which are very rapidly absorbed through the skin may require formulation with absorption-retarding agents or barriers. Ophthalmic formulations, eye ointments, powders and solutions are also contemplated as being within the scope of this invention.

The present compounds may also be administered in the form of liposomes. As is known in the art, liposomes are generally derived from phospholipids or other lipid substances. Uposomes are formed by mono- or multi-lamellar hydrated liquid crystals that are dispersed in an aqueous medium. Any non-toxic, physiologically-acceptable and metabolizable lipid capable of forming liposomes may be used. The present compositions in liposome form may contain,

in addition to the compounds of the present invention, stabilizers, preservatives, excipients, and the like. The preferred lipids are the phospholipids and the phosphatidyl cholines (lecithins), both natural and synthetic. Methods to form liposomes are known in the art. See, for example, Prescott, Ed., Methods in Cell Biology, Volume XIV, Academic Press, New York, N. Y., (1976), p 33 *et seq.*

In order to reduce unwanted peripherally-mediated side-effects, it is advantageous, but not essential, to incorporate into the composition a peripherally-acting anti-cholinergic such as N-methylscopolamine, N-methylatropine, propantheline, methantheline, or glycopyrrolate.

Compounds of the invention which have one or more asymmetric carbon atoms may exist as the optically-pure enantiomers, pure diastereomers, mixtures of enantiomers, mixtures of diastereomers, racemic mixtures of enantiomers, diastereomeric racemates or mixtures of diastereomeric racemates. It is to be understood that the present invention anticipates and includes within its scope all such isomers and mixtures thereof. The terms "R" and "S" used herein are configurations as defined in IUPAC 1974 Recommendations for Section E, Fundamental Stereochemistry, *Pure Appl. Chem*., **1976,** *45*: 13-30.

The compounds of the present invention may be synthesized as shown in reaction schemes I and II presented below using the reactions and techniques described in this section. The reactions are performed in a solvent appropriate to the reagents and materials employed are suitable for the transformation being effected. It is understood by those skilled in the art of organic synthesis that the functionality present on the heterocyclic ring and other portions of the molecule must be consistent with the chemical transformation proposed. This will, on occasion, necessitate judgment by the routineer as to the order of synthetic steps, protecting groups required, and deprotection conditions. Substituents on the starting materials may be incompatible with some of the reaction conditions required in some of the methods described, but alternative methods and substituents compatible with the reaction conditions will be readily apparent to skilled practitioners in the art. The use of nitrogen-protecting groups is well known in the art for protecting amino groups against undesirable reactions during a synthetic procedure and many such protecting groups are known, cf, for example, T.H. Greene, Protective Groups in Organic Synthesis, John Wiley & Sons, New York (**1981**).

## SCHEMES

In accordance with Scheme 1, a 2-carboxyl-substituted azacycloalkyl compound (1), wherein the azacycloalkyl groups represents the A portion of the compound of formula (I) wherein A is selected from option (i), wherein n is as described above, $R^3$ is H and wherein the Y is a $C_1$-$C_3$-alkyl or a suitable protecting group, such as BOC or CBZ, for example, which may subsequently be removed and replaced with H or $C_1$-$C_3$-alkyl, is converted to the hydroxymethyl compound of formula (2) with a suitable reducing agent, such as Red-Al®, borane/THF, borane/methyl sulfide or $LiAlH_4$, for example. Compound (2) may be reacted with an appropriately substituted 3-hydroxypyridine (wherein $R^4$ is as described above, or in the case wherein $R^4$ is hydroxyl, $R^4$ is protected with a suitable protecting group, which may be removed after the coupling reaction) in the presence of triphenylphosphine and DEAD as described by O. Mitsunobu (*Synthesis*, **1981:** 1) to form the pyridine compound of formula (3). This compound is then treated with a reagent suitable for removing the N-protecting group, such as trifluoroacetic acid, HCl in glacial acetic acid or HBr in acetic acid, for example, to form the unprotected compound (4). The ring nitrogen is then alkylated, with for example, treatment with alkyl halide in the presence of a base, formaldehyde in formic acid, or with an aldehyde and sodium cyanoborohydride, in an alcohol solvent, such as methanol, ethanol or isopropanol, to give the desired compound (5), wherein $R^1$ is as described above, which is compound (I), wherein A is selected from option (i) and B is selected from option (ii).

## Scheme I

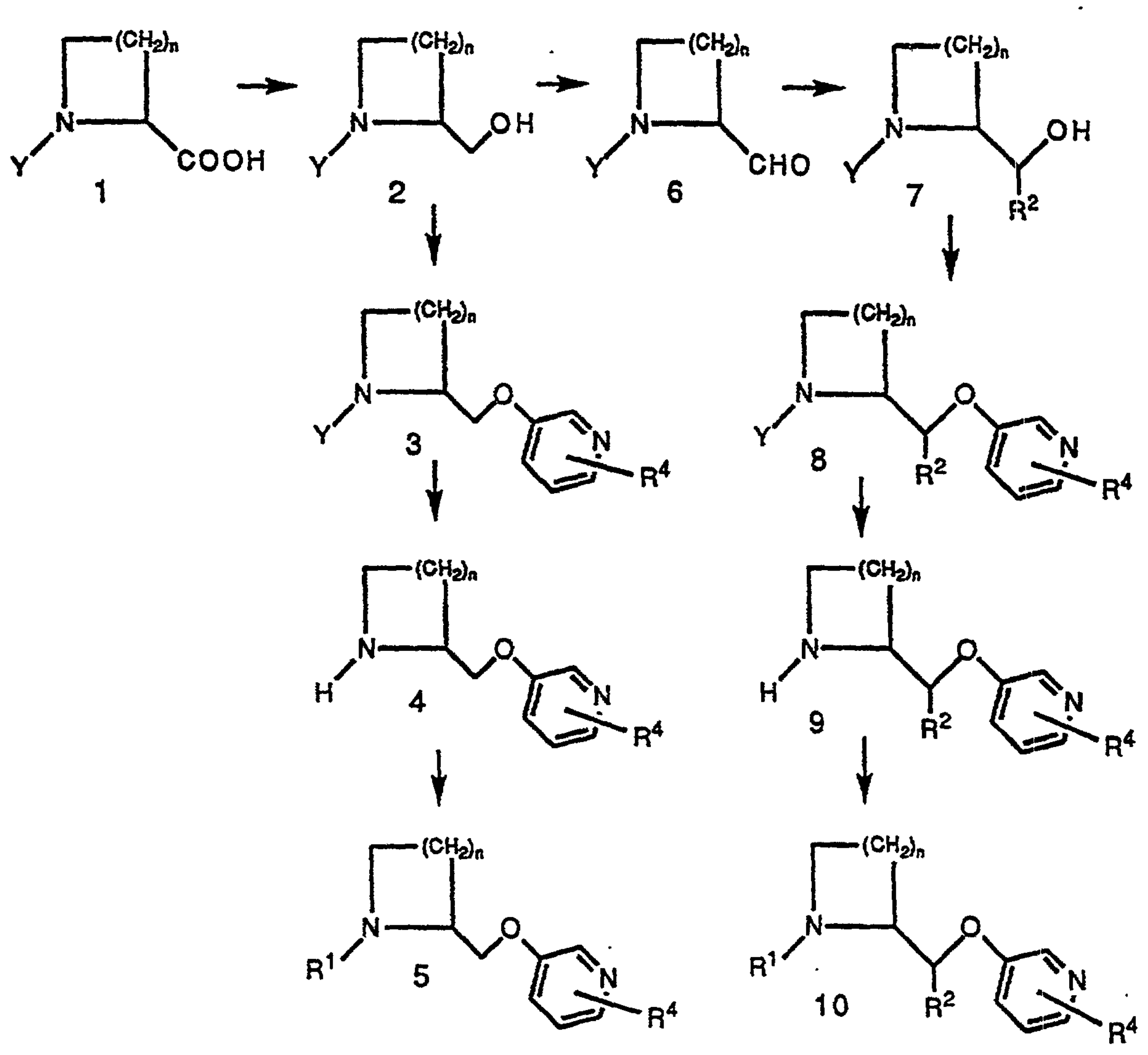

1 2 6 7 3 8 4 9 5 10

Alternately, compound (2) may be oxidized using a suitable mild oxidizing agent such as DMSO/pyridine•$SO_3$, pyridium chlorochromate or DMSO/oxalyl chloride, for example, to afford the aldehyde (6). The aldehyde is then reacted with a suitable organometallic nucleophile, for example, a Grignard reagent, to afford the alcohol (7), wherein $R^2$ is as described above. The alcohol is then reacted as described above with a 3-hydroxypyridine and carried through the same series of reactions as described above, starting with compound (2) and leading to compounds (3), (4) and (5), to give the compounds (8), (9) and (10), wherein $R^1$ is as described above.

## Scheme II

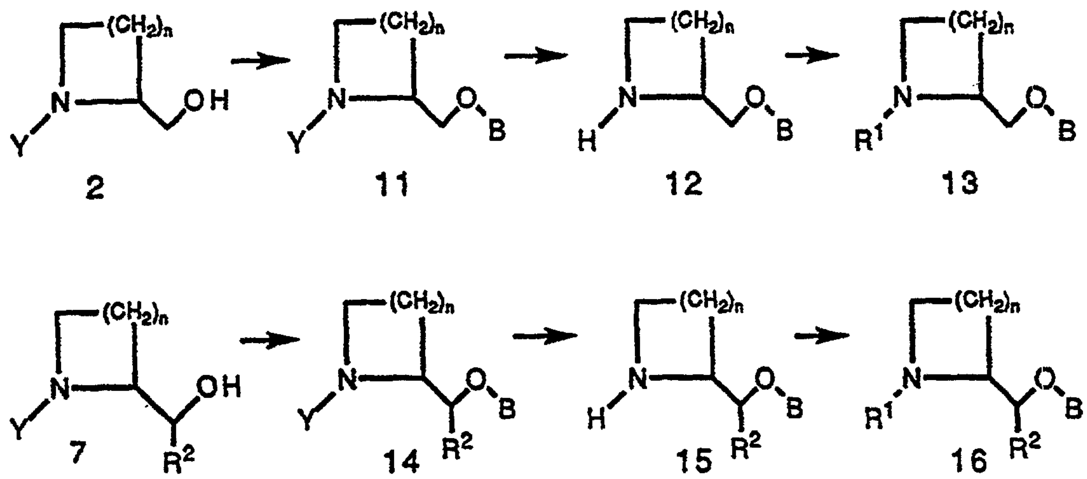

2 11 12 13

7 14 15 16

In accordance with Scheme II, the compound (2) wherein Y is H or is a $C_1$-$C_3$-alkyl or a suitable protecting group, such as BOC or CBZ, for example,is reacted with 3,6-dichloropyridazine, 2-chloropyrazine, 3-chloroquinoline, 5-chloroisoquinoline, bromothiazole or 5-bromopyrimidine, in the presence of a strong base, such as sodium hydride, or in the presence of $K_2CO_3$, CuBr and $PPh_3$, in an inert solvent, such as THF, to afford compound (11), where B is 3-(6-chloropyridazinyl), 2-pyrazinyl, 3-quinolinyl, 2-thiazolyl, or 5-pyrimidinyl, respectively. Compound (11) is then carried through the same series of reactions as described in Scheme I above (starting with compound (3) and leading to compounds (4) and (5)) to give the compounds (12) and (13), wherein $R^1$ is as described above. Alternately, compound (7) is reacted with one of the reagents as described for compound (2) above to give compound (14). Compound (14) is then carried through the same series of reactions as described in Scheme 1 above (starting with compound (8) and leading to compounds (9) and (10)) to give the compounds (15) and (16), wherein $R^1$ and $R^2$ are as described above.

In accordance with Scheme III, the amine compound (17), wherein $R^5$ is $C_1$-$C_3$-alkyl, a chiral auxiliary group (for example, (R)-1-phenylethyl, that is easily removed and replaced with H or the desired $C_1$-$C_3$-alkyl group), or an N-protecting group (such as BOC or CBZ, which may be subsequently removed and

## Scheme III

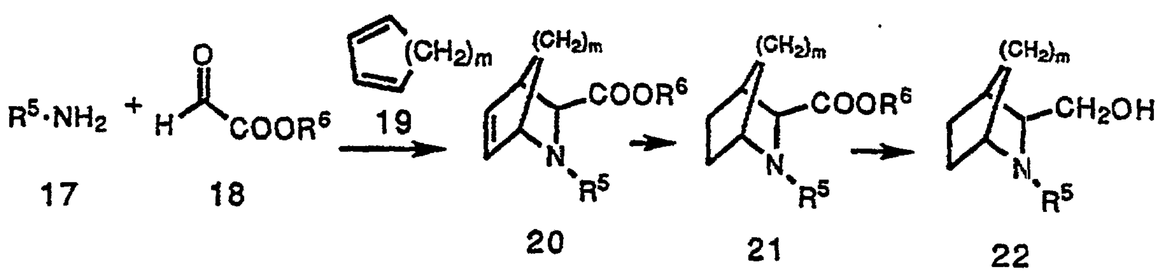

17 18 19 20 21 22

replaced with H or the desired $C_1$-$C_3$-alkyl group), is reacted with an appropriate glyoxylic ester (18), wherein $R^6$ may be $C_1$-$C_5$-alkyl, in the presence of an appropriate $C_5$-$C_7$-cycloalka-1,3-diene compound (19), to give the variously substituted unsaturated compound (20), wherein m is 1, 2 or 3, and $R^5$ and $R^6$ are as described immediately above. Compound (20) is then reduced with $H_2$ in the presence of a noble metal catalyst, such as Pt or Pd/C to give compound (21), wherein m is as described above. Compound (21) is then reacted with a suitable ester reducing agent, such as $LiAlH_4$, $LiBH_4$, borane/THF or borane/methyl sulfide, for example, to afford the appropriately substituted alcohol compound (22), wherein m is as described above. Compound (22) may be substituted for compound (2) and carried forward and reacted according to the alternate reaction pathways shown for compounds (2) or (7) in Schemes I and II to give the desired compound of formula (I), wherein A is selected from option (ii), as described above, and wherein B may

be selected from options (i)-(vii), as described above.

In accordance with Scheme IV are prepared suitable starting materials needed for the preparation of compounds of formula (I). In these, wherein A is selected from option (iii), the starting material may also be represented by formula (I) wherein B is replaced by H. In the case wherein p is 2, this starting material compound may be prepared by the method of Langstrom (Chemica Scripta, 5:170-178 (1974)), to prepare compound (30). In the case wherein p is 1, the desired material is prepared as follows. The commercially available compound (23) is protected with a t-butyldimethylsilyl group by reaction with t-butyldimethylsilyl chloride in the presence of imidazole to give compound (24). The amide nitrogen atom of (24) is protected by reaction with a suitable protecting agent, such as sodium hydride and di-*t*-butyldicarbonate, for example, to give compound (25). This compound is then reacted with LDA followed by bromoacetic ester to prepare compound (26). The ester and the lactam carbonyl

Scheme IV

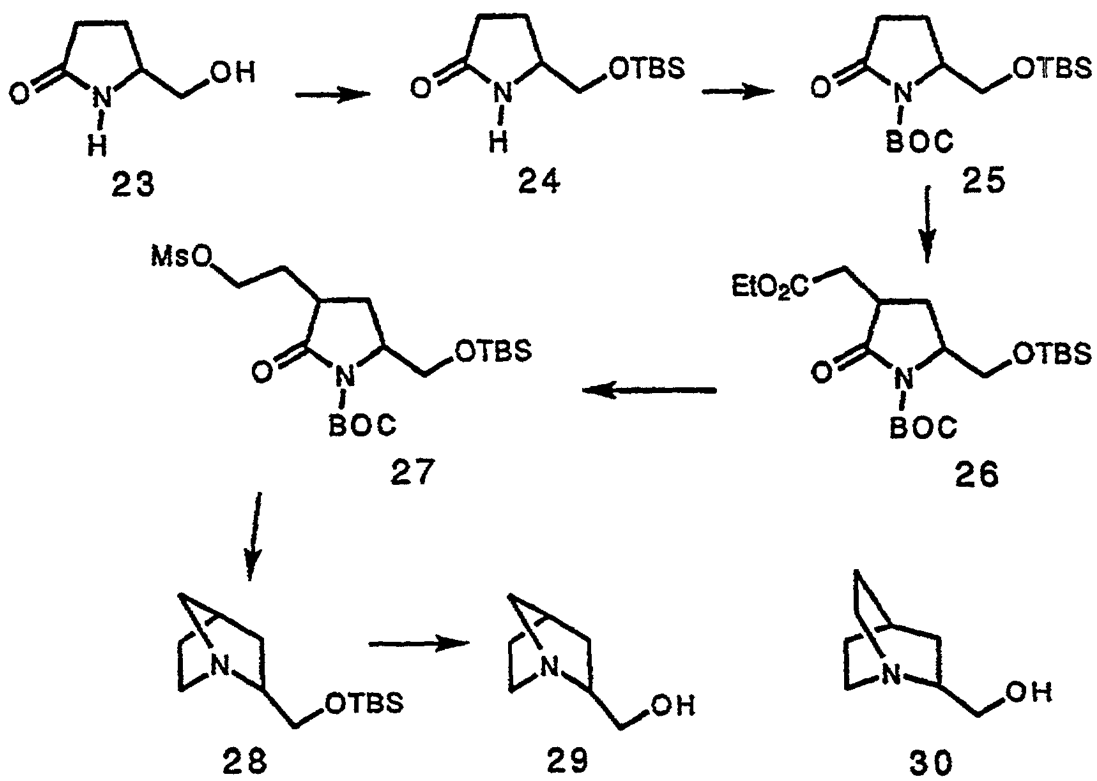

are reduced with suitable reducing agents, such as borane, and the resulting alcohol is immediately reacted with methanesulfonyl chloride chloride in the presence of triethylamine to give the compound (27). The internal ring closure to give compound (28) is accomplished by reacting compound (27) with trimethylsilyl iodide followed by treatment with a base. Removal of the t-butyldimethylsilyl protecting group gives the desired compound (29). Compounds (29) and (30) may be substituted for compound (2) and carried forward and reacted according to the alternate reaction pathways shown for compounds (2) or (7) in Schemes I and II to give the desired compound of formula (I), wherein A is selected from option (iii), as described above, and wherein B may be selected from options (i)-(vii), as described above.

In accordance with Scheme V, wherein a 2-carboxyl-substituted cyclic-amine compound (31), wherein q is 0, 1 or 2, as described above, which is protected at the ring nitrogen with a suitable protecting group, Y, such as BOC or CBZ, for example, is converted to the aldehyde compound (32). To prepare compound (33), wherein q is as described above and r is 0, 1 or 2, alternate but similar methods are utilized. For the case wherein r is 0, compound (32) is reacted with the ylide reagent (A), in a suitable nonpolar solvent and under an inert gas atmosphere, followed by reaction of the intermediate with $Hg^{++}$ ion under aqueous, alkaline conditions. In the case wherein r is 1, compound (32) is reacted with the ylid reagent (B), followed by catalytic reduction with $H_2$ over a

## Scheme V

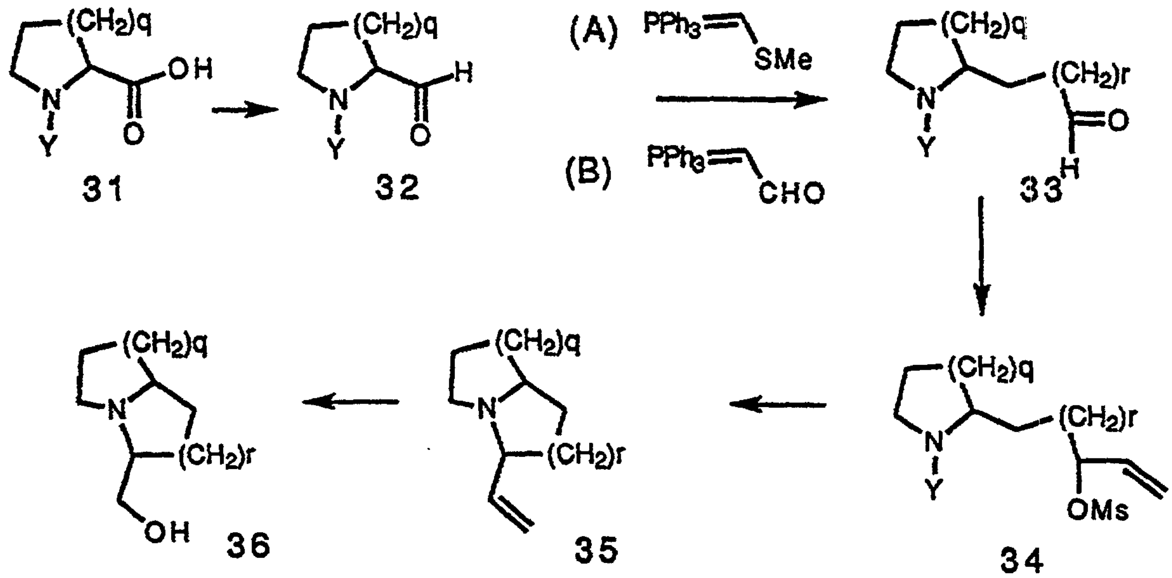


31, 32, 33, 34, 35, 36

noble metal catalyst, such as Pd/C. In the case wherein r is 2, compound (32) is reacted first with the ylide reagent (A), followed by reduction with reaction of the intermediate with $Hg^{++}$ ion, as described above, then reacting the intermediate product next with the ylide reagent (B), followed by catalytic reduction with $H_2$ as described above. The compound (33), wherein q and r are as desired is thus formed and reacted with vinyl magnesium bromide Grignard reagent, followed by mesyl chloride in the presence of triethylamine to give the desired compound (34). Compound (34) is subjected to treatment with trifluoroacetic acid, for example, to remove the N-protecting group, and then with a strong base to cyclize and produce compound (35), where q and r are independently as desired, except that both q and r may not concurrently be 0. The desired alcohol compound (36) is then produced by reacting compound (35) with $OsO_4$ and $NaIO_4$, followed by reduction of the intermediate aldehyde product with a suitable reducing agent, such as $NaBH_4$. Compound (34) may be substituted for compound (2) and carried forward and reacted according to the alternate reaction pathways shown for compounds (2) or (7) in Schemes I and II to give the desired compound of formula (I), wherein A is selected from option (iii),

## Scheme VI

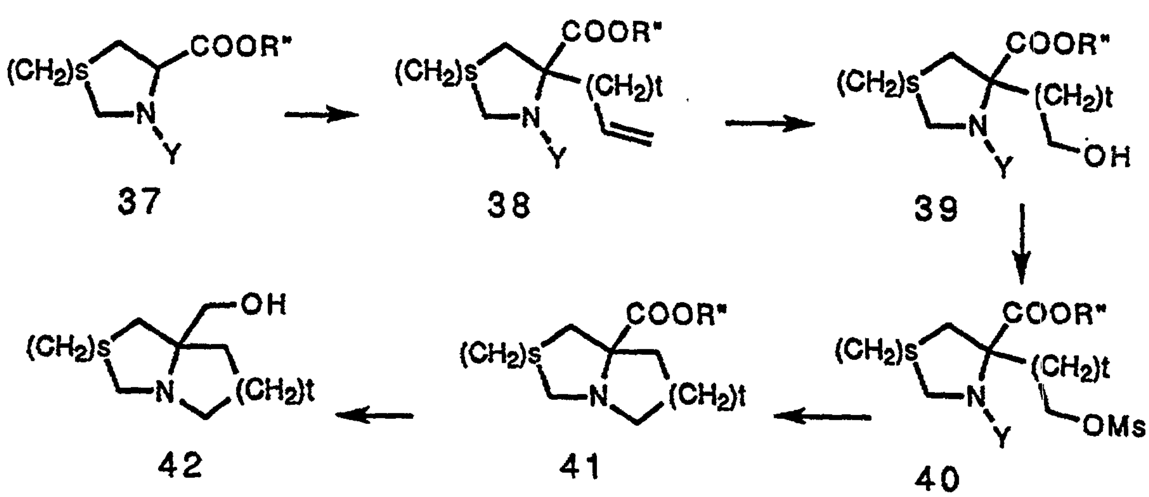

37, 38, 39, 40, 41, 42

In accordance with Scheme VI, it is possible to prepare the precursors of compounds of formula (I), wherein

A is selected from option (v). The compound (37), wherein s is 0, 1, or 2, as desired, R' is a suitable $C_1$-$C_5$-alkyl group, and Y is an N-protecting group, such as CBZ, is reacted with LDA, followed by either 3-bromo-1-propene or 4-bromo-1-butene, to give compound (38), wherein t is 1 or 2, respectively. In the instance wherein it is desired that t be 1 or 2, compound (38) is reacted with $BH_3$ followed by oxidation with $H_2O_2$ to give the alcohol compound (39). In the alternate instance wherein it is desired that t be 0, compound (38) is first oxidized with $NaIO_4$ and $OsO_4$, followed by reduction with $NaBH_4$, to give the compound (39) wherein t is 0. The alcohol (39), wherein t is 0, 1, or 2, is then reacted with methanesulfonyl chloride in a non-polar solvent and presence of a base, such as triethylamine, to give compound (40). By removal of protecting group Y from (40) under standard conditions, followed by treatment with base, such as $K_2CO_3$ or triethylamine, the ring closure is accomplished to afford compound (41). Reduction of the ester function of compound (41) with LiAlH4, for example, prepares the compound (42), wherein s and t are independently as described, except that both s and t may not concurrently be 0. Compound (42) may be substituted for compound (2) and carried forward and reacted according to the alternate reaction pathways shown for compounds (2) or (7) in Schemes I and II to give the desired compound of formula (I), wherein A is selected from option (iii), as described above, and wherein B may be selected from options (i)-(vii), as described above.

Scheme VII

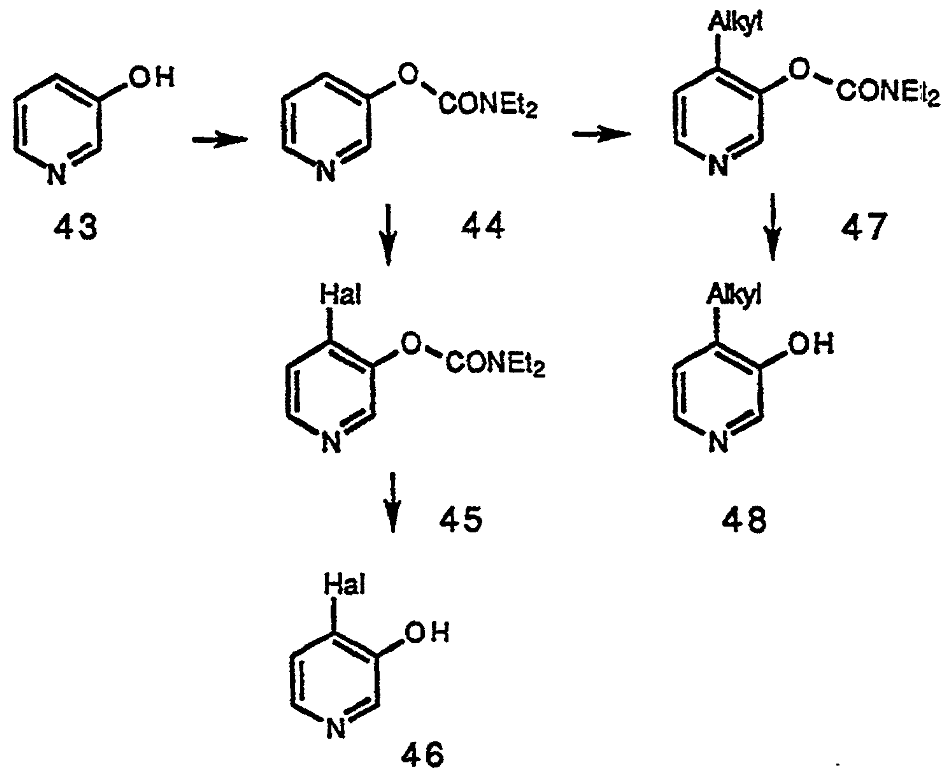

43 44 47 45 48 46

In accordance with Scheme VII, wherein is described the preparation of precursors of the "B" portion of the compounds of formula (I), wherein B is selected from option (ii) wherein $R^4$ is 4-substituted $C_1$-$C_3$-alkyl or Hal group, as defined above, the 3-hydroxypyridine is protected by reaction with dimethylaminocarbonyl chloride to give the compound (44). It is possible to place the Hal substitutent on compound (44) by reaction with *sec*-BuLi followed by reagents represented by the formula Hal-Z, wherein Z is a leaving group, such as 1,2-dibromoethane, N-halosuccinimide, or molecular halogen, for example, to give compound (45). By removing the protecting group from compound (45) with a reagent such as sodium methoxide, for example, the desired compound (46) is produced. Alternately, where it is desired to place an alkyl substituent upon the ring, compound (44) is reacted with seo-BuLi followed by and alkyl halide, such as alkyl bromide or alkyl iodide, for example, to give compound (47), which is then deprotected with a reagent such as sodium methoxide, for example, to give the desired compound (48).

## Scheme VIII

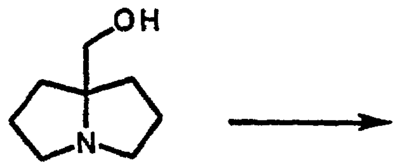

(I)

49

In accordance with Scheme VIII, wherein it is desired to produce compounds of formula (I), wherein A is selected from option (v), as described above, the starting material compound (49) (prepared according to S. Miyano *et al*., J. Heterocyclic Chem., 24:47 **(1987))** is substituted for compound (2) and carried forward and reacted according to the alternate reaction pathways shown for compounds (2) or (7) in Schemes I and II to give the desired compound of formula (I), wherein A is selected from option (v), as described above, and wherein B may be selected from options (i)-(vii), as described above.

## Scheme IX

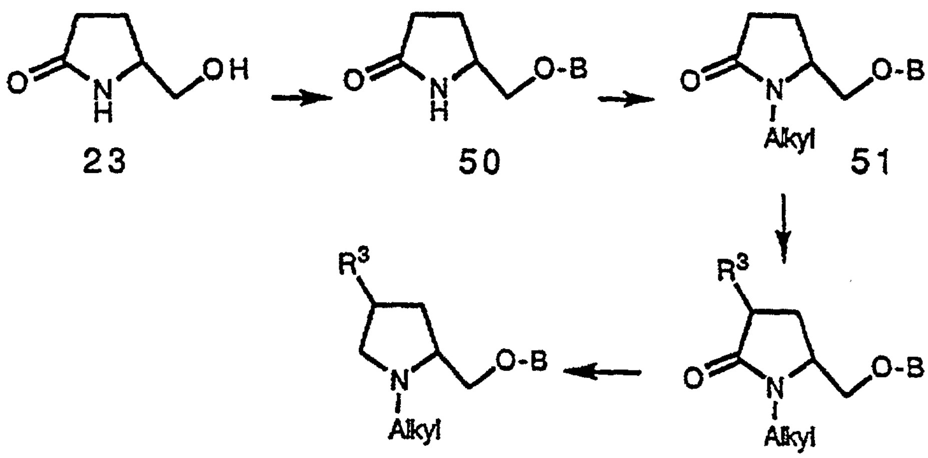

23 50 51

53 52

In accordance with Scheme IX, the compound (23) may be reacted according to the alternate reaction pathways shown for compounds (2) or (7) in Schemes I and II to give the desired compound (50). The N-alkyl group, wherein the alkyl group is $C_1$-$C_6$-alkyl, is added to compound (50) by reaction with NaH and the appropriate alkyl iodide in anhydrous THF to give compound (51). Compound (51) is treated with LDA and E-X, wherein X is a leaving group and E is the appropriate precursor group of $R^3$, as defined above, (except in the case wherein $R^3$ is $C_1$-$C_3$-alkoxy, in which case the first reaction produces an alcohol which is then reacted under standard Williamson ether synthesis conditions to give the ether), such as an oxaziridine, formaldehyde, a halomethyl ether, N-halo-succinimide, or molecular halogen, for example, to give the substituted compound (52), wherein $R^3$ is as described above, which by reducing with $BH_3$ followed by treatment with CsF gives the desired compound (53).

## Scheme X

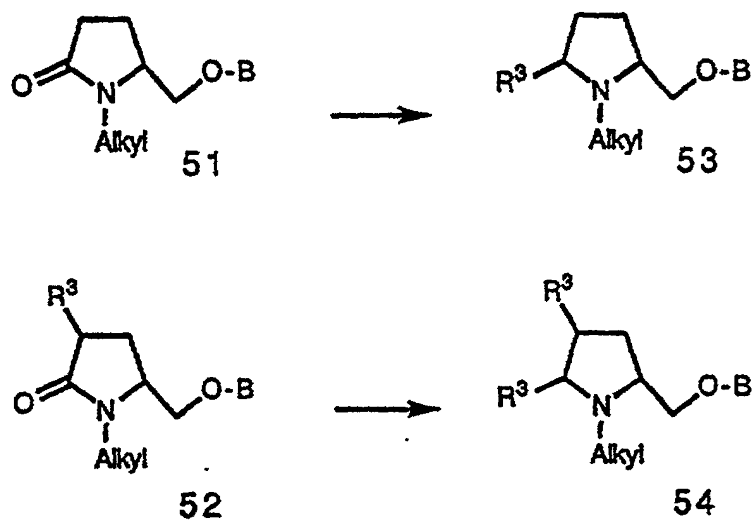

51 53

52 54

In accordance with Scheme X are synthesized the compounds of formula (I), wherein A is selected from option (i), wherein $R^3$ therein represents the appropriate 5-substituent group, wherein $R^3$ is as described above, compound (51) is reacted with an $C_1$-$C_3$-alkyl Grignard reagent followed by $NaBH_3CN$ to give the compound (53). When compound (52) is reacted with a $C_1$-$C_3$-alkyl Grignard reagent followed by $NaBH_3CN$, the compound (54) is prepared, wherein $R^3$ is as described above, and may be two substituent groups.

*IN VITRO* DETERMINATION OF NEURONAL NICOTINIC RECEPTOR BINDING POTENCIES AND SELECTIVITY

For the purpose of identifying compounds as cholinergic agents which are capable of interacting with cholinergic channel receptors in the brain, a ligand-receptor binding assay was carried out as the initial screen. Compounds of the present invention were effective at interacting with neuronal nicotinic cholinergic receptors as assayed for their ability (compared to (-)-nicotine) to displace radioligand from neuronal nicotinic cholinergic channel receptors labeled with [$^3$H]-cytisine ([$^3$H]-CYT). The functional nature of this ligand-receptor interaction was determined by measuring the ability of the novel ligand to activate cholinergic channel receptors in PC12 cells

The ability of the compounds of the invention to interact with cholinergic channel receptors can be demonstrated *in vitro* using the following protocols.

A. Protocols For Determination of Nicotinic Cholinergic Channel Receptor Binding Potencies of Ligands

Binding of [$^3$H]-cytisine ([$^3$H]-CYT) to nicotinic receptors was accomplished using crude synaptic membrane preparations from whole rat brain (Pabreza *et al*., *Molecular Pharmacol*., **1990,** 39:9). Washed membranes were stored at-80°C prior to use. Frozen aliquots were slowly thawed and resuspended in 20 volumes of buffer (containing: 120 *mM* NaCl, 5 *mM* KCl, 2 *mM* $MgCl_2$, 2 *mM* $CaCl_2$ and 50 *mM* Tris-Cl, pH 7.4 @4°C). After centrifuging at 20,000x g for 15 minutes, the pellets were resuspended in 30 volumes of buffer. Homogenate (containing 125-150 µg protein) was added to triplicate tubes containing concentrations of test compound and [$^3$H]-CYT (1.25 *nM*) in a final volume of 500 µL. Samples were incubated for 60 minutes at 4°C, then rapidly filtered through Whatman GF/B filters presoaked in 0.5% polyethylimine using 3 x 4 mL of ice-cold buffer. The filters are counted in 4 mL of Ecolume® (ICN). Nonspecific binding was determined in the presence of 10 µM (-)-nicotine and values were expressed as a percentage of total binding. $IC_{50}$ values were determined with the RS-1 (BBN) nonlinear least squares curve-fitting program and $IC_{50}$ values were converted to Ki values using the Cheng and Prusoff correction (Ki=$IC_{50}$/(1+[ligand]/Kd of ligand). Alternately, data were expressed as a percentage of the total specific binding. The results (shown in Table 1) suggest that the compounds of the present invention have high affinity for the neuronal nicotinic cholinergic channel receptor.

B. Protocols for the Determination of Cholinergic Channel Activation in PC12 Cells.

The cholinergic channel activator properties of Example 9 were investigated in PC12 cells using the whole-cell patch-clamp approach to measure current flow through ligand-gated membrane channels. The electrophysiological approach demonstrates clear agonist activity of the activator, and indicates that this is due to direct activation of cholinergic ligand-gated channels.

Table 1.

| Binding to Neuronal Nicotinic Receptors | |
|---|---|
| Example number | Ki(mM) |
| (-)-nicotine | 0.69 |
| 1 | 21 |
| 2 | 0.17 |
| 3 | 16 |
| 4 | 0.2 |
| 5 | 87 |
| 6 | 6.7 |
| 7 | 0.44 |
| 8 | 28 |
| 9 | 0.05 |
| 10 | 0.25 |
| 11 | 110 |
| 12 | 38 |
| 13 | 65 |
| 14 | 0.14 |
| 15 | 0.13 |
| 16 | 0.6 |
| 17 | 16.7 |
| 18 | 0.28 |
| 19 | 1.47 |
| 20 | 341 |
| 21 | 23 |
| 22 | 25 |
| 23 | 78.3 |
| 24 | 4.2 |
| 25 | 14.7 |
| 26 | 76 |
| 27 | 5.8 |
| 28 | 0.23 |
| 29 | 1.3 |
| 30 | 1.3 |
| 31 | 110 |
| 32 | 36 |
| 33 | 74.3 |
| 34 | 53 |
| 35 | 103 |
| 36 | 18 |
| 37 | 2 |
| 38 | 4 |
| 39 | 0.2 |
| 40 | 11 |
| 41 | 39 |

Table 1. (continued)

| Binding to Neuronal Nicotinic Receptors | |
|---|---|
| Example number | Ki(mM) |
| 42 | 26 |
| 43 | 0.5 |
| 44 | 13 |
| 45 | 0.04 |
| 46 | 0.05 |
| 47 | 9 |
| 48 | 349 |

PC12 cells are a rat pheochromocytoma cell line that, upon treatment with nerve growth factor (NGF) differentiate into a neuronal phenotype and express nicotinic cholinergic channels (Greene & Tischier, *Proceedings of the National Academy of Sciences of the United States of America*, 73: 2424-2428, **1976**; Dichter *et al*., *Nature*, 268: 501-504, **1977**). Prominent neuronal cholinergic channel subunits in these cells are α3, α5, β2, β3 and β4 (Rogers *et al*., *Journal of Neuroscience*, 12: 4611-4623, **1992**). Thus, PC12 cells can be used to directly evaluate the ability of substances to activate or inhibit certain subtypes of neuronal nicotinic cholinergic channels.

PC12 cells were obtained initially from ATCC and, using standard techniques were maintained in DMEM containing 10% heat-inactivated fetal calf serum and 5% heat-inactivated horse serum (37°C, 95% 02 & 5% CO2). Electrophysiologic recordings were obtained from differentiated (neurite-bearing) cells after 4-7 days exposure to NGF. For this purpose, the undifferentiated cells were first plated onto

polylysine-coated 12 mm round glass coverslips in 60x15 mm plastic Petri dishes. After 20 minutes, differentiating medium was added (DMEM containing 100 ng/ml nerve growth factor, 5% heat-inactivated fetal calf serum and 2.5% heat-inactivated horse serum) and the cells were refed with this medium every 3 days.

The whole-cell patch-clamp technique was used to record voltage-activated currents and ligand-gated currents activated by substances applied through the U-tube flow-reversal technique (Fenwick *et al*., *The Journal of Physiology (London)* 331: 577-597, **1982).** A coverslip bearing the cells was transferred from culture dish to the recording chamber (350 µL volume) and superfused (1 ml/min) at room temperature with an extracellular solution containing 150 mM NaCl, 2.8 mM KCl, 2.0 mM $CaCl_2$, 1.0 mM $MgCl_2$, ≥ 10 mM dextrose and 10 mM Na-HEPES buffer (7.3 pH, 325 mOsm). The intracellular (recording pipette) solution consisted of 140 mM KCl, 1.0 mM $CaCl_2$, 2.0 mM $MgCl_2$, 11 mM K-EGTA, and 10 mM K-HEPES buffer (7.3 pH, 315 mOsm). Osmolarities were adjusted using dextrose such that the extracellular solution was 10 mOsm hypertonic compared to the intracellular solution. Voltage-gated inward (sodium) and outward (potassium) currents were monitored throughout the experiment to determine establishment and maintenance of the whole-cell recording configuration.

After establishing the recording, cells were kept at a holding potential of-60 mV and cholinergic channel ligands dissoived in bathing solution were applied to the cells through computer-controlled U-tube flow reversal for a period of 5 seconds during which typical nicotinic desensitization was observed. The ligand was applied at least twice at each concentration in every cell, and applications were separated by ≥ 3 minutes to allow for recovery from desensitization, washout of the bathing solution, and re-equilibration of the U-tube. Antagonists were applied to the bathing solution through superfusion for 5-10 minutes prior to application of both antagonist and agonist through the U-tube flow reversal.

Results

Example 9 elicited responses very similar to those of (-)-nicotine in PC12 cells (see Table 2). Mecamylamine (10 µM) inhibited the responses to Example 9 (10 µM) by ≥ 80% (n=4), indicating that the response was mediated by cholinergic channel receptors. Example 9 appeared more potent and/or efficacious than (-)-nicotine, because the response to 10 µM mecamylamine was larger than the response to 50 µM (-)-nicotine ((-)-nicotine $EC_{50}$ = 52 µM). In five cells, the peak current responses to 10 µM Example 9 ranged between 165 and 702 pA, while the responses to 50 µM (-)-nicotine ranged between 81 and 539 pA. Overall, the response to 10 µM Example 9 was 168 ± 12 % of the response to 50 µM (-)-nicotine in the same cell (mean ± S.E.M., n=5). These data demonstrate the utility of Example 9 to potently activate cholinergic channels.

Table 2.

| PC12 Current Responses to Example 9 Compared to (-)-nicotine. | | | |
|---|---|---|---|
| PC12 CELL | CURRENT AT PEAK (pA) | | RESPONSE RATIO(%) |
| | Example 9 | (-)-NICOTINE | Example 9 /(-)-NICOTINE |
| A | 165 | 81 | 203 |
| B | 173 | 108 | 161 |
| C | 484 | 283 | 171 |
| D | 283 | 163 | 173 |
| E | 702 | 539 | 130 |
| MEAN±S.E.M. | 361 ±103 | 235 ± 84 | 168 ± 12 |

*IN VIVO* STUDIES DEMONSTRATING ACTIVITY AS COGNITION ENHANCERS

A. Inhibitory Avoidance Studies

The inhibitory (or sometimes called passive) avoidance (IA) test is a well accepted animal model of learning/memory used to assess the activity of novel cholinergic agonists to enhance cognitive function (Wanibuchi *et al*., *Eur. J. Pharmacol*., **1990,** *187*:479). Animals are placed in the illuminated (12 X 14 X 11 cm) portion of a two-chambered box, from which they enter through a guillotine door to the larger (24 X 13.5 X 12 cm) dark compartment of the box. Entry to the dark compartment is accompanied by a mild (0.5 mA), brief (2 seconds) footshock. Initial latencies to cross are recorded, with an imposed 60 second ceiling. Following a 72 hour retention interval, animals are returned to the illuminated chamber, and latency to return to the dark compartment is again recorded, with a ceiling of 180 seconds. No footshock is administered on the test day.

Animals received systemic injections of (-)nicotine. the test compound (0.06-6.2 µmol/kg, IP) or saline (control) 15 minutes before training in the inhibitory avoidance task, and retention was evaluated 24 hours later. Twelve animals were used in each group. (-)-Nicotine induced a dose-dependent facilitation of retention of the avoidance response at 0.62 µmol/kg ($p<0.05$). The results of the experimental compounds upon the retention of the avoidance response are given in Table 3.

B. Mouse Elevated Plus-Maze Studies

The mouse elevated plus-maze is a conflict test that probes anxiolytic activity of test compounds (Lister, *Psychopharmacology*, **1987,** *92*:180). It is based on the fact that exposure of mice to an elevated open arm leads to an avoidance response considerably stronger than that evoked by exposure to an enclosed arm.

The apparatus required to perform this test is made of plywood and consists of two open arms (17 X 8 cm) and two enclosed arms (17 X 8 X 15 cm) extending from a central platform (8 X 8 cm). It is mounted on a plywood base rising 39 cm above the floor. Mice are released on the central platform and the time spent in the open and enclosed arms is recorded during a 5 minute test period. The test compounds were administered to CD1 mice 15 minutes before the test. (-)Nicotine (0.62 and 1.9 µmol/kg, $p<0.05$) induced a significant increase in the time spent by the mice in the open arms of the maze (a measure of anxiolytic effect) as compared to saline-injected mice. The results of the experimental compounds upon the the time spent by thë mice in the open arms of the maze is also given in Table 3. The data in Table 3 demonstrate the utility of these compounds in enhancing cognitive performance or as anxiolytic agents.

Table 3.

| Results* of *In Vivo* Tests for Inhibitory Avoidance (IA) and Elevated Plus-Maze (EPM) | | |
|---|---|---|
| Example # | IA (µmol/kg) | EPM (µmol/kg) |
| (-)-nicotine | significant [0.19-0.62] | significant [0.62-1.9] |
| 3 | NE [0.062-1.9] | NE [0.019-0.62] |
| 4 | significant [0.019] | NE [0.019-0.62] |

* Results are expressed in levels of improvement in performance at the dose range tested. NT = Not tested. NE = No effect or no improvement at the dose range tested.

Table 3. (continued)

| Results* of *In Vivo* Tests for Inhibitory Avoidance (IA) and Elevated Plus-Maze (EPM) | | |
|---|---|---|
| Example # | IA (μmol/kg) | EPM (μmol/kg) |
| 7 | marginal | NE [0.019-0.62] |
| 8 | NE [0.062- 1.9] | NE [0.062- 1.9] |
| 9 | NE [0.0019- 0.062] | significant [0.0062] |
| 10 | marginal [0.62] | significant [0.19] |
| 12 | NE [0.062- 1.9] | NE [0.019- 1.9] |
| 14 | NE [0.019- 0.62] | marginal [0.019] |
| 15 | significantly inhibits [0.062] | significant [0.062- 0.19] |
| 16 | significant [0.019] | NE [0.019- 0.62] |
| 17 | significant [0.062, 0.62, 1.9] | significant [1.9] |
| 18 | NE [0.019- 0.62] | significant [0.062- 0.19] |
| 19 | NE [0.019- 0.62] | NE [0.019-62] |
| 22 | NE [0.062- 1.9] | NE [0.062-1.9] |
| 29 | significantly Inhibits [19] | significant [0.62, 1.9] |
| 34 | NE [0.062-1.9] | marginal [0.62] |

* Results are expressed in levels of improvement in performance at the dose range tested. NT = Not tested. NE = No effect or no improvement at the dose range tested.

The following examples, which are provided for illustration and not limitation of the invention, will serve to further illustrate preparation of the novel compounds of the invention and their biological activity. Thin-layer chromatography (TLC) was performed on 0.25 mm E. Merck precoated silica gel plates (60 F-254). Flash chromatography was performed on 200-400 mesh silica gel (E. Merck), while column chromatography was performed on 70-230 mesh silica gel (E. Merck).

The following abbreviations are used: THF for tetrahydrofuran, DMF for N, N-dimethylformamide, $D_2O$ for deuterium oxide, $CDCl_3$ for deuterochloroform, DMSO-$d_6$ for deuterodimethylsulfoxide, BOC for t-butyloxycarbonyl, CBZ for benzyloxycarbonyl, Bz for benzyl, Ms for methanesulfonyl, PAW for pyridine/acetic acid/water (20:6:11), DCC for dicyclohexylcarbodiimide, DIBAL for diisobutylaluminum hydride, DIEA for diisopropylethylamine, DPPA for diphenylphosphoroazidate, EDCl for 1 -(3-dimethyl-aminopropyl)-3-ethylcarbodiimide hydrochloride, EtOH for ethanol, IBCF for isobutyl chloroformate, HOAc for acetic acid, HOBT for 1-hydroxybenzotriazole, LAH for lithium aluminum hydride, $NH_4OAc$ for ammonium acetate, NMM for N-methylmorpholine, TEA for triethylamine.

Example 1

3-(1-Methyl-2-(R)-pyrrolidinylmethyloxy)pyridine hydrochloride

1a. 3-(1-Methyl-2-(R)-pyrrolidinylmethyloxy) pyridine

To a solution of triphenylphosphine (509 mg, 1.94 mmol) in 10 mL of tetrahydrofuran at room temperature was added diethyl azodicarboxylate (DEAD) (0.35 mL, 1.94 mmol) dropwise with stirring. After stirring at room temperature for 30 minutes, (R)-1-methyl-2-pyrrolidinemethanol (Aldrich Chemical Co., 150 mg, 1.30 mmol) and 3-hydroxypyridine (Aldrich Chemical Co., 185 mg, 1.94 mmol) were added to the reaction mixture. The resultant solution was then allowed to stir at room temperature overnight. After all of the starting material was consumed, the organic solvent was evaporated *in vacuo*. The residue was purified by column silica gel chromatography eluting with chloroform:methanol (10: 1) to provide 76 mg (21% yield) of the title compound. MS (DCl/$NH_3$) m/e 193 $(M+H)^+$. $^1H$ NMR ($CDCl_3$, 300 MHz) $\delta$: 8.32 (t, J= 1.5 Hz, 1H), 8.22 (t, J= 3 Hz, 1H) 7.24-7.2 (m, 2H), 4.14-4.05 (dd, J= 9, 6 Hz, 1H), 4.00-3.93 (dd, J=9, 6 Hz, 1H), 3.24-3.14 (m, 1H), 2.81-2.7 (m, 1H), 2.54 (s, 3H), 2.44-2.31 (m, 1H), 2.14-2.00 (m, 1H), 1.96-1.71 (m, 3H).

1b. 3-(1-Methyl-2-(R)-pyrrolidinylmethyloxy) pyridine hydrochloride

The product from Example 1a (76 mg, 0.4 mmol) was dissolved in ethanol. Hydrochloric acid in diethyl ether was added dropwise to a stirring solution of base at ambient temperature. The resultant white precipitate was then collected by evaporation of solvent and triturated with three portions of diethyl ether. The hygroscopic solid was obtained

in 50% yield (53 mg). $[\alpha]^{25}D$= +6,54 ° (c=1, MeOH). MS ($DCI/NH_3$) m/e 193 $(M+H)^+$. $^1H$ NMR ($D_2O$, 300 MHz) δ: 8.43 (br s, 1H), 8.33 (d, J= 5.6 Hz, 1 H), 7.88-7.82 (m, 1H), 7.72 (dd, J= 8.50, 5.15 Hz, 1H), 4.59 (dd, J=11,3Hz, 1H), 4.42 (dd, J=11.4, 6.2 Hz, 1H), 4.17-4.25 (m, 1H), 4.05-3.9 (m, 1H), 3.82-3.74 (m, 1H), 3.34-3.22 (m, 1H), 3.04 (s, 3H), 2.42-2.36 (m, 1H), 2.26-2.06 (m, 3H). Analysis calculated for $C_{11}H_{16}N_2O$•HCl•0.2 H2O: C, 49.15; H, 6.90; N, 10.42; Found: C, 48.90; H, 7.17; N, 10.89.

Example 2

3-(2-(R)-pyrrolidinylmethyloxy)pyridine hydrochloride

2a. 3-(1-t-butoxycarbonyl-2-(R)-pyrrolidinylmethyloxy) pyridine

To a solution of triphenylphosphine (1.24g, 4.74 mmol) in 20 mL of tetrahydrofuran at room temperature was added diethyl azodicarboxylate (0.746 mL, 4.74 mmol) dropwise with stirring. After stirring at room temperature for 30 minutes, (R)-1-t-butoxycarbonyl-2-pynolidinemethanol (350 mg, 3.16 mmol) and 3-hydroxypyridine (450 mg, 4.74 mmol) were added to the reaction mixture. The resultant solution was then allowed to stir at room temperature overnight. After all of the starting material was consumed, the organic solvent was evaporated *in vacuo*. The residue was purified by silica gel column chromatography. Elution with chloroform:methanol (10:1) provided 518 mg of the title compound along with substantial amount of reduced DEAD reagent. MS ($DCI/NH_3$) m/e 279 $(M+H)^+$.

2b. 3-(2-(R)-pyrrolidinylmethyloxy)pyridine

To a solution of the product of Example 2a in 2 mL of methylene chloride solution was added 2 mL of trifluoroacetic acid. The resultant solution was allowed to stir at room temperature for 2.5 hour. Evaporation of both solvent and trifluoroacetic acid gave a brown oil which was basified with saturated ammonium hydroxide solution. This oil was purified by silica gel column chromatography. Elution with a mixture of chloroform:methanol: ammonium hydroxide (10: 1:0.1) provided the desired product. MS ($DCI/NH_3$) m/e 179 $(M+H)^+$. $^1H$ NMR ($D_2O$, 300 MHz) δ: 8.33 (br s, 1H), 8.22 (m, 1H), 7.22(m, 2H), 3.82-4.03 (m, 2H), 3.50-3.61 (m, 1H), 2.92-3.10(m, 2H), 1.70-2.10 (m, 4H), 1.51-1.66 (m, 1H).

2c. 3-(2-(R)-pyrrolidinylmethyloxy)pyridine hydrochloride

The product from Example 2b (76 mg, 0.4 mmol) was dissolved in ethanol. Hydrochloric acid in diethyl ether was added dropwise to a stirring solution of base at ambient temperature. The resultant white precipitate was then collected by evaporation of solvent and triturated with three portions of diethyl ether. The hygroscopic solid was obtained in 50% yield (53 mg). MS ($DCI/NH_3$) m/e 179 $(M+H)^+$. 1 H NMR ($D_2O$, 300 MHz) δ: 8.43 (br s, 1 H), 8.34 (br s 1H), 7.85(dd, J=8.80, 2.90 Hz, 1H), 7.72 (dd, J= 8.80, 5.15 Hz, 1H), 4.54 (dd, J=11, 3.3 Hz, 1H), 4.32 (dd, J= 10.6, 3.3 Hz, 1H), 4.10-4.19 (m, 1H), 3.42 (t, J= 7.5 Hz, 1H), 2.27-2.35 (m, 1H), 2.06-2.21 (m, 2H), 1.90-2.02 (m, 1 H). Analysis calculated for $C_{10}H_{14}N_2O$•2.4 HCl: C, 45.20; H, 6.22; N, 10.54; Found: C, 45.12; H, 6.00; N, 10.33.

Example 3

2-(1-Methyl-2-(S)-pyrrolidinylmethyloxy)pyrazine fumarate

3a. 2-(1-Methyl-2-(S)-pyrrolidinylmethyloxy) pyrazine

(S)-(-)-1-Methyl-2-pyrrolidinemethanol (Aldrich Chemical Co., 0.5 g, 4.34 mmol) was dissolved in anhydrous THF and brought to 0°C. NaH ((80% dispersion in mineral oil), 131 g, 4.56 mmol) was added and the reaction mixture was allowed to warm to room temperature with stirring. After 30 minutes 2-chloropyrazine (.497 g, 4.34 mmol) was added via syringe. The mixture was stirred for 48 hours. The solvent was then evaporated *in vacuo* and the mixture diluted with chloroform, washed with saturated $NaHCO_3$ and a brine solution. The organic layer was then dried over $MgSO_4$. The resulting crude material was purified by flash chromatography (10% $MeOH/CHCl_3$) to give .81g (97% yield) of the title compound as an oil. TLC $R_f$=0.3 (10%$MeOH/CHCl_3$). MS ($DCI/NH_3$) m/e 194 $(M+H)^+$. $^1H$ NMR ($CDCl_3$, 300 MHz) δ: 8.24 (d, J=1.5 Hz, 1H), 8.11 (d, J=3 Hz, 1H), 8.07 (dd, J=3, 1.5 Hz, 1H), 4.36 (dd, J=9, 6 Hz, 1H), 4.28 (dd, J=9, 6 Hz, 1H), 3.16-3.08 (m, 1H), 2.7-2.58 (m, 1H), 2.47 (s, 3H), 2.34-2.24 (m, 1H), 2.08-1.93 (m, 1H), 1.91-1.67 (m, 3H).

3b. 2-(1-Methyl-2-(S)-pyrrolidinylmethyloxy)pyrazine fumarate

The product of Example 3a was dissolved in anhydrous MeOH and brought to 0°C with stirring. Fumaric acid was dissolved in MeOH with sonication and added dropwise to the base. The mixture was warmed to room temperature with stirring. After 30 minutes the solvent was evaporated *in vacuo* and the remaining solid was triturated with anhydrous diethyl ether. The resulting white solid was vacuum filtered to yield .117g (50% yield) of product. m.p.= 116-118°C. MS ($DCl/NH_3$) m/e 194 $(M+H)^+$. $^1H$ NMR ($D_2O$, 300 MHz) δ: 8.33 (d, J=1.1 Hz, 1H), 8.23-8.21 (m, 2H), 6.65 (br s, 2H), 4.76-4.71 (dd, J=12.5,3 Hz, 1H), 4.60-4.54 (dd, J=12.5, 6 Hz, 1H), 3.98-3.9 (m, 1H), 3.8-3.72 (m, 1H), 3.3-3.2 (m, 1H), 3.04 (s, 3H), 2.45-2.32 (m, 1H), 2.28-2.01 (m, 3H). Analysis calculated for $C_{10}H_{15}N_3O \bullet C_4H_4O_4$: C, 54.36; H, 6.19; N, 13.58; Found: C, 54.23; H, 6.04; N, 13.58.

Example 4

3-(1-Methyl-2-(S)-pyrrolidinylmethyloxy)pyridine fumarate

4a. 3-(1-Methyl-2-(S)-pyrrolidinylmethyloxy) pyridine

(S)-1-Methyl-2-pyridinemethanol was reacted with triphenylphosphine, DEAD and 3-hydroxypyridine according to the procedure outlined in Example 1a. The crude product was purified by flash chromatography (2X) using (10%MeOH/ $CHCl_3$) to remove the diethyl hydrazinedicarboxylate impurity and give a yellow oil in 31% yield. MS (DCl/ $NH_3$) m/e 193 $(M+H)^+$. $^1H$ NMR ($CDCl_3$, 300 MHz) δ: 8.32 (t, J= 1.5 Hz, 1H), 8.22 (t, J= 3 Hz, 1H), 7.24-7.2 (m, 2H), 4.14-4.05 (dd, J= 9, 6 Hz, 1H), 4.00-3.93 (dd, J= 9, 6 Hz, 1H), 3.24-3.14 (m, 1H), 2.81-2.7 (m, 1H), 2.54 (s, 3H), 2.44-2.31 (m, 1H), 2.14-2.00 (m, 1H), 1.96-1.71 (m, 3H).

4b. 3-(1-Methyl-2-(S)-pyrrolidinylmethyloxy)pyridine fumarate

The product of Example 4a was dissolved in anhydrous MeOH and brought to 0°C with stirring. Fumaric acid was dissolved in MeOH with sonication and added dropwise to the solution containing amine. The mixture was warmed to room temperature with stirring. After 30 minutes the solvent was evaporated *in vacuo* and the remaining solid was vacuum filtered. The solid was then recrystallized with $MeOH/Et_2O$ to give the desired product as a white powder (21% yield). m.p.= 124-125°C. $[\alpha]^{25}_D$ = -3.9° (c=1, MeOH). MS ($DCl/NH_3$) m/e 193 $(M+H)^+$. $^1H$ NMR ($D_2O$, 300 MHz) δ: 8.43 (br s, 1H), 8.33 (d, J= 4.5 Hz, 1H), 7.88-7.84 (m, 1H), 7.3 (dd, J= 8.82, 5.15 Hz, 1H), 6.58 (s, 2H), 4.59 (dd, J=11,3 Hz, 1H), 4.42 (dd, J=11,5.88 Hz, 1H), 4.05-3.9 (m. 1H), 3.82-3.74 (m, 1H), 3.34-3.22 (m, 1H), 3.05 (s, 3H), 2.47-2.37 (m, 1H), 2.30-2.06 (m, 3H). Analysis calculated for $C_{11}H_{16}N_2O \bullet C_4H_4O_4$: C, 58.43; H, 6.54; N, 9.09; Found: C, 58.32; H, 6.67; N, 8.99.

Example 5

2-(1-Methyl-2-(R)-pyrrolidinylmethyloxy)-6-chloropyridazine oxalate

5a. 2-(1-Methyl-2-(R)-pyrrolidinylmethyloxy)-6-chloropyridazine

(R)-1-Methyl-2-pyrrolidinemethanol (300 mg, 2.61 mmol) was dissolved in anhydrous THF and brought to 0°C with stirring.
NaH ((80% dispersion in mineral oil) .082g, 2.9 mmol) was added and the mixture was slowly warmed to room temperature with stirring. After 30 minutes a THF solution 3, 6-dichloropyridazine (.41g, 2.74 mmol) was added to the mixture via syringe. The reaction was allowed to stir for 48 hrs. The solvent was then evaporated *in vacuo* and the mixture diluted with chloroform, washed with saturated $NaHCO_3$ and then with brine. The organic layer was dried over $MgSO_4$. The resulting crude material was purified by flash chromatography (10% $MeOH/CHCl_3$) to give 0.25g (42% yield) of the title compound as a cream colored solid.. MS ($DCl/NH_3$) m/e 228 $(M+H)^+$.$[\alpha]^{25}_D$ = +32° (c=1, MeOH).

5b. 2-(1-Methyl-2-(R)-pyrrolidinylmethyloxy)-6-chloropyridazine oxalate

The product of Example 5a was dissolved in anhydrous ether and cooled to 0°C with stirring. Oxalic acid, pre-dissolved in ether, was added dropwise to the solution and mixture warmed to room temperature. After 30 minutes of stirring, the solvent was removed *in vacuo* and the resulting solid was recrystallized with $MeOH/Et_2O$ to yield 0.28g (82% of the title compound as a white powder. m.p.= 153-154°C. MS ($DCl/NH_3$) m/e 228 $(M+H)^+$. $^1H$ NMR ($D_2O$, 300 MHz) δ: 7.76 (d, J=9 Hz, 1H), 7.36 (d, J=9 Hz, 1H), 4.83 (dd, J=12, 3 Hz, 1H), 4.66 (dd, J=12, 6 Hz, 1H), 4.0-3.92 (m,

1H), 3.8-3.72 (m, 1H), 3.3-3.21 (m, 1H), 3.04 (s, 3H), 2.46-2.37 (m, 1H), 2.28-2.02 (m, 3H). Analysis calculated for $C_{10}H_{14}N_3OCl\ C_2H_2O_4$: C, 45.36; H, 5.08; N, 13.23; Found C, 45.46; H, 5.02, N, 13.26.

Example 6

1-(Methyl)-2-(S)-(1-(3'-pyridyloxy)ethyl)pyrrolidine hydrochloride

6a. N-(Carbobenzyloxy)-2-(1'-hydroxyethyl)-pyrrolidine.

To a solution of N-carbobenzyloxy prolinal (1.0g, 4.28 mmol, see Mori *et al*., *Tetrahedron*, **1985**, 41:5465) in $Et_2O$ (15 mL) at 0 °C was added methyl Grignard (1.4M in THF/toluene; 3.36 mL, 4.71 mmol), and two more 1 mL aliquots of methyl Grignard were added at 5 minute intervals. The reaction mixture was then diluted with $Et_2O$ (100 mL) and washed with 50 mL portions of 5% aq. HCl (1X), and brine (1X), dried ($MgSO_4$), and concentrated to afford the crude product as a clear oil (956 mg). Chromatographic purification (silica, EtOAc/Hex 1:2) afforded the product as a mixture of diastereomers (432 mg, 40% yield). $^1$H-NMR ($CDCl_3$) δ: 7.36 (m, 5H); 5.15 (s, 2H); 4.02 (br s, 1H); 3.86-3.39 (m, 4H); 2.06-1.60 (m, 4H); 1.18, 1.11 (two d, 3). MS(DCl/NH3) m/e 250 $(M+H)^+$.

6b. N-(Methyl)-2-(S)-(1-hydroxyethyl)pyrrolidine.

To a solution of the product of Example 6a (224 mg, 0.898 mmol) in $Et_2O$ (6 mL) was added LAH (-50 mg, excess) and the reaction mixture refluxed for 0.5 hours, then stirred at room temperature for 17 hours. The reaction mixture was then quenched with $Na_2SO_4$ decahydrate (~500 mg) followed by EtOAc (6 mL), then filtered and the salts washed.with ethanol. The combined washes were combined and concentrated to afford the crude product as a gel (138 mg). $^1$H-NMR ($CDCl_3$) δ: 3.87, 3.72, 3.40, 3.05 (m, 2); 2.47, 2.32 (two s, 3); 2.44-2.33 (m, 1); 1.98-1.84 (m, 1); 1.83-1.63, 1.55-1.45 (m, 4); 1.16, 1.12(two d, 3). MS(DCl/$NH_3$) m/e 130 $(M+H)^+$.

6c. N-(Methyl)-2-(S)-(1-(3'-pyridyloxy)ethyl)pyrrolidine hydrochloride

To a solution of triphenylphosphine (353 mg, 1.34 mmol) in THF (4 mL) at 0°C was added DEAD (212 µL, 1.34 mmol), and after 3 minutes a solution of the product of Example 6b (116 mg, 0.897 mmol) and 3-hydroxypyridine (94 mg, 0.98 mmol) in THF (5 mL) was added. The reaction mixture was stirred at 0 °C for 6 hours then at room temperature for 15 hours. The reaction mixture was then diluted with $CH_2Cl_2$ (30 mL) and washed with 10-mL portions of 10% aq. MaOH (2X) followed by 10% aq. HCl (3X). The combined acidic aqueous layers were basified to pH 14 and extracted with $CH_2Cl_2$ (3x10 mL), the combined $CH_2Cl_2$ washes dried (MgS04) and concentrated to afford the crude product. Treatment with 40% KOH (to remove DEAD by-product) followed by chromatographic purification (silica, EtOAc/EtOH/.$NH_4OH$ 100:30:0.5) afforded the product as a mixture of diastereomers, which was convened to the hydrochloride salt as a white hygroscopic solid. (9 mg, 4% yield). $^1$H-NMR ($D_2O$) δ: 8.58, 8.47, 8.26, 8.04 (four m, 4), 5.07-4.89 (m, 1); 3.97-3.52 (m, 2); 3.34-3.14 (m, 1); 3.09, 2.93 (two s, 3); 3.07-2.84 (m, 1); 2.53-1.78 (m, 4); 1.50, 1.44 (two d, 3). MS(DCl/NH3) m/e 207 $(M+H)^+$.

Example 7

2-(2-(S)-azetidinylmethyloxy)pyrazine hydrochloride

7a. N-t-Butyloxycarbonyl-2-(S)-azetidinecarboxylic acid

To an ice cooled solution of 2-(S)-azetidinecarboxylic acid (10.15g, 100.39 mmol) in 1,4 dioxane:water (300ml, 1:1) was added di-*tert*-butyl dicarbonate (28.48g, 130.51 mmol), followed by 4-methylmorpholine (11.68g, 115.45 mmol). The reaction mixture continued to stir 18 hours, gradually warming to room temperature. The reaction mixture was then poured into a ice cooled saturated solution of sodium bicarbonate (250 ml) and washed with ethyl acetate (3x250ml). The aqueous was then acidified with potassium hydrogen sulfate (pH=1) and the product extracted with ethyl acetate (3x300ml). These extracts were then dried ($Na_2SO_4$), filtered and concentrated *in vacuo.* The resulting semisolid was carried forward without further purification. MS (DCl/$NH_3$) m/e 202 $(m+H)^+$, 219 $(m+NH_4)^+$. $^1$H NMR ($CDCl_3$, 300MHz) δ: 10.0 (br s, 1H), 4.81-4.76 (t, J=15Hz, 1H), 3.99-3.83 (m, 2H), 2.62-2.38 (m, 2H), 1.48 (s, 9H).

7b. 1-t-Butyloxycarbonyl-2-(S)-hydroxymethyleneazetidine

To an ice-cooled solution of the product of Example 7a (9.39g, 46.72 mmol) in tetrahydrofuran (100ml) was

added one molar solution of borane/THF complex (210ml, 4.5eq.) under nitrogen. The reaction was stirred 48 hours, gradually warming to room temperature, after which a 10% aqueous potassium hydrogen sulfate solution was added (60 ml) gradually, then the volatiles were evaporated *in vacuo*,. The remaining slurry was diluted with ethyl acetate (100ml), and triturated two additional times . The organic phase was then washed with a saturated solution of sodium hydrogen carbonate (3x75 ml), dried ($MgSO_4$), filtered and concentrated *in vacuo,* yielding a colorless oil (8.4 g, 96% yield). This material was carried forward without further purification. MS ($DCl/NH_3$) m/e 188$(m+H)^+$. $^1H$ NMR ($CDCl_3$, 300MHz) δ: 4.49-4.40 (ddd, J=9.0 Hz, J=9.0 Hz, J=3.0 Hz, 1H), 3.95-3.68 (m, 4H), 2.23-2.12 (m, 1H), 1.99-1.87 (m, 1H), 1.46 (s, 9H).

7c. 2-(1-t-Butyloxycarbonyl-2-(S)-azetidinylmethyloxy)pyrazine

To an ice cooled solution of the product of Example 7b (941 mg, 5.0 mmol) in tetrahydrofuran (30 ml) under nitrogen, was added sodium hydride (80% dispersion in mineral oil, 180mg, 7.5 mmol), portionwise. The reaction mixture stirred fifteen minutes followed by the dropwise addition of 2-chloropyrazine (590mg, 5.1 mmol). The reaction was stirred 24 hours gradually warming to room temperature. Additional sodium hydride (40 mg) was added and stirring at room temperature continued until complete disappearance of starting material by TLC. The reaction mixture was then treated with a 10% aqueous solution of potassium hydrogen sulfate (20ml) and the volatiles evaporated *in vacuo*. The mixture was then diluted with ethyl acetate (50ml) and the organic phase was washed with saturated sodium hydrogen carbonate (2x20ml). The organic phase was then dried ($MgSO_4$), filtered and concentrated *in vacuo.* The resultant orange oil (1.37g) was purified by flash silica gel chromatography (ethyl acetate:hexane=1:1), resulting In product isolated as an oil, in 80% yield (1.07g). $[\alpha]^{23°}_D$ = -62.8°(c 1.35, $CHCl_3$). MS ($CI/NH_3$) m/e 266 $(M+H)^+$, 283 $(M+NH_4)^+$. $^1H$ NMR ($CDCl_3$, 300 MHz) δ 8.28- 8.27 (d, J=1.1Hz, 1H), 8.13-8.12 (d, J=2.58Hz, 1H), 8.08-8.06 (dd, J= 2.58, J=1.5Hz, 1H), 4.66-4.61 (dd, J=10.83, J=4.6, 1H), 4.58-4.51 (m, 1H), 4.50-4.45 (dd, J=10.83, J=3.13, 1H), 3.92-3.87 (dd, J=8.3, J=6.25, 2H), 2.41-2.29 (m, 1H), 2.26-2.16 (m, 1H), 1.41 (s, 9H).

7d. 2-(2-(S)-azetidinylmethyloxy)pyrazine hydrochloride

The product of Example 7c (335mg, 1.26 mmol) was stirred in methylene chloride (2ml) and cooled to 0°C. To this was added trifluoroacetic acid in methylene chloride (3ml, 1:1). The reaction was allowed to stir 18 hours, gradually warming to room temperature. The mixture was then basified (pH=9) with a saturated solution of sodium hydrogen carbonate and continuously extracted with methylene chloride for 16 hours. The extract was dried ($MgSO_4$), filtered and concentrated *in vacuo.* The resultant oil was not purified further, but was was dissolved in ethanol (2ml) and immediately treated with diethyl ether saturated with hydrogen chloride gas. Crystals began to form immediately. Recrystallization from ethanol and diethyl ether yielded pure product (51.2 mg, 0.256 mmol, 80% yield) as the hydrochloride salt. mp (dec) MS ($DCl/NH_3$) m/e 166 $(M+H)^+$, 183 $(M+NH_4)^+$. $^1H$ NMR ($D_2O$, 300 MHz) δ: 8.37 (s, 1H), 8.23 (s, 2H), 4.97-4.92 (m, 1H) 4.74-4.58 (m, 2H), 4.19-4.00 (m, 2H), 2.72-2.62 (dd, J=16.8 Hz, J=8.8 Hz, 2H). Anal. calcd. for $C_8H_{11}N_3O$•2.4 HCl: C, 38.02; H, 5.34; N, 16.63. Found: C, 37.99; H, 5.37; N, 16.60.

Example 8

2-(1-Methyl-2-(S)-azetidinylmethyloxy)pyrazine hydrochloride

The product of Example 7a (102.9 mg, 0.62 mmol) was stirred with excess paraformaldehyde in ethanol (5 ml), on ice and under nitrogen, and the pH adjusted to five with the addition of acetic acid and sodium acetate. The reaction was stirred for 15 minutes and sodium cyanoborohydride (59mg, 0.936 mmol) was added. A very small amount of bromocresol green was added directly to the reaction mixture as indicator. The reaction was stirred 18 hours, allowed to warm to room temperature, and additional formaldehyde (0.25 ml) and sodium cyanoborohydride (20 mg, 0.32 mmol) was added to push the reaction to completion. The reaction mixture was then acidified (pH=1) with 10% solution of potassium hydrogen sulfate and the volatiles evaporated. The aqueous phase was washed with ethyl acetate (3x10ml), basified with sodium carbonate (pH=9.5), and products extracted with ethyl acetate (5x15ml). These extracts were dried ($MgSO_4$), filtered, and concentrated *in vacuo*. The resultant oil (111.7mg, 100% yield), was then purified by flash silica gel chromatography (chloroform:methanol:ammonium hydroxide/98:2:0.1), yielding the pure product (60.1 mg, 54% yield), which was dissolved in ethanol and converted to the hydrochloride salt in a similar manner as that of Example 7d, (62.2 mg, 86% yield). mp 161-162°C (dec). MS ($DCl/NH_3$) m/e 180 $(M+H)^+$. $^1H$ NMR ($D_2O$, 300 MHz) δ: 8.37 (S, 1H), 8.24 (s, 2H), 4.87-4.76 (m, 1H), 4.32-4.23 (ddd, J=10 Hz, J= 10 Hz, J=6 Hz, 1H), 4.04-3.95 (dd, J=19.5 Hz, J=9.6 Hz, 1H), 2.97 (s, 3H), 2.70-2.58 (m; 2H). Anal. calcd. for $C_9H_{13}N_3O$·2.0 HCl·0.3 $H_2O$: C, 41.97; H, 6.10; N, 16.32. Found: C, 42.25; H, 5.93; N, 16.02.

Example 9

3-(2-(S)-azetidinylmethyloxy)pyridine hydrochloride

9a. 3-(1-t-Butyloxycarbonyl-2-(S)-azetidinylmethyloxy)pyridine

An ice cooled solution of the product of Example 7b (2.8 g, 14.97 mmol) in tetrahydrofuran (40ml) was stirred under a nitrogen atmosphere To this was added DEAD (3.54 ml, 22.46 mmol) followed by triphenylphosphine (4.78 g, 22.46 mmol) and the mixture was allowed to stir 10 minutes. 3-Hydroxypyridine (2.14 g, 22.46 mmol) was then added to the reaction with additional tetrahydrofuran (40 ml). After 18 hours, additional 3-hydroxypyridine (0.1 g, 1.05 mmol) was added and the reaction stirred 24 hours longer. When all starting azetidine alcohol was consumed, the reaction mixture was concentrated *in vacuo*. The crude mixture was then acidified (pH<2) with a 10% solution of potassium hydrogen sulfate (80ml), and washed with ethyl acetate (3x75ml). The aqueous portion was then basified with a saturated solution of potassium carbonate (pH=10) and products extracted with ethyl acetate (4x75ml). These extracts were dried ($MgSO_4$), filtered and concentrated *in vacuo* to a red-brown oil (1.84g, 50% yield). The crude product was purified via flash silica gel chromatography Rf=0.19, (ethyl acetate:hexane=2:1), yielding product as a light yellow oil in 25% yield. MS ($DCI/NH_3$) m/e 265 $(M+H)^+$, 282 $(M+NH_4)^+$. $^1H$ NMR ($CDCl_3$) δ: 8.36-8.35 (dd, J=3.7 Hz, J=0.7 Hz, 1H), 8.24-8.22 (dd, J=4.0 Hz, J=1.5 Hz, 1H), 7.25-7.22 (m, 2H), 4.56-4.48 (m, 1H), 4.36-4.31 (dd, J=10 Hz, J=4.9 Hz, 1H), 4.17-4.12 (dd, J=10 Hz, J=2.9 Hz, 1H), 3.92-3.87 (dd, J=8.2 Hz, J=6.8 Hz, 2H), 2.42-2.25 (m, 2H), 1.42 (s, 9H).

9b. 3-(2-(S)-azetidinylmethyloxy)pyridine hydrochloride

To an ice cooled solution of the product of Example 9a (286mg, 1.08 mmol) in absolute ethanol (4ml), was added a hydrogen chloride saturated ethanol solution (4ml), under nitrogen. The reaction mixture stirred 18 hours gradually warming to room temperature. The reaction mixture was then concentrated *in vacuo,* the product dissolved in absolute ethanol and triturated with diethyl ether. Two recrystallizations from ethanol and diethyl ether yielded pure product in 81% yield as a white powder (174mg, 87 mmol). MS ($DCI/NH_3$) m/e 165 $(M+H)^+$, 182 $(M+NH_4)^+$. $^1$NMR ($D_2O$, 300 MHz) δ: 8.60-8.59 (d, J=2.9 Hz, 1H), 8.48-8.46 (d, J=5.8 Hz, 1H), 8.25-8.21 (ddd, J=9.0 Hz, J=2.6 Hz, J=1.1 Hz, 1H), 5.05-4.97 (m, 1 H), 4.59-4.57 (d, J= 4.0 Hz, 2H), 4.22-4.05 (m, 2H), 2.77-2.67 (dd, J=16.9 Hz, J=8.45 Hz, 2H). Anal. calcd. for $C_9H_{12}N_2O{\cdot}2.7HCl{\cdot}0.2\ H_2O$: C, 40.60; H, 5.71; N, 10.52. Found: C, 40.75; H, 5.76; N, 10.51.

Example 10

3-(1-Methyl-2-(S)-azetidinylmethyloxy)pyridine hydrochloride

To an ice cooled solution of the product of Example 9a (550mg, 1.89 mmol) in methylene chloride (3 ml) under nitrogen, was added trifluoroacetic acid in methylene chloride (5 ml, 1:1). The reaction was allowed to stir 18 hours, gradually warming to room temperature. The reaction mixture was then concentrated *in vacuo*, brought up in absolute ethanol (5ml). Paraformaldehyde was added, and the acidity adjusted to pH 5 with the addition of acetic acid and sodium acetate. The reaction mixture was stirred for 15 minutes and sodium cyanoborohydride (180mg, 2.86 mmol) was added. A small amount of bromocresol green was added to the reaction mixture as indicator. The mixture stirred 18 hours after which time the reaction was complete as monitored by TLC. The reaction mixture was then acidified (pH=1) with saturated solution of potassium hydrogen sulfate, and the volatiles evaporated *in vacuo*. The aqueous phase was then washed with ethyl acetate (3x20 ml), basified (pH=10) with potassium carbonate, and the crude products extracted with ethyl acetate (4x20 ml). The extracts were dried ($MgSO_4$), filtered and concentrated *in vacuo* (274 mg, 81% yield). The crude product was then purified by flash silica gel chromatography, yielding a colorless oil (147 mg, 44% yield).This oil was dissolved in absolute ethanol (1.5 ml) and treated with hydrogen chloride saturated diethyl ether. After one recrystallization from ethanol and diethyl ether, pure product resulted in the form of fine hygroscopic needles. MS ($CI/NH_3$) m/e 179 $(M+H)^+$, 196 $(M+NH_4)^+$. $^1H$ NMR ($D_2O$/300MHz) δ 8.53 (d, J=2.2Hz, 1H), 8.42-8.41 (d, J=5.5 Hz, 1H), 8.09-8.05 (dd, J=8.8 Hz, J=3.0 Hz, 1H), 7.91-7.86 (dd, J=8.8 Hz, J=5.4 Hz, 1H), 4.90-4.80 (m, 1H), 4.63-4.58 (dd, J=11.8 Hz, J=2.9 Hz, 1H), 4.56-4.50 (dd, J=11.8 Hz, J=5.3 Hz, 1H), 4.34-4.25 (ddd, J=9.9 Hz, J=9.9 Hz, J=5.1, 1H), 4.06-3.97 (dd, J=19.5 Hz, J=9.4 Hz, 1H), 3.00 (s, 3H), 2.77-2.60 (m, 2H). Anal. calc. for $C_{10}H_{14}N_2O{\bullet}2.0\ HCl{\bullet}0.7\ H_2O$: C, 45.54; H, 6.65; N, 10.62. Found: C, 45.38; H, 6.35; N, 10.53.

Example 11

2 - (1-Methyl-2-(S)-pyrrolidinylmethyloxy)thiazole hydrochloride

(S)-(-)-1-Methyl-2-pyrrolidine methanol (484 mg, 4.2 mmol) was reacted with NaH ((80% dispersion in mineral oil) 0.164 g 5.46 mmol) and 2-bromothiazole (0.417 g, 4.62 mmol) according to the procedure outlined in Example 3a to afford after column chromatography eluting with (10% MeOH/$CHCl_3$ + 1%$NH_3$) (616.2 mg, 3.1 mmol, 74% yield) of 2-(1-methyl-2-(S)-pyrrolidinylmethyloxy)-thiazole. The pure base was then converted to its hydrochloride salt by treating the amine (109.4 mg, 0.552 mmol) with a saturated solution of HCl in $Et_2O$ dropwise with stirring until no more precipitate formation was observed. This afforded 21.2 mg, 0.09 mmol, 16% of the title compound, mp = 99°C. $[\alpha]^{D}_{20}$ = +2.86° (c=0.021, MeOH). MS (DCl/$NH_3$) m/e 199 $(M+H)^+$. $^1H$ NMR ($CD_3OD$, 300 MHz) δ: 7.18 (d, J=4.04 Hz, 1H), 6.99 (d, J=4.04 Hz, 1H), 4.85 (dd, J=12.50, 3.31 Hz, 1H), 4.65 (dd, J=12.5, 6.62 Hz, 1H), 4.09-3.85 (m, 1H), 3.85-3.65 (m, 1H), 3.03 (br s, 3H), 2.45-2.3 (m, H), 2.3-1.95 (m, 3H). Analysis calculated for $C_9H_{15}N_2OSCl$•0.6 $H_2O$•0.1$Et_2O$: C, 44.63; H, 6.85; N, 11.07; Found: C, 44.68; H, 6.46; N, 10.68.

Example 12

2-(1-Methyl-2-(S)-pyrrolidinylmethyloxy)-6-chloropyridazine fumarate

12a. 2-(1-Methyl-2-(S)-pyrrolidinylmethyloxy)-6-chloropyridazine

(S)-(-)-1-Methyl-2-pyrrolidinemethanol (968 mg, 8.4 mmol) was reacted in a similar fashion as.that described for the (R)-isomer of Example 5a to afford 1.31g (69% yield) of the title compound. $[\alpha]^{25}$D = -28.30 (c=1.1, MeOH).

12b. 2-(1-Methyl-2-(R)-pyrrolidinylmethyloxy)-6-chloropyridazine fumarate

The product of Example 12a (37.3 mg, 0.16 mmol) was reacted with 19 mg of fumaric acid in a process similar to that described in Example 4b to afford 39 mg (70% yield) of the title compound as a white solid, m.p.= 155°C. $[\alpha]^{D}$20 = +4.80º (c=1.0, MeOH). MS (DCl/$NH_3$) m/e 228 $(M+H)^+$. $^1H$ NMR ($D_2O$, 300 MHz) δ: 7.6 (d, J=9.19 Hz, 1H), 7.37 (d, J=9.19 Hz, 1H), 6.64 (s, 2H), 4.82 (dd, J=12.14, 3.1 Hz, 1H), 4.66 (dd, J=12.13, 5.88 Hz, 1H), 4.0-3.92 (m, 1H), 3.8-3.72 (m, 1H), 3.3-3.21 (m, 1H), 3.04 (s, 3H), 2.46-2.36 (m, 1H), 2.27-2.02 (m, 3H). Analysis calculated for $C_{14}H_{18}N_3O_5Cl$: C, 48.91; H, 5.27; N, 12.22; Found: C, 48.86; H, 4.87; N, 11.98.

Example 13

6-Chloro-3-(1-Methyl-2(S)-methyloxyazetidinyl)pyridazine oxalate

13a. 6-Chloro-3-(1-t-butyloxycarbonyl-2(S)-methyloxyazetidinyl)pyridazine

The product from Example 7b (3.18 g, 17.0 mmol), sodium hydride (80% dispersion in mineral oil, 510 mg, 17.0 mmol), and 3,6-dichloropyridazine (3.8 g, 25.5 mmol) were combined in a similiar manner as that described in Example 7c. The crude product was purified by flash chromatography on silica gel using EtOAc/hexane (1:6 to 1:4) as the elutant to give 3.87 g of a viscous oil, which solidified in the refrigerator (76% yield). TLC $R_f$=0.58 (EtOAc/hexane 1:1). m.p. = 50-54°C. MS (Cl) m/e 300 $(M+H)^+$. $^1H$ NMR (DMSO-$d_6$, 300 MHz) δ: 7.73 (d, J=9.2 Hz, 1H), 7.31 (d, J=9.2 Hz, 1H), 4.72 (dd, J=11.0 Hz, 4.6 Hz, 1H), 4.59 (dd, J=11.0 Hz, 3.7 Hz, 1H), 4.56-4.50 (m, 1H), 3.79 (t, J=7.6 Hz, 2H), 2.40-2.38 (m, 1H), 2.20-2.09 (m, 1H), 1.36 (s, 9H).

13b. 6-Chloro-3-(1-methyl-2(S)-methyloxyazetidinyl)pyridazine

The product from Example 13a (805 mg, 2.68 mmol) was treated in a similiar fashion as that described under Example 7d. The crude was subject to flash chromatography on silica gel with 10% MeOH in $CHCl_3$ to 0.5% $NH_4OH$ in 10% MeOH in $CHCl_3$ used as the elutant to give 352 mg of a yellow oil (66% yield). MS (Cl) m/e 200 $(M+H)^+$. The amine deprotected material (330 mg, 1.66 mmol) was then subjected to reductive amination conditions previously described in Example 8. The crude product was purified by flash chromatography on silica gel using 2% MeOH in $CHCl_3$ to 5% MeOH in $CHCl_3$ as the elutant to give 125 mg of a clear oil (35% yield). MS (Cl) m/e 214 $(M+H)^+$. $^1H$ NMR ($CDCl_3$, 300 MHz) δ 7.36 (d, J=9.2 Hz, 1H), 7.02 (d, J=9.2 Hz, 1H), 4.54 (dd, J=11.6 Hz, 3.8 Hz, 1H), 4.47 (dd, J=11.6 Hz, 5.7 Hz, 1H), 3.48-3.39 (m, 2H), 2.89-2.81 (m, 1H), 2.38 (s, 3H), 2.17-2.03 (m, 2H).

13c. 6-Chloro-3-(1-methyl-2(S)-methyloxyazetidinyl)pyridazine oxalate

The product of Example 13b (120 mg, 0.56 mmol) was dissolved in 8 mL of diethyl ether and cooled to 0°C. Oxalic acid (55.5 mg, 0.62 mmol) in 1 mL of diethyl ether was added dropwise to the reaction vessel, and the reaction was allowed to stir for 30 minutes. The solvent was then removed in *vacua* and the remaining white solid recrystallized out of hot methanol to give 128 mg of the titile compound (75% yield). m.p. = 165-167°C. $[\alpha]^{23}_D$ = -26.7° (c 0.75, $H_2O$). MS (Cl) m/e 214 (M+H)+. $^1H$ NMR ($D_2O$, 300 MHz) δ: 7.78 (d, J=9.2 Hz, 1H), 7.40 (d, J=9.2 Hz, 1H), 4.89-4.73 (m, 3H), 4.32-4.22 (m, 1H), 4.05-3.94 (m, 1H), 2.97 (s, 3H), 2.73-2.60 (m, 2H). Anal cal'd for $C_{11}H_{14}ClN_3O_5$: C, 43.50; H, 4.65; N, 13.84. Found: C, 43.59; H, 4.50; N, 13.65.

Example 14

3-(2-(S)-pyrrolidinylmethyloxy)pyridine furmarate

14a. 3-(1-t-butoxycarbonyl-2-(S)-pyrrolidinylmethyloxy) pyridine

To a solution of triphenylphosphine (1.97g, 7.5 mmol) in 30 mL of tetrahydrofuran at room temperature was added diethyl azodicarboxylate (DEAD) (1.13 mL, 7.5 mmol) dropwise with stirring. After stirring at room temperature for 30 minutes, (S)-1-t-butoxycarbonyl-2-pyrrolidinemethanol (1 g, 5.0 mmol) and 3-hydroxypyridine (713 mg, 7.5 mmol) were added to the reaction mixture. The resultant solution was stirred at room temperature for 16 hr. After all the starting material was consumed, the organic solvent was evaporated *in vacuo*. The residue was purified by silica gel column chromatography. Elution with chloroform:methanol (10 :1) provided 2 g of the title compound. MS (DCl/NH3) m/e 279 (M+H)+.

14b. 3-(2-(S)-pyrrolidinylmethyloxy)pyridine

To a solution of the product of step 14a in 12 mL of methylene chloride solution was added 12 mL of trifluoroacetic acid. The resultant solution was stirred at room temperature for 3 hr. Evaporation of both solvent and trifluoroacetic add gave a brown oil which was basified with saturated ammonium hydroxide solution. This oil was purified by silica gel column chromatography. Elution with a mixture of chloroform:methanol: ammonium hydroxide (10:1:0.1) provided the desired product . MS (DCl/$NH_3$) m/e 179 $(M+H)^+$. $^1H$ NMR ($D_2O$, 300 MHz) δ: 8.33 (br s, 1H), 8.22 (m, 1H), 7.22(m, 2H), 3.82-4.03 (m, 2H), 3.50-3.61 (m, 1H), 2.92-3.10(m, 2H), 1.70-2.10 (m, 4H), 1.51-1.66 (m, 1H).

14c. 3-(2-(R)-pyrrolidinylmethyloxy)pyridine fumarate

The product of step 14b (281 mg, 1.57 mmol) was dissolved in anhydrous MeOH and brought to 0°C with stirring. Fumaric add was dissolved in MeOH with sonication and added dropwise to the base. The mixture was warmed to room temperature with stirring. After 30 minutes the solvent was evaporated *in vacua,* and the remaining solid was triturated with anhydrous diethyl ether. The product was obtained as a hygroscopic solid in 47% yield (218 mg). MS (DCl/$NH_3$) m/e: 179 $(M+H)^+$. $^1H$ NMR ($D_2O$, 300 MHz) δ: 8.38 (m, 1H), 8.30 (m 1H), 7.76-7.72 (m, 1H), 7.72-7.62 (m, 1H), 6.57 (s, 2H), 4.60-4.50 (m, 1H), 4.35-4.25 (m, 1H), 4.10-4.08 (m, 1H), 3.42 (t, J=7.5 Hz, 2H), 2.37-2.23 (m, 1H), 2.23-2.06 (m, 2H), 2.06-1.98 (m, 1H). Anal calc. for $C_{10}H_{14}N_2O•C_2H_4O_2•0.9\ H_2O$: C, 54.15; H, 6.42; N, 9.02; Found: C, 54.48; H, 6.02; N, 8.67.

Example 15

5-Chloro-3-(2-(S)-pyrrolidinylmethyloxy)pyridine dihydrochloride

15a. (S)-1-t-Butoxycarbonyl-2-pyrrolidinemethanol

N-t-BOC-proline (Sigma Chemical Co., 12.97 g, 60.02 mmol) was dissolved in anhydrous THF and brought to 0°C with stirring. Borane/THF complex was added dropwise via syringe over a 10 minute period. The reaction mixture was stirred at room temperature for 1 hour, then the reaction was quenched slowly with saturated $NaHCO_3$ and stirred for an additional hour. The solvent was removed *in vacuo,* and the residue was diluted with $H_2O$. The desired compound was extracted from the aqueous phase with $Et_2O$ (3X). The organic layer was then washed with brine (2X) dried ($MgSO_4$) and evaporated. The resulting material was carried on without further purification.

15b. 5-Chloro-3-(N-t-butoxycarbonyl-2-(S)-pyrrolidinylmethyloxy)pyridine

(S)-1-t-Butoxycarbonyl-2-pyrrolidinemethanol (1.75 g, 8.71 mmol) from Example 15a and 5-chloro-3-pyridinol (1.69 g, 13.10 mmol, Aldrich Chemical Co.). were allowed to react in the presence of triphenylphosphine and DEAD as described in Example 2a. The crude product was purified by chromatography over silica gel eluted with hexane/EtOAc to provide 1.49 g (60%) of the title compound as a pale yellow oil. TLC $R_f$ 0.75 (1:1 EtOAc/Hex). MS ($DCl/NH_3$) m/e 313 $(M + H)^+$ with $^{35}Cl$ and m/e 315 $(M + H)^+$ with $^{37}$ Cl.

15c. 5-Chloro-3-(2-(S)-pyrrolidinylmethyloxy)pyridine

The product of example 15b (0.440 g, 1.41 mmol) was treated with trifluoroacetic acid in $CH_2Cl_2$ as described in Example 2b, then saturated $K_2CO_3$ was added and the aqueous phase was extracted with $CH_2Cl_2$ (3X). The organic layer was dried ($MgSO_4$) and evaporated, and the crude product was purified by chromatography over silica gel eluted with $CHCl_3/MeOH/NH_4OH$/to give 0.299g (100%) of the title compound as a pale yellow oil. MS ($DCl/NH_3$) m/e: 213 $(M + H)^+$ with $^{35}Cl$ and 215 $(M + H)^+$ with $^{37}Cl$. $^1H$ NMR ($CDCl_3$, 300 MHz) $\delta$: 8.22 (d, J=2.6 Hz, 2H), 8.19 (d, J=2.2 Hz, 1H), 7.22 (t, J= 2.50 Hz, 1H), 3.97 (dd, J=9, 5 Hz, 1H), 3.90 (dd, J= 9, 7 Hz, 1H), 3.60-3.51 (m, 1H), 3.08-2.95 (m, 2H), 2.32 (br s, 1H), 2.03-1.74 (m, 3H), 1.62-1.51 (m, 1H). $[\alpha]^{25}_D$=+13.94° (c=1.04, MeOH).

15d. 5-Chloro-3-(2-(S)-pyrrolidinylmethyloxy)pyridine dihydrochloride

The product of Example 15c was treated with HCl and isolated as described in Example 1b to afford a cream colored powder. mp 183-186°C. MS ($DCl/NH_3$) m/e: 213 $(M + H)^+$ with $^{35}Cl$ and 215 $(M + H)^+$ with $^{37}Cl$. $^1H$ NMR ($D_2O$, 300 MHz) $\delta$: 8.23 (m, 2H), 7.60 (t, J=2.20 Hz, 1H), 4.47 (dd, J=10.60, 3.70 Hz, 1H), 4.25 (dd, J=10.60, 8 Hz, 1H), 4.14-4.10 (m, 1H), 3.44-3.39 (t, J=7 Hz, 2H), 2.32-2.23 (m, 1H), 2.19-2.07 (m, 2H), 2.01-1.92 (m, 1H). Anal. Calc. for $C_{10}H_{13}N_2OCl\cdot 2.00$ HCl: C, 42.06; H, 5.29; N, 9.81; Found C, 42.47; H, 5.34; N, 9.90. $[\alpha]^{25}_D$=+10.10° (c=1, MeOH).

Example 16

5-Chloro-3-(1-methyl-2-(S)-pyrrolidinylmethyloxy)pyridine dihydrochloride

16a. 5- Chloro-3-(1-methyl-2-(S)-pyrrolidinylmethyloxy)pyridine

To 5-chloro-3-(N-t-butoxycarbonyl-2-(S)-pyrrolidinylmethyloxy)pyridine (0.600 g, 1.92 mmol) from Example 15b was added a solution of formic acid/ formaldehyde (1:2, 3 mL). The mixture was heated at reflux for 3 hours, then basified with sat. $K_2CO_3$. The aqueous phase was extracted with $CH_2Cl_2$ (3X) then the organic layer was dried ($MgSO_4$) and concentrated. The crude product was purified by chromatography over silica gel eluted with CHCl3/MeOH to afford 0.274 g (63%) of the title compound as a pale yellow oil. TLC $R_f$=0.23 (10%MeOH/$CHCl_3$). MS ($DCl/NH_3$) m/e: 227 $(M + H)^+$ with $^{35}Cl$ and 229 $(M + H)^+$ with $^{37}Cl$. $^1H$ NMR ($CDCl_3$, 300 MHz) $\delta$: 8.22 (d, J=2.6 Hz, 1H), 8.19 (d, J=2.2 Hz, 1H), 7.22 (t, J=2 Hz, 1H), 4.00 (dd, J=9, 5.50 Hz, 1H), 3.92 (dd, J=9, 5.20 Hz, 1H), 3.14-3.08 (m, 1H), 2.69-2.64 (m, 1H), 2.47 (s, 3H), 2.36-2.27 (m, 1H), 2.07-1.97 (m, 1H), 1.88-1.69 (m, 3H).

16b. 5-Chloro-3-(1-methyl-(S)-pyrrolidinylmethyloxy)pyridine dihydrochloride

The product of Example 16a was treated with HCl and isolated as described in Example 1b to give a white powder, mp = 200°C (dec). MS ($DCl/NH_3$) m/e: 227 $(M + H)^+$ with $^{35}Cl$ and 229 $(M + H)^+$ with $^{37}Cl$. $^1H$ NMR ($D_2O$, 300 Hz) $\delta$: 8.25-8.24 (m, 2H), 7.63-7.61 (m, 1H), 7.62 (t, J=2.3 Hz), 4.53 (dd, J=11, 3Hz, 1H), 4.36 (dd, J=9, 6 Hz, 1H), 3.94 (m, 1H), 3.76 (m, 1H), 3.17-3.10 (m, 1H), 3.04 (s, 3H), 2.44-2.35 (m, 1H), 2.25-2.02 (m, 3H). Anal. Calc. for $C_{11}H_{15}N_2OCl\cdot 2.00$ HCl: C, 44.10; H, 5.72; N, 9.35; Found C, 44.07; H, 5.69; N, 9.35. $[\alpha]^{25}_D$=-5.60° (c=1, MeOH).

Example 17

2-Methyl-3-(2-(S)-pyrrolidinylmethyloxy)pyridine dihydrochloride

17a. 2-Methyl-3-(1-t-butoxycarbonyl-2-(S)-pyrrolidinylmethyloxy)pyridine

To a solution of triphenylphosphine (3.83 g, 14.6 mmol) in 40 mL of anhydrous THF at 0°C was added diethyl azodicarboxylate (2.30 ml, 14.6 mmol) dropwise. The mixture was stirred at 0°C for 30 minutes, then brought to room temperature. (S)-1-t-Butoxycarbonyl-2-pyrrolidinemethanol (1.96g, 9.75 mmol, Aldrich Chemical Co.) and 2-methyl-3-hydroxypyridine (Aldrich Chemical Co., 1.60g, 14.6 mmol) were added to the reaction vessel, and the mixture was stirred for 16 hours. Solvent was removed *in vacuo,* and the residue was diluted with hexane and sonicated for 30 minutes. The resulting precipitate was filtered and washed with hexane. The hexane was removed *in vacua.* The residue was purified by silica gel flash chromatography (ethyl acetate) to give 1.42g (50% yield) of the title compound as a pale yellow oil. TLC $R_f$=0.50 (EtOAc). MS (DCl/$NH_3$) m/e: 293 $(M + H)^+$.

17b.. 2-Methyl-3-(2-(S)-pyrrolidinylmethyloxy)pyridine

To a solution of the product of Example 17a (0.407g, 1.39 mmol) in 2 mL of methylene chloride at 0°C was added 2 mL of trifluoroacetic acid. The reaction was stirred at this temperature for 40 minutes. The temperature was raised to room temperature, and the reaction was stirred for an additional 30 minutes. Once the starting material was consumed, saturated $K_2CO_3$ was added and the product was extracted from the aqueous phase with $CH_2Cl_2$ (3X). The organic layer was then dried over $MgSO_4$. The resulting crude material was purified by silica gel flash chromatography using a gradient from 100% $CHCl_3$ to 10% MeOH/$CHCl_3$ and finally 1%$NH_4OH$/10%MeOH/$CHCl_3$ to give 0.236g (88%) of the title compound as a pale yellow oil. MS (DCl/$NH_3$) m/e: 193 $(M + H)^+$. $^1H$ NMR ($CDCl_3$, 300 MHz) δ: 8.07 (t, J=3 Hz, 1H), 7.07 (m, 2H), 3.95-3.85 (m, 2H), 3.60-3.55 (m, 1H), 3.10-2.97 (m, 2H), 2.48 (s, 3H), 2.23 (br s, 1H), 2.04-1.93 (m, 1H), 1.90-1.78 (m, 2H), 1.67-1.58 (m, 1H).

17c. 2-Methyl-3-(2-(S)-pyrrolidinylmethyloxy)pyridine dihydrochloride

The free base from Example 17b was dissolved in diethyl ether and brought to 0°C with stirring. The solution was treated with diethyl ether saturated with hydrogen choride gas. The solvent was removed *in vacuo*. The resulting salt was triturated with diethyl ether (2X) and dried under vacuum to give a beige powder. m.p. > 240°C, and decomposition occurs at 250°C and higher. MS (DCl/$NH_3$) m/e: 193 $(M + H)^+$. $^1H$ NMR ($D_2O$, 300 MHz) δ: 8.17 (d, J=5.5 Hz, 1H), 7.86 (d, J=7.5 Hz, 1H), 7.67 (dd, J=8.50, 5.50 Hz, 1H), 4.58 (dd, J=11, 3 Hz, 1H), 4.34 (dd, J=11, 8 Hz, 1H), 4.25-4.19 (m, 1H), 3.46-3.42 (m, 2H), 2.62 (s, 3H), 2.36-2.28 (m, 1H), 2.22-196 (m, 3H). Anal. Calc. for $C_{11}H_{16}N_2O \cdot 2.00$ HCl: C, 48.82; H, 6.84; N, 10.56; Found C, 49.60; H, 6.88; N, 10.44. $[\alpha]^{25}_D$=+21.40° (c=1, MeOH).

Example 18

6-Methyl-3-(2-(S)-pyrrolidinylmethyloxy)pyridine dihydrochloride

18.a. 6-Methyl-3-(1-t-butoxycarbonyl-2-(S)-pyrrolidinylmethyloxy)pyridine

To a solution of triphenylphosphine (3.83g, 14.60 mmol) in 40 ml of anhydrous THF at 0°C was added diethyl azodicarboxylate (2.3 ml, 14.60 mmol) dropwise. The mixture was stirred at 0°C for 30 minutes, then brought to room temperature. (S)-1-t-Butoxycarbonyl-2-pyrrolidinemethanol (1.96g, 9.75 mmol, Aldrich Chemical Co.) and 6-methyl-3-pyridinol (Aldrich Chemical Co.,1.60g, 14.60 mmol) were added to the reaction vessel, and the mixture was stirred for 16 hours. Solvent was removed in vacuo, and the residue was diluted with hexane and sonicated for 30 minutes. The resulting precipitate was filtered and washed with hexane. The hexane was removed *in vacuo.* The residue was purified by silica gel flash chromatography using a gradient from 100% hexane to a 1:1 solution of ethyl acetate/hexane (100% Hex, 10%EtOAc/Hex, 20%EtOAc/Hex, 1:1EtOAc/Hec) to give 1.61 g (57%) of the title compound as a pale yellow oil. TLC Rf=0.42 (1:1 EtOAc/Hex). MS (DCl/NH3) m/e: 293 $(M + H)^+$.

18b. 6-Methyl-3-(2-(S)-pyrrolidinylmethyloxy)pyridine

To a solution of the product of Example 18a above (0.320g, 1.09 mmol) in 2 mL of methylene chloride at 0°C was added 2 mL of trifluoroacetic acid. The reaction was stirred at this temperature for 30 minutes. The temperature was raised to room temperature, and the reaction was stirred for an additional 30 minutes. Once the starting material was consumed, saturated $K_2CO_3$ was added, and the product was extracted from the aqueous phase with $CH_2Cl_2$ (3X). The organic layer was then dried over $MgSO_4$. The resulting crude material was purified by silica gel flash chromatography using a gradient from 100% $CHCl_3$ to 10% MeOH/$CHCl_3$ and finally 1%$NH_4OH$/10%MeOH/$CHCl_3$ to give 0.200g (95%) of the title compound as a pale yellow oil. MS (DCl/$NH_3$) m/e: 193 (M + H)+. $^1H$ NMR ($CDCl_3$, 300 Mz) δ: 8.20 (d, J=3 Hz, 1H), 7.12 (dd, J=8.50, 3 Hz, 1H), 7.05 (d, J=8.50 Hz, 1H), 3.94 (dd, J=9, 5 Hz, 1H), 3.87 (dd, J=9, 7 Hz, 1H), 3.57-3.48 (m, 1H), 3.07-2.92 (m, 2H), 2.48 (s, 3H), 2.18 (br s, 1H), 2.01-1.89 (m, 1H), 1.88-1.71 (m, 2H), 1.62-1.51 (m, 1H). $[\alpha]^{25}_D$=+11.40° (c=1, MeOH).

18c. 6-Methyl-3-(2-(S)-pyrrolidinylmethyloxy)pyridine dihydrochloride

The free base from Example 18b was dissolved in diethyl ether and brought to 0°C with stirring. The solution was treated with diethyl ether saturated with hydrogen chloride gas. The solvent was removed *in vacuo*. The resulting salt was triturated with diethyl ether (2X) and dried under vacuum to give a beige powder. m.p.> 100°C (dec). MS (DCl/$NH_3$) m/e: 193 $(M + H)^+$. $^1H$ NMR ($D_2O$, 300 MHz) δ: 8.27 (d, J=3 Hz, 1H), 7.81 (dd, J=9, 3 Hz, 1H), 7.59 (d, J=9 Hz, 1H), 4.51 (dd, 10.50, 3.50 Hz, 1H), 4.31 (dd, 10.50, 7.50 Hz, 1H), 4.19-4.12 (m, 1H), 3.43 (d, J=7 Hz, 2H), 2.60 (s, 3H), 2.33-2.24 (m, 1H), 2.20-1.92 (m, 3H). Anal. Calc. for $C_{11}H_{16}N_2O{\cdot}2.00{\cdot}HCl$: C, 49.82; H, 6.84; N, 10.56; Found C, 49.78; H, 6.53; N, 10.26. $[\alpha]^{25}_D$=+10.00° (c=1, MeOH).

Example 19

6-Methyl-3-(1-methyl-2-(S)-pyrrolidinylmethyloxy)pyridine dihydrochloride

19a. 6-Methyl-3-(1-methyl-2-(S)-pyrrolidinylmethyloxy)pyridine

To 3 mL of a solution of formic acid/ formaldehyde (1:2) was added the compound from Example 19a (.600g, 2.05 mmol). The mixture was brought to a gentle reflux and stirred at this temperature (80°C) for 3 hours. The reaction was basified with sat. $K_2CO_3$. The desired compound was extracted from the aqueous phase with $CH_2Cl_2$ (3X). The organic layer was dried over $MgSO_4$. The resulting crude material was purified by silica gel flash chromatography using a gradient from 100% chloroform to 10% methanol/chloroform (5% increments) to give 0.403g (95%) of the title compound as a clear colorless oil. TLC Rf=0.17 (10% MeOH/$CHCl_3$). MS (DCl/NH3) m/e 207 $(M + H)^+$. $^1H$ NMR ($CDCl_3$, 300 MHz) δ: 8.21 (d, J=3 Hz, 1H), 7.13 (dd, J=8.50, 3 Hz, 1H), 7.05 (d, J=8.50 Hz, 1H), 4.06 (dd, J=9, 5.50 Hz, 1H), 3.93 (dd, J=9, 5.50 Hz, 1H), 3.19-3.14 (m, 1H), 2.74-2.69 (m, 1H), 2.52 (s, 3H), 2.48 (s, 3H), 2.37-2.31 (m, 1H), 2.08-1.99 (m, 1H), 1.89-1.74 (m, 3H).

19b. 6-Methyl-3-(1-methyl-2-(S)-pyrrolidinylmethyloxy)pyridine dihydrochloride

The free base from Example 19a was dissolved in diethyl ether and brought to 0°C with stirring. This solution was treated with diethyl ether saturated with hydrogen chloride gas. The solvent was removed in vacuo. The resulting salt was triturated with diethyl ether (2X) and dried under vacuum to give a white powder. m.p.=213-216°C. MS (DCl/$NH_3$) m/e: 207 $(M+H)^+$. $^1H$ NMR ($D_2O$, 300 MHz) δ: 8.28 (d, J=3 Hz, 1H), 7.79 (dd, J=9, 3 Hz, 1H), 7.58 (d, J=9 Hz, 1H), 4.56 (dd, J=11, 3 Hz, 1 H), 4.39 (dd, J=11, 6 Hz, 1H), 3.95 (br s, 1H), 3.77 (br s, 1H), 3.28 (br s, 1H), 3.04 (s, 3H), 2.59 (s, 3H), 2.42-2.37 (m, 1H), 2.25-2.08 (m, 3H). Anal. Calc. for $C_{12}H_{18}N_2O{\cdot}2.00\ HCl$: C, 51.62; H, 7.22; N, 10.03; Found C, 51.49; H, 7.36; N, 9.96. $[a]^{25}_D$=-6.40° (c=1, MeOH).

Example 20

4-Bromo-3-(2-(S)-pyrrolidinylmethyloxy)pyridine dihydrochloride

20a. 3-Pyridyl diethylcarbamate

To a refluxing solution of 3-pyridinol (9.7 g, 0.10 mol) and triethylamine (17.0 mL, 0.12 mol) in anhydrous benzene (300 mL) was slowly added a solution of N,N-dimethylcarbamyl chloride (14.4 mL, 0.11 mol) in anhydrous benzene (50 mL). After heating at reflux for 12 hours, the resultant mixture was filtered, and the salt was washed with benzene (3X10 mL). The filtrate was concentrated and distilled under reduced pressure to provide 18.4 g (95% yield) of the title compound as a light yellow oil (lit.: bp 91-93°C/3.5 mmHg, Millner, O.E, Jr.; Stanley, J.W; Purcell, W. P. *J. Med. Chem.* **1974**, *17*, 13). TLC Rf 0.57 (10:1 $CHCl_3$/MeOH). MS (DCl/$NH_3$) m/e 212 $(M+NH_4)^+$, 195 $(M+H)^+$. $^1H$ NMR ($CDCl_3$, 300 MHz) δ: 8.44 (d, J = 0.9 Hz, 1 H, ArH), 8.43 (dd, J = 4.8, 1.8 Hz, 1 H, ArH), 7.53 (ddd, J = 7.5, 1.8, 0.9 Hz, 1H, ArH), 7.30 (dd, J = 7.5, 4.8 Hz, 1H, ArH), 3.43 (q, J = 7.2 Hz, 2H, $NCH_2$), 3.40 (q, J = 7.2 Hz, 2H, NCH2), 1.27 (t, J = 7.2 Hz, 3H, $CH_3$), 1.21 (t, J = 7.2 Hz, 3H, $CH_3$).

20b. 4-Bromo-3-pyridyl diethylcarbamate

To a cooled (-78°C) solution of TMEDA (787 mg, 6.6 mmol) in anhydrous THF (15 mL)-was slowly added *sec*-butyl lithium (1.30 M, 5.08 mL, 6.6 mmol), and the resultant solution was stirred at -78°C for 10 minutes. 3-Pyridyl diethylcarbamate (1.16 g, 6.0 mmol, from step 20a) in THF (3 mL) was slowly added, and the mixture was stirred at -78°C for 30 minutes. 1,2-Dibromoethane (0.575 mL, 6.6 mmol) was then added, and the mixture was stirred for an

additional 2 hours. Brine (1 mL) was added and the mixture was slowly warmed up to room temperature. The organic layer was decanted, and the residue was washed with ethyl acetate (3X5 mL). The combined organic layers were dried ($Na_2SO_4$) and concentrated under reduced pressure. The crude product was purified by flash chromatography on silica gel eluting with hexane/EtOAc (1:1 and 1:2) to provide 1.25 g (76% yield) of the title compound. TLC $R_f$ 0.48 (1:2 hexane/EtOAc). MS ($DCl/NH_3$) m/e 290 with $^{79}Br$ and 292 $(M+NH_4)^+$ with $^{81}Br$, 273 with $^{79}Br$ and 275 $(M+H)^+$ with $^{81}Br$. $^1H$ NMR ($CDCl_3$, 300 MHz) δ: 8.47 (s, 1H, ArH), 8.27 (d, J = 7.2 Hz, 1H, ArH), 7.16 (d, J = 7.2 Hz, 1H, ArH), 3.52 (q, J = 7.5 Hz, 2H, $NCH_2$), 3.41 (q, J = 7.5 Hz, 2H, NCH2), 1.33 (t, J = 7.5 Hz, 3H, $CH_3$), 1.24 (t, J = 7.5 Hz, 3H, $CH_3$).

20c. 4-Bromo-3-pyridinol

To a solution of 4-bromo-3-pyridyl diethylcarbamate (1.24 g, 4.50 mmol) in methanol (10 mL) was added sodium methoxide in methanol (2.04 g, 9.40 mmol), and the resultant mixture was refluxed for 1.5 hours. After removal of MeOH, EtOAc (15 mL) and water (1 mL) were added, the pH was then adjusted to 9 using 20% $H_2SO_4$. The organic layer was decanted, and the residue washed with EtOAc (3X5 mL). The combined organic layers were dried ($Na_2SO_4$) and concentrated. The crude product was purified by flash chromatography on silica gel eluting with hexane/EtOAc (1:1 and 1:2) to provide 691 mg (89% yield) of the title compound. TLC $R_f$ 0.38 (1:2 hexane/EtOAc). MS ($DCl/NH_3$) m/e 174 with $^{79}Br$ and 176 $(M+H)^+$ with $^{81}Br$. $^1H$ NMR ($CDCl_3$, 300 MHz) δ: 8.43 (d, J =1.5 Hz, 1H, ArH), 8.02 (d, J = 7.2 Hz, 1H, ArH), 7.54 (dd, J = 7.2, 1.5 Hz, 1 H, ArH).

20d. 4-Bromo-3-(2-(S)-pyrrolidinylmethyloxy)pyridine

(S)-1-t-Butoxycarbonyl-2-pyrrolidinemethanol (792 mg, 3.94 mmol), 4-bromo-3-pyridinol (685 mg, 3.94 mmol), DEAD (485 uL, 4.33 mmol) and PPh3 (1.14 g, 4.33 mmol) were allowed to react as described in Example 2a. The crude product was directly treated with trifluoroacetic acid (5 mL) at room temperature for 3 hours. The trifluoroacetic acid was removed under reduced pressure, and water (8 mL) was added. The mixture was extracted with EtOAc (2X20 mL), and the resultant aqueous layer was basified with excess solid sodium bicarbonate. The resultant slurry was washed extensively with EtOAc (4X10 mL). The combined organic layers were dried ($Na_2SO_4$) and evaporated. The crude product was purified by flash chromatography on silica gel eluting with $CHCl_3/MeOH/NH_4OH$ (10:1.5:0.02 and 10:1.5:0.1) to provide 371 mg (37% yield from 4-bromo-3-pyridinol) of the title compound. TLC $R_f$ 0.16 (10:1:0.02 $CHCl_3/MeOH/NH_4OH$). MS ($DCl/NH_3$) m/e 257 with $^{79}Br$ and 259 $(M+H)^+$ with $^{81}Br$. $^1H$ NMR ($CDCl_3$, 300 MHz) δ: 8.25 (s, 1H, ArH), 8.08 (d, J = 6.6 Hz, 1H, ArH), 7.48 (d, J = 6.6 Hz, 1H, ArH), 4.28 (dd, J = 4.8, 10.2 Hz, 1H, OCHH), 4.17 (dd, J = 6.3, 10.2 Hz, 1H, OCHH), 3.90-3.80 (m, 1H, NCH), 3.30-3.14 (m, 2H, NCH2), 2.18-1.80 (m, 4H, $2CH_2$).

20e. 4-Bromo-3-(2-(S)-pyrrolidinylmethyloxy)pyridine dihydrochloride

The product of 20d (140 mg) was treated with HCl and isolated as described in Example 1b to afford 120 mg (67% yield) of the title compound as a light yellow powder. mp 191-193°C. MS ($DCl/NH_3$) m/e 257 with $^{79}Br$ and 259 $(M+H)^+$ with $^{81}Br$. $^1H$ NMR ($D_2O$, 300 MHz) δ: 8.28 (s, 1H, ArH), 8.09 (d, J = 5.2 Hz, 1H, ArH), 7.77 (d, J = 5.2 Hz, 1H, ArH), 4.61 (dd, J = 3.3,10.7 Hz, 1H, OCHH), 4.36 (dd, J = 7.4, 10.7 Hz, 1H, OCHH), 4.24-4.16 (m, 1H, NCH), 3.52-3.37 (m, 2H, NCH2), 2.38-1.98 (m, 4H, 2CH2). Anal. Calc. for $C_{10}H_{15}N_2OCl_2Br$: C, 36.39; H, 4.58; N, 8.49. Found: C, 36.00; H, 4.24; N, 8.38.

Example 21

4-Bromo-3-(1-methyl-2-(S)-pyrrolidinylmethyloxy)pyridine dihydrochloride

21a. 4-Bromo-3-(1-methyl-2-(S)-pyrrolidinylmethoxy)pyridine (74)

The product of 20d (225 mg) was treated with formic acid and formaldehyde and isolated as described in Example 16a to afford 40 mg (18% yield) of the title compound. TLC $R_f$ 0.37 (10:1$CHCl_3$/MeOH). MS ($DCl/NH_3$) m/e 271 with $^{79}Br$ and 273 $(M+H)^+$ with $^{81}Br$. $^1H$ NMR ($CDCl_3$, 300 MHz) δ:8.24 (s, 1H, ArH), 8.07 (d, J = 6.3 Hz, 1H, ArH), 7.48 (d, J = 6.3 Hz, 1H, ArH), 4.34-4.03 (m, 3H), 3.35-2.80 (m, 2H), 2.14 (s, 3H, $CH_3$), 2.35-1.70 (m, 4H, $2CH_2$).

21b. 4-Bromo-3-(1-methyl-2-(S)-pyrrolidinylmethyloxy)pyridine dihydrochloride (81)

The product of 21 a (35 mg) was treated with HCl and isolated as described in Example 1b to afford 43 mg (96% yield) of the title compound as a light yellow powder. mp 191-193°C. MS ($DCl/NH_3$) m/e 271 with $^{79}Br$ and 273 $(M+H)^+$ with $^{81}Br$. $^1H$ NMR ($D_3O$, 300 MHz) δ: 8.32 (s, 1H, ArH), 8.14 (d, J = 5.3 Hz, 1H, ArH), 7.87 (d, J = 5.3 Hz, 1

H, ArH), 4.69 (dd, J = 3.3, 11.4 Hz, 1H, OCHH), 4.42 (dd, J = 7.0, 11.4 Hz, 1H, OCHH), 4.08-3.98 (m, 1H, NCH), 3.83-3.76 (m, 1H, NCH), 3.32-3.22 (m, 1H, NCH), 3.14 (s, 3H, $NCH_3$), 2.44-2.02 (m, 4H, $2CH_2$). Anal. Calc. for $C_{11}H_{17}N_2OCl_2Br \cdot 0.10\ Et_2O$: C, 38.96; H, 5.16; N, 7.93. Found: C, 39.22; H, 4.94; N, 8.28.

Example 22

3-(1-Methyl-5-methyl-2-(S)-pyrrolidinylmethyloxy)pyridine dihydrochloride

22a. 3-(2-oxo-5-(S)-pyrrolidinylmethyloxy)pyridine

To a solution of triphenylphosphine (52.46g, 0.20 mol) in 400 mL of anhydrous THF at 0°C was added diethyl azodicarboxylate (31.49 ml, 0.20 mol) dropwise. The mixture was stirred at 0°C for 30 minutes, then brought to room temperature. (S)-5-(Hydroxymethyl)-2-pyrrolidinone (Aldrich Chemical Co., 15.19g, .13 mol) and 3-hydroxypyridine (19.02g, 0.20 mol) were added to the reaction vessel, and stirre for 16 hours. Solvent was removed *in vacuo*. The residue was diluted with $CH_2Cl_2$ and washed with 1N NaOH. After a brine wash (2X), the organic layer was dried over $MgSO_4$. The residue was purified by silica gel flash chromatography using a gradient from 100% $CHCl_3$ to 10% MeOH/$CHCl_3$ (purification system was run twice), then recrystallized with ethyl acetate to give 4.0 g (16%) of the title compound as a white powder, m.p. = 121-122°C. TLC $R_f$=0.31 (10%MeOH/$CHCl_3$). MS (DCI/$NH_3$) m/e: 193 $(M + H)^+$ and 210 $(M + NH_4^+)^+$. $^1H$ NMR ($CDCl_3$, 300 MHz) 8.32-8.30 (m, 1H), 8.27 (dd, J=4.40, 1.50 Hz, 1H), 7.25-7.16 (m, 2H), 6.17 (br s, 1H), 4.14-4.08 (m, 1H), 4.05 (dd, J=8.80, 3.70 Hz, 1H), 3.89 (dd, J=8.80,7.70 Hz, 1H), 2.49-2.23 (m, 3H), 1.99-1.87 (m, 1H). Anal. Calc. for $C_{10}H_{12}N_2O_2$: C, 62.49; H, 6.29; N, 14.57; Found C, 62.53; H, 6.25; N, 14.71.

22b. 3-(1-Methyl-2-oxo-5-(S)-pyrrolidinylmethyloxy)pyridine

To a solution of the compound from Example 22a (0.100 g, 0.52 mmol) in anhydrous THF at 0°C was added NaH (80% dispersion, 0.02g, 0.83 mmol) was added, and the reaction mixture was stirred for 20 minutes at this temperature. The reaction was then warmed to room temperature, and iodomethane (0.06 ml, 0.89 mmol) was added via syringe. After starting material was consumed, $NaHCO_3$ was added to the reaction followed by $CH_2Cl_2$. The desired compound was extracted from the aqueous phase, and the organic layer was subjected to a brine wash (2X). The organic layer was dried over $MgSO_4$. The residue was purified by silica gel flash chromatography (5%MeOH/$CHC_3$) to give 0.107g (100%) of the title compound as a white powder. m.p. = 73-74°C. MS (DCI/$NH_3$) m/e: 207 $(M + H)^+$ and 224 $(M + NH_4^+)^+$, $^1H$ NMR ($CDCl_3$, 300 MHz) δ: 8.33-8.28 (m, 2H), 7.24-7.18 (m, 2H), 4.14 (dd, J=9.50, 4 Hz, 1H), 4.04 (dd, J=9.50, 4.40 Hz, 1H), 3.95-3.89 (m, 1H), 2.92 (s, 3H), 2.55-2.50 (m, 1H), 2.45-2.21 (m, 2H), 2.02-1.94 (m, 1H). Anal. Calc. for $C_{11}H_{14}N_2O_2$: C, 64.06; H, 8.84; N, 13.58; Found C, 64.07; H, 6.67; N, 13.67. $[\alpha]^{25}_D$=+37.30° (c=1.03, MeOH).

22c. 3-(1-Methyl-5-methyl-2-(S)-pyrrolidinylmethyloxy)pyridine

To a solution of the product of step 22b (0.400g, 1.94 mmol) in anhydrous THF at 0°C was added MeMgCl (Aldrich Chemical Co., 3M solution in THF, 1.94 ml, 5.80 mmol). An immediate precipitate formed in the reaction, but the reaction was stirred at this temperature for 2 hours. The reaction was then brought to room temperature and sonicated for 30 minutes followed by stirring for an hour. After the starting material was consumed the reaction was quenched with MeOH. Bromocresol green indicator was added followed by enough 2N HCl/MeOH to turn the color of the reaction mixture yellow (acidic pH). Sodium cyanoborohydride (.182g, 2.92 mmol) was added to the reaction and the mixture was stirred for an additional 3 hours (adding 2N HCl/MeOH to maintain the pH). Saturated $K_2CO_3$ was added to the reaction mixture slowly. After the aqueous phase tested basic, $CH_2Cl_2$ was added to extract the desired material. The organic layer was then washed with a brine solution (2X) and dried over $MgSO_4$. The resulting material was purified by silica gel flash chromatography (10%MeOH/$CHCl_3$) to give 0.170g (42.5%) of the *cis*-5'-methyl compound and 0.057g (14%) of the *trans* -5'-compound ( the data for the *cis* compound given). MS (DCI/$NH_3$) m/e: 207 $(M + H)^+$. $^1H$ NMR ($CDCl_3$, 300 MHz) δ: 8.32 (m, 1H), 8.20 (t, J=3 Hz, 1 H), 7.20-7.18 (m, 2H), 4.05 (dd, J=9.20, 5.50 Hz, 1H), 3.89 (dd, J=9.20, 6.25 Hz, 1H), 2.80-2.75 (m, 1H), 2.41 (s, 3H), 2.40-2.37 (m, 1H), 2.01-1.87 (m, 2H), 1.65-1.61 (m, 1H), 1.47-1.44 (m, 1H), 1.13 (d, J=6.25 Hz, 3H).

22d. 3-(1-Methyl-5-methyl-2-(S)-pyrrolidinylmethyloxy)pyridine dihydrochloride

The free base from step 22c was dissolved in diethyl ether and brought to 0°C with stirring. The solution was treated with diethyl ether saturated with hydrogen chloride gas. The solvent was removed *in vacuo*. The resulting salt was triturated with diethyl ether (2X) and dried under vacuum to give a white powder. MS (DCI/$NH_3$) m/e: 207 (M +

H)$^+$. $^1$H NMR ($D_2O$, 300 MHz) δ: 8.41 (br s, 1H), 8.31 (d, J=4.40 Hz, 1H), 7.81-7.77 (m, 1H), 7.66 (dd, J=8.80, 5.10 Hz, 1H), 4.57 (dd, J=11.40, 3 Hz, 1H), 4.44 (dd, J=8.80, 5.90 Hz, 1H), 4.03-3.96 (m, 1H), 3.61-3.53 (m, 1H), 3.03 (s, 3H), 2.43-2.30 (m, 2H), 2.14-2.03 (m, 1H), 1.92-1.81 (m, 1H),1.47 (d, J=6.6 Hz, 3H). Anal. Calc. for $C_{12}H_{18}N_2O$*2.20 HCl: C, 50.31; H, 7.11; N, 9.78; Found C, 50.07; H, 7.10; N, 9.77. $[\alpha]^{25}_D$=+8.60° (c=1, MeOH).

Example 23

3-(*trans*-1-Methyl-4-hydroxy-2(S)-pyrrolidinylmethyloxy)pyridine dihydrochloride

23a.3-(*trans*-1-Methyl-4-hydroxy-5-oxo-2(S)-pyrrolidinylmethyloxy)pyridine

A sample of lactam (1.0 g, 5.6 mmol) from Example 22b was dissolved in 25 mL of THF and cooled to -78°C. Lithium diisopropyl amide (LDA) solution (1.5 M in hexane, 7.5 mL, 11.2 mmol) was added, and the solution was stirred at-78°C for 30 min. Next a solution of 1.17 g (5.12 mmol) of (+)-(camphorsulfonyl)oxaziridine in 24 mL of THF was added. After stirring at -78°C for 1 h, the reaction mixture was gradually warrmed to room temperature and allowed to stir for an additional 2 hours. The reaction was then quenched by addition of methanol. The resultant mixture was stirred for 15 min, and the solvent was removed. The residue was subjected to flash chromatography on silica gel using chloroform:methanol (10:1) as eluent. The title compound was isolated as an oil (0.76 g, 69% yield). MS m/e : 223 (M+H)$^+$, 240 (M+$NH_4$)$^+$,. $^1$H NMR ($CDCl_3$, 300 MHz) δ: 8.37-8.26 (m, 2H), 7.31-7.16 (m, 2H), 4.59 (t, J=7.5 Hz, 1H,), 4.18 (dd, J= 4.5, 9 Hz, 1H), 4.04 (dd, J=4.5, 9 Hz, 1H), 3.96-3.88 (m, 1H), 2.96 (s, 3H), 2.52-2.42 (m, 1H), 2.21-1.98 (m, 1H).

23b.3-(*trans*-1-Methyl-4-hydroxy-2(S)-pyrrolidinylmethyloxy)pyridine

To the product of Example 23a (275 mg, 1.24 mmol) in 5 mL of THF was added, under nitrogen and dropwise over a period of 5 minutes, 2.5 mL (2.48 mmol) of a 1 M solution of borane in THF. After stirring under reflux for 3 hours, methanol was added dropwise, and the reaction was stirred for an additional 15 minutes. The solvent was then removed *in vacuo,* affording a white solid borane complex. This solid was dissolved in anhydrous ethanol. Cesium fluoride (0.286 g, 2.48 mmol) was added, and the resultant solution was stirred under reflux for 16 hr. Evaporation of the solvent provided a white solid which was purified on a silica gel column, eluting with chloroform:methanol (10:1) to give 220 mg of the desired alcohol as an oil in 85% yield. TLC $R_f$ = 0.38 (10:1$CHCl_3$ : MeOH). MS m/e: 209 (M+H)$^+$. $^1$H NMR ($CDCl_3$, 300 MHz) δ: 8.35-8.33 (m, 1H), 8.25-8.21 (m, 1H), 7.24-7.20 (m, 2H), 4.54-4.44 (m, 1H), 4.08-3.97 (m, 2H), 3.52-3.44 (m, 1H), 3.08-2.99 (m, 1H), 2.51 (s, 3H), 2.43-2.33 (m, 1H), 2.14-1.97 (m, 2H).

23c.3-(*trans*-1-Methyl-4-hydroxy-2(S)-pyrrolidinylmethyloxy)pyridine dihydrochloride

A solution of HCl in ether was added dropwise to a stirred solution of compound 23b (220 mg, 1.05 mmol) in diethyl ether at room temperature. The resultant white precipitate was then collected by centrifugation and triturated with three portions of diethyl ether. The hygroscopic solid was obtained in 59% yield (174 mg). mp 145-147°C. MS m/e (DCI/$NH_3$): 209 (M+H$^+$), 226 (M+$NH_4^+$). $^1$H NMR (D20, 300 MHz) δ: 8.42 (d, J=2.6 Hz, 1 H), 8.32 (d, J=4.4 Hz, 1 H), 7.77-7.85(m, 1H), 7.66 (dd, J=5.1, 8.4 Hz, 1H), 4.64 (dd, J=4.6, 11.0 Hz, 1H), 4.44 (dd, J= 5.9, 11.4 Hz, 1H), 4.34-4.21 (m, 1H), 4.04-3.89 (m, 1H), 3.20-3.11 (m, 1H), 3.34-3.03 (m, 1H), 3.15 (s, 3H), 2.38-2.34 (m, 2H), Anal. Calc., for $C_{11}H_{16}N_2O_2$·1.9 HCl·0.1$H_2O$: C, 47.30; H, 6.53; N, 10.01. Found: C, 47.63; H, 6.42; N, 9.68. $[\alpha]_D$ = + 2.2° (c 0.41, MeOH).

Example 24

3-(*trans*-1,4-Dimethyl-2(S)-pyrrolidinylmethyloxylpyridine dihydrochloride

24a.3-(*trans*-1,4-Dimethyl-5-oxo-2(S)-pyrrolidinylmethyloxy) pyridine

A sample of lactam (0.17 g, 0.83 mmol) from Example 22b was dissolved in 5 mL of THF and cooled to -78°C. Lithium diisopropyl amide (LDA) solution (1.5 M in hexane, 1.11 mL, 1.66 mmol) was added, and the solution was stirred at 0°C for 30 min. After cooling to -78°C, MeI (0.1 mL, 1.66 mmol) was added, and the resultant solution was allowed to stir at -78°C for 3 hours. The reaction was then quenched by addition of saturated ammonium chloride aqueous solution. The resultant mixture was stirred for 15 min, and the aqueous layer was extracted with ethyl acetate. The combined organic layers were then dried over anhydrous magnesiun sulfate. Filtration and concentration under vacuum provided a yellow oil, which was subjected to flash chromatography on silica gel using chloroform:methanol

(10:1) as eluent. The title compound was isolated as an oil (0.13 g, 75% yield). TLC $R_f$ = 0.10 (100:1$CHCl_3$ : MeOH). MS m/e : 221 $(M+H)^+$, 238 $(M+NH_4)^{+}$,. $^1H$ NMR ($CDCl_3$, 300 MHz) δ: 8.37-8.22 (m, 2H), 7.35-7.23(m, 2H), 4.09 (ddd, J=4.5, 9.0, 15 Hz, 2H,), 3.89-3.81(m, 1H), 2.93 (s, 3H), 2.73-2.60 (m, 1H), 2.29-2.20 (m, 1H),.1.23 (d, J=8 Hz, 3H).

24b.3-(*trans*-1.4-Dimethyl-2(S)-pyrrolidinylmethyloxy)pyridine

To the product of Example 24a (130 mg, 0.63 mmol) in 4 mL of THF was added, under nitrogen and dropwise over a period of 5 minutes, 1.25 mL (1.25 mmol) of a 1 M solution of borane in THF. After stirring under reflux for 2 hours, methanol was added dropwise and the reaction was stirred for an additional 15 minutes. The solvent was then removed *in vacuo*, affording a white solid borane complex. This solid was dissolved in anhydrous ethanol. Cesium fluoride (0.218 g, 1.89 mmol) was added, and the resultant solution was stirred under reflux for 16 hr. Evaporation of the solvent provided a white solid which was purified on a silica gel column, eluting with chloroform:methanol (10:1) to give 59 mg of the desired methyl compound as an oil in 46% yield. TLC $R_f$ = 0.16 (10:1$CHCl_3$ : MeOH). MS m/e: 207 $(M+H)^+$. $^1H$ NMR ($CDCl_3$, 300 MHz) δ: 8.34 (brs, 1H), 8.26-8.18 (m, 1H), 7.25-7.18 (m, 2H), 4.20-4.02 (m, H), 4.02-3.90 (m, 1H), 3.33-3.21 (m, H), 3.00-2.80 (m, 1H), 2.54 (s, 3H), 2.45-2.30 (m, 1H), 2.12-1.91 (m, 2H), 1.74-1.54 (m, 1H), 1.06 (d, J=8.0 Hz).

24c.3-(*trans*-1,4-Dimethyl-2(S)-pyrrolidinylmethyloxy)pyridine dihydrochloride

A solution of HCl in ether was added dropwise to a stirred solution of compound 24b (55 mg, 0.27 mmol) in diethyl ether at room temperature. The resultant white precipitate was then collected by centrifugation and triturated with three portions of diethyl ether. The hygroscopic solid was obtained in 56% yield (42 mg). mp 223-225°C, MS m/e (DCI/NH3): 207 $(M+H^+)$. $^1H$ NMR (DMSO-$d_6$, 300 MHz) δ: 8.65 (d, J=2.9 Hz, 1H), 8.46 (d, J=5.9 Hz, 1H), 8.00 (dd, J=2.3, 8.7 Hz, 1H), 7.81 (dd, J=5.2, 8.7 Hz, 1H), 4.60 (dd, J=8.1, 11.0 Hz, 1H), 4.49 (dd, J= 3.5, 10.4 Hz, 1H), 4.04-3.95 (m, 1H), 3.64-3.58 (m, 1H), 2.92 (s, 3H), 2.80-2.74 (m, 1H), 2.45-2.40 (m, 1H); 2.04-1.98 (m, 1H), 1.95-1.89 (m, 1H), 1.07 (d, J=6.9 Hz, 3H). Anal. Calc. for $C_{12}H_{18}N_2O$•2.0 HCl: C, 51.62; H, 7.22; N, 10.03. Found: C, 51.84; H, 7.36; N, 9.90. $[\alpha]_D$ = -2.3° (c 0.32, MeOH).

Example 25

3-(*trans*-1-Methyl-4-ethyl-2(S)-pyrrolidinylmethyloxy)pyridine dihydrochloride

25a.3-(*trans*-1-Methyl-4-ethyl-5-oxo-2(S)-pyrrolidinylmethyloxy)pyridine

A sample of lactam (0.195 g, 0.94 mmol) from Example 22b was dissolved in 3 mL of THF and cooled to -78°C. Lithium diisopropyl amide (LDA) solution (1.5 M in hexane, 0.94 mL, 1.42 mmol) was added and the solution was stirred at -78°C for 30 min. EtI (0.113 mL, 1.42 mmol) was then added and the resultant solution was allowed to stir at -78 °C for 3 hours and gradually warmed to room temperature. The reaction was quenched by addition of saturated ammonium chloride aqueous solution. The resultant mixture was stirred for 15 min and the aqueous layer was extracted with ethyl acetate. The combined organic layers were then dried over anhydrous magnesiun sulfate. Filtration and concentration under reduced pressure provided a yellow oil which was subjected to flash chromatography on silica gel using chloroform:methanol (10:1) as eluent. The title compound was isolated as an oil (0.216 g, 98% yield). TLC $R_f$ = 0.42 (10:1$CHCl_3$ : MeOH). MS m/e : 235 $(M+H)^+$. $^1H$ NMR ($CDCl_3$, 300 MHz) δ: 8.35-8.29 (m, 1H), 8.30-8.25 (m, 1H), 7.25-7.16 (m, 2H), 4.18-4.00 (m 2H), 3.89-3.75 (m, 1H), 2.94 (s, 3H), 2.62-2.48 (m, 1H), 2.23-2.12 (m, 1H),.2.00-1.80 (m, 2H), 1.70-1.30 (m, 2H), 0.98 (t, J=7.5 Hz, 3H).

25b.3-(*trans*-1-Methyl-4-ethyl-2(S)-pyrrolidinylmethyloxy)pyridine

To the product of Example 25a (230 mg, 0.63 mmol) in 2 mL of THF was added under nitrogen and dropwise over a period of 5 minutes 2.95 mL (2.95 mmol) of a 1 M solution of borane in THF. After stirring under reflux for 3 hours, methanol was added dropwise and the reaction stirred for an additional 15 minutes. The solvent was then removed *in vacuo*, affording a white solid borane complex. This solid was dissolved in anhydrous ethanol. Cesium fluoride (0.335 g, 2.95 mmol) was added, and the resultant solution was stirred under reflux overnight. Evaporation of the solvent provided a white solid which was purified on a silica gel column, eluting with chloroform:methanol (10:1) to give 104 mg of the desired methyl compound as an oil in 48% yield. TLC $R_f$ = 0.14 (10:1$CHCl_3$: MeOH). MS m/e: 221 $(M+H)^+$. $^1H$ NMR ($CDCl_3$, 300 MHz) δ: 8.35-8.31 (m, 1H), 8.24-8.19 (m, 1H), 7.24-7.18 (m, 2H), 4.08-3.86 (m, 2H), 3.25-3.15 (m, 1H), 2.80-2.66 (m, 1H), 2.47 (s, 3H), 2.20-2.04 (m, 1H), 1.99 (dd, J=9.0, 9.6 Hz, 1H), 1.95-1.85 (m, 1H), 1.72-1.57 (m, 2H), 1.48-1.31 (m, 2H), 0.92 (t, J=8.0 Hz).

25c. 3-(*trans*-1-Methyl-4-ethyl-2(S)-pyrrolidinylmethyloxy)pyridine dihydrochloride

A solution of hydrochloride in ether was added dropwise to a stirred solution of compound 25b (55 mg, 0.27 mmol) in diethyl ether at room temperature. The resultant white precipitate was then collected by centrifugation and triturated with three portions of diethyl ether. The hygroscopic solid was obtained in 56% yield (42 mg). mp 219-220°C. MS m/e (DCI/NH3): 221 ($M+H^+$). $^1$H NMR ($D_2O$, 300 MHz) δ: 8.45 (d, J=2.6 Hz, 1H), 8.36 (d, J=5.1 Hz, 1H), 7.91 (m, 1H), 7.76 (dd, J=5.2, 8.8 Hz, 1H), 4.59 (dd, J=2.9, 11.0 Hz, 1H), 4.43 (dd, J= 5.9, 11.0 Hz, 1H), 4.04-3.95 (m, 1H), 4.08-4.00 (m, 1H), 3.83 (dd, J=6.6, 11.0 Hz), 3.05 (s, 3H), 2.95 (t, J=11.0 Hz, 1H), 2.45-2.00 (m, 2H), 2.14-2.03 (m, 1H), 1.60-1.45 (m, 2H), 0.94 (t, J=7.4 Hz, 3H). Anal, Calcd. for $C_{13}H_{20}N_2O \cdot 1.9$ HCl: C, 53.92; H, 7.62; N, 9.67. Found: C, 54.00; H, 7.59; N, 9.35. $[\alpha]_D$ = -1.9° (c 0.37, MeOH).

Example 26

3-(1-Methyl-2-pipecolinylmethyloxy)pyridine oxalate salt

1-Methyl-2-piperidinemethanol (0.857 g, 6.65 mmol) was allowed to react with 3-bromopyridine (0.67 mL, 6.98), cuprous bromide (0.257 g, 1.33 mmol), triphenylphosphine (0.698 g, 2.66 mmol) and potassium carbonate (0.919 g, 6.65 mmol). The reaction mixture was heated to 90 °C and stirred for 120 hr, then cooled to 25 °C, acidified with HCl (1.5 M; 35 mL) and washed with ethyl acetate (4 x 50 mL). The aqueous layer was basified with saturated aqueous potassium carbonate, and the product was extracted with chloroform (6 x 100 mL), dried ($MgSO_4$) and concentrated *in vacuo* to an oil. The crude product was purified to yield the free base of the title compound after chromatography on silica gel ($CHCl_3$/MeOH/$NH_4OH$ 1500:30:3). The amine was dissolved in EtOH (1 mL) and treated with oxalic acid (*ca.* 65 mg) to yield after recrystallization (EtOH/$Et_2O$) the title compound (0.088 g, 4%) as a hygroscopic white solid. MS (DCI/$NH_3$) m/e: 207 $(M+H)^+$. $^1$H NMR ($D_2O$, 300 MHz) δ: 8.46 (d, J=2.9 Hz, 1H), 8.37 (dd, J=5.2, 1.1 Hz, 1H), 7.94 (ddd, J=8.8, 2.9, 1.1 Hz, 1H), 7.80 (dd, J=8.8, 5.9 Hz, 1H), 4.69 (dd, J=11.2, 3.1 Hz, 1H), 4.35 (dd, J=11.2, 2.0 Hz, 1H), 3.56 (m, 2H), 3.18 (dt, J=12.7, 3.0 Hz, 1H), 2.93 (S, 3H), 2.05-1.65 (m, 6H). Anal. calcd for $C_{14}H_{20}N_2O_5 \cdot 0.4\ C_2H_2O_4$: C, 53.49; H, 6.31; N, 8.43. Found: C, 53.39; H, 6.09; N, 8.19.

Example 27

4-Methyl-3-(2-(S)-pyrrolidinylmethyloxy)pyridine dihydrochloride

27a. 4-Methyl-3-pyridyl diethylcarbamate

To the cooled (-78°C) solution of TMEDA (516.5 mg, 4.40 mmol) in anhydrous THF (10 mL) was slowly added *sec*-butyl lithium (1.3 M, 3.38 mL, 4.40 mmol), and the resultant solution was stirred at -78°C for 10 minutes. 3-Pyridyl diethylcarbamate (776 mg, 4.0 mmol) in THF (3 mL) was slowly added, and the mixture was stirred at -78°C for 30 minutes, lodomethane (275.4 uL, 4.40 mmol) was then added, and the mixture was stirred for two hours. Brine (1 mL) was added, and the mixture was slowly warmed up to room temperature. The organic layer was decanted, and the residue washed with ethyl acetate (3X5 mL). The combined organic layers were dried ($Na_2SO_4$) and concentrated. The crude product was purified by chromatography on silica gel eluting with hexane/EtOAc (1:2 and 0:1) to provide 765 mg (92% yield) of the title compound. TLC $R_f$ 0.28 (1:2 hexane/EtOAc). MS (DCI/$NH_3$) m/e 209 $(M+H)^+$. $^1$H NMR ($CDCl_3$, 300 MHz) δ: 8.34 (s, 1 H, ArH), 8.32 (d, J = 6.9 Hz, 1 H, ArH), 7.16 (d, J = 6.9 Hz, 1H, ArH), 3.48 (q, J = 7.5 Hz, 2H, $NCH_2$), 3.41 (q, J = 7.5 Hz, 2H, $NCH_2$), 1.28 (t, J = 7.5 Hz, 3H, $CH_3$), 1.22 (t, J = 7.5 Hz, 3H, $CH_3$).

27b. 4-Methyl-3-pyridinol

To the solution of 4-methyl-3-pyridyl diethylcarbamate (760 mg, 3.65 mmol) in methanol (10 mL) was added sodium methoxide (623 mg, 11.0 mmol), and the resultant mixture was refluxed for 20 hours. Methanol was evaporated, EtOAc (15 mL) and water (1 mL) were added, pH was adjusted to 9 using 20% yield $H_2SO_4$. Organic layer was decanted and the residue washed with EtOAc (3X5 mL). The combined organic layers were dried ($Na_2SO_4$) and concentrated. The crude product was purified by flash chromatography on silica gel eluting with $CHCl_3$/MeOH (20:1 and 10:1) to provide 325 mg (82% yield) of the title compound. TLC Rf 0.30 (10:1 $CHCl_3$/MeOH). MS (DCI/$NH_3$) m/e 110 $(M+H)^+$. $^1$H NMR ($CDCl_3$, 300 MHz) δ: 8.24 (s, 1H, ArH), 7.98 (d, J = 7.2 Hz, 1H, ArH), 7.16 (d, J = 7.2 Hz, 1H, ArH).

27c. 4-Methyl-3-(2-(S)-pyrrolidinylmethyloxy)pyridine

(S)-1-t-Butoxycarbonyl-2-pyrrolidinemethanol (590 mg, 2.94 mmol), 4-methyl-3-pyridinol (320 mg, 2.94

mmol), DEAD (509 uL, 3.23 mmol) and PPh3 (848 mg, 3.23 mmol) in THF (100 mL) were allowed to react as described in Example 2a. Solvent was removed, and the residue was chromatographied with $CHCl_3$/MeOH (10:1) to provide 1.8 g of the crude mixture. This material was immediately treated with trifluoroacetic acid (2.0 mL) at room temperature for 3 hours, and excess trifluoroacetic acid was removed under reduced pressure. Water (3 mL) and EtOAc (20 mL) were added, and the mixture was stirred for 5 minutes. The organic layer was decanted, and the residue washed with EtOAc (3X10 mL). The combined organic layers were dried ($Na_2SO_4$) and evaporated. The crude product was purified by flash chromatography on silica gel eluting with $CHCl_3$/MeOH/$NH_4OH$ (10:1:0.02 and 10:1.5:0.1) to provide 52 mg (14% yield from 4-methyl-3-pyridinol) of the title compound. TLC Rf 0.18 (10:1:0.02 $CHCl_3$/MeOH/$NH_4OH$). MS (DCl/$NH_3$) m/e 193 $(M+H)^+$. $^1H$ NMR ($CDCl_3$, 300 MHz) δ: 8.16 (s, 1H, ArH), 8.12 (d, J = 6.9 Hz, 1H, ArH), 7.07 (d, J = 6.9 Hz, 1H, ArH), 4.12-4.00 (m, 2H, $OCH_2$), 3.75-3.63 (m, 1H, NCH), 3.18-3.02 (m, 2H, $NCH_2$), 2.24 (s, 3H, $NCH_3$), 2.08-1.64 (m, 4H, $2CH_2$).

27d. 4-Methyl-3-(2-(S)-pyrrolidinylmethyloxy)pyridine dihydrochloride

The product of 27c was treated with HCl and isolated as described in Example 1b to afford a cream colored powder. MS (DCl/$NH_3$) m/e 193 $(M+H)^+$. $^1H$ NMR ($D_2O$, 300 MHz) δ: 8.34 (s, 1H, ArH), 8.33 (d, J = 5.5 Hz, H, ArH), 7.82 (d, J = 5.5 Hz, 1H, ArH), 4.60 (dd, J = 3.3, 10.3 Hz, H, OCHH), 4.37 (dd, J = 7.4, 10.3 Hz, 1H, OCHH), 4.25-4.17 (m, 1H, NCH), 3.44 (t, J =7.4 Hz, 2H, $NCH_3$), 2.50 (s, 3H, $NCH_3$), 2.40-1.98 (m, 4H, $2CH_2$). Anal. Calc, for $C_{11}H_{18}N_2OCl_2$·0.30 HCl: C, 47.85; H, 6.76; N, 10.21. Found: C, 48.01; H, 6.44; N, 10.19.

Example 28

5-Bromo-3-(1-methyl-2-(S)-pyrrolidinylmethyloxy)pyridine dihydrochloride

28a. 5-Bromo-3-(1-methyl-2-(S)-pyrrolidinylmethyloxy)pyridine

(S)-1-Methyl-2-pyrrolidinemethanol (4.96 g, 40.0 mmol) was carefully added to the suspension of sodium hydride (1.32 g, 80% yield, 44.0 mmol) in anhydrous DMF (100 mL). After stirring at room temperature for 0.5 hour, 3,5-dibromopyridine (4.83 g, 20.0 mmol) was added, and the reacting mixture was allowed to stir at 50°C for 4 hours. Another 5.0 mL of water was added, and the solvents were removed under reduced pressure. Again, water (5.0 mL) was added, and the slurry was washed extensively with EtOAc (4X40 mL). The combined organic layers were dried ($Na_2SO_4$) and concentrated. The crude product was purified by flash chromatography on silica gel eluting with $CHCl_3$/MeOH (10:1) to provide 4.50 g (83% yield) of the title compound. TLC Rf 0.33 (10:1 $CHCl_3$/MeOH). MS (DCl/$NH_3$) m/e 271 with $^{79}Br$ and 273 $(M+H)^+$ with $^{81}Br$. $^1H$ NMR ($CDCl_3$, 300 MHz) δ: 8.37 (d, J =1.8 Hz, 1H, ArH), 8.26 (d, J = 2.7 Hz, 1H, ArH), 7.39 (dd, J = 1.8, 2.7 Hz, 1H, ArH), 4.01 (dd, J = 3.3,11.1 Hz, 1H, OCHH), 3.93 (dd, J = 6.9, 11.1 Hz, 1H, OCHH), 3.20-3.10 (m, 1H, NCH), 2.76-2.64 (m, 1H, NCH), 2.49 (s, 3H, NCH3), 2.40-2.28 (m, 1H, NH), 2.44-2.00 (m, 4H, $2CH_2$).

28b. 5-Bromo-3-(1-methyl-2-(S)-pyrrolidinylmethyloxy)pyridine dihydrochlorlde

The product of 28a (190 mg) was treated with HCl and isolated as described in Example 1b to afford 170 mg (71% yield) of the title compound as a light yellow powder. mp 223-225°C. MS (DCl/$NH_3$) m/e 271 with $^{79}Br$ and 273 $(M+H)^+$ with 81 Br. $^1H$ NMR ($CDCl_3$, 300 MHz) δ: 8.42 (d, J =1.5 Hz, 1H, ArH), 8.35 (d, J = 2.7 Hz, 1 H, ArH), 7.93 (dd, J = 1.5, 2.7 Hz, 1H, ArH), 4.55 (dd, J = 3.0, 11.4 Hz, 1H, OCHH), 4.38 (dd, J = 6.3, 11.4 Hz, 1H, OCHH), 3.98-3.86 (m, 1H, NCH), 3.80-3.72 (m, 1H, NCH), 3.30-3.20 (m, 1H, NCH), 3.03 (s, 3H, $NCH_3$), 2.44-2.00 (m, 4H, $2CH_2$). Anal. Calc. for $C_{12}H_{16}N_2OClF_3$·0.10 $Et_2O$: C, 38.96; H, 5.16; N, 7.93. Found: C, 39.05; H, 4.80; N, 8.27.

Example 29

2-Methyl-3-(2-(S)-azetidinylmethyloxy)pyridine dihydrochloride

29a. 2-Methyl-3-(N-*t*-Butoxycarbonyl-2-(S)-azetidinylmethyloxy) pyridine

An ice-cooled solution of the product from Example 7b (0.623 g, 3.33 mmol) was allowed to react with 2-methyl-3-hydroxypyridine (0.399 g, 3.66 mmol) under the conditions of Example 2a to yield the title compound (0.511 g, 55%). MS (DCl/$NH_3$) m/e: 279 $(M+H)^+$. $^1H$ NMR ($CDCl_3$, 300 MHz) δ: 8.10 (dd, J=4.4, 1.5 Hz, 1H), 7.15-7.06 (m, 2H), 4.54-4.53 (m, 1H), 4.35-4.34 (m, 1H), 4.07 (dd, J=10.3, 2.6 Hz, 1H), 3.96-3.88 (m, 2H), 2.51 (s, 3H), 2.42-2.31 (m, 2H), 1.40 (s, 9H).

29b. 2-Methyl-3-(2-(S)-azetidinylmethyloxy) pyridine dihydrochloride

The product from Example 29a (0.181 g, 0.65 mmol) was treated with saturated ethanolic HCl (5 mL). After 4 hr, the volatiles were removed *in vacuo*, and the dihydrochloride was recrystallized ($EtOH/Et_2O$) to yield the title compound (0.157 g, 96% yield) as a white solid. mp 153-154°C. MS ($DCl/NH_3$) m/e: 179 $(M+H)^+$. $^1H$ NMR ($D_2O$, 300 MHz) δ: 8.21-8.19 (d, J=5.5 Hz, 1H), 7.94 (d, J=8.4 Hz, 1H), 7.74 (dd, J=5.5, 3.1 Hz, 1H), 5.04-4.93 (m, 1H), 4.61-4.50 (m, 2H), 4.21-4.08 (m, 2H), 2.78-2.65 (m, 2H), 2.69 (s, 3H). Anal. calc. for $C_{10}H_{16}Cl_2N_2O \cdot 0.8H_2O$: C, 45.23; H, 6.68; N, 10.55. Found: C, 45.15; H, 6.85; N, 10.51. $[\alpha]^D{}_{23}$ +7.27 ° (c =0.11 in MeOH).

Example 30

3-(1-methyl-2-(S)-pyrrolidinylmethyloxy)quinoline hydrochloride

To (S)-1-methyl-2-pyrrolinylmethanol (1.07 mL, 9.0 mmol) and 3-bromoquinoline (1.46 mL, 10.8 mmol) was added triphenylphosphine (472 mg, 1.8 mmol), CuBr (129 mg, 0.9 mmol) and $K_2CO_3$ (1.19g, 9 mmol), and the reaction mixture was heated to 100°C for 16 hours. The reaction mixture was then quenched with 10% aq. HCl, and the aqueous layer was washed with methylene chloride (4 x 15 ml). The aqueous layer was adjusted to approx. pH 14 with $K_2CO_3$ (solid) and extracted with methylene chloride (2 x 30 ml). The organic extracts were dried ($MgSO_4$) and concentrated to afford the crude product as an oil. Chromatographic purification (silica, $MeOH/CHCl_3$) afforded the product as a free base (571 mg, 26%), which was converted to the hydrochloride salt in a manner similar to Example 1b, using ether instead of ethanol. MS(DCl/NH3) m/e: 243 $(M+H)^+$; $^1H$-NMR ($D_2O$) δ: 8.79 (d, 1H), 8.14 (d, 1H), 8.09-8.0 (m, 2H), 7.86-7.73 (m, 2H), 4.67 (dd, 1H), 4.50 (dd, 1H), 4.02 (m, 1H), 3.80 (m, 1H), 3.30 (m, 1H), 3.10 (s, 3H), 2.53-2.38 (m, 1H), 2.30-2.09 (m, 2H). Anal. Calc for $C_{15}H_{18}N_2O \bullet 2HCl$: C, 57.15; H, 6.39; N, 8.89; Found: C, 57.14; H, 6.35; N, 8.90.

Example 31

4-(1-methyl-2-(S)-pyrrolidinylmethyloxy)isoquinoline hydrochloride

To (S)-1-methyl-2-pyrrolinyl methanol (1.07 mL, 9.0 mmol) and 4-bromoisoquinoline (2.24 g, 10.8 mmol) was added triphenylphosphine (472 mg, 1.8 mmol), CuBr (129 mg, 0.9 mmol) and $K_2CO_3$ (1.19g, 9 mmol), and the reaction mixture was heated to 100°C for 16 hours. The reaction mixture was then quenched with 10% aq. HCl and the aqueous layer was washed with methylene chloride (4 x 15 ml). The aqueous layer was then adjusted to approx. pH 14 with $K_2CO_3$ (solid) and extracted with methylene chloride (2 x 30 ml). The organic extracts were dried ($MgSO_4$) and concentrated to afford the crude product as an oil. Chromatographic purification (silica, $MeOH/CHCl_3$) gave the product as a free base (216 mg, 10%), which was converted to the hydrochloride salt in a manner similar to Example 1b, using ether instead of ethanol. MS(DCl/NH3) m/e: 243 $(M+H)^+$; $^1H$-NMR ($D_2O$) δ: 9.33 (s, 1H), 8.50-8.42 (m, 2H), 8.25-8.18 (m, 2H), 8.11-8.03 (m, 1H), 4.96-4.72 (m, 2H; partially buried under $H_2O$ peak), 4.16 (m, 1H), 3.84 (m, 1H), 3.25 (m, 1H), 3.17 (s, 3H), 2.57-2..46 (m, 1H), 2.36-2.16 (m, 2H). Anal. Calc for $C_{15}H_{18}N_2O \cdot 0.5\ H_2O \cdot 2HCl$: C, 55.56; H, 6.53; N, 8.64; Found: C, 55.63; H, 6.28; N, 8.50.

Example 32

6-Chloro-3-(1-(8-pyrrolizidinyl)methyloxy)pyridazine oxalate salt

32a. 1-Benzyloxycarbonyl-2-(methoxycarbonyl)-2-(3-propenyl)pyrrolidine

Under a nitrogen atmosphere, diisopropylamine (16.4 mL, 117.2 mmol) was dissolved in THF (117 mL) and cooled to -78°C. A 2.5 M solution of n-butyllithium in hexane (43 mL, 107.5 mmol) was then added dropwise to the solution. After stirring for 15 minutes, 1-benzyloxycarbonylproline methyl ester (25.7 g, 97.7 mmol) in THF (575 mL) was added to the reaction vessel dropwise over a 40 minute period. The reaction was allowed to stir for 15 minutes at -78°C, then neat allyl bromide (25.4 mL, 293 mmol) was added at a steady rate followed with stirring for 15 minutes at -78°C and for an additional 2 hours at between-35°C and -25°C. A phosphate buffer solution (~100 mL), pH=7, was next poured into the reaction vessel, and the reaction was allowed to warm to ambient temperature. The mixture was diluted with EtOAc and then washed in succession with 2 N HCl and brine. The organic phase was then dried over $Na_2SO_4$ and concentrated. The resulting residue was purified using flash chromatography on silica gel with EtOAc/ hexane (1:20 to 1:15 to 1:10) as the elutant to give 17.8 g of a yellow oil, 63% yield. TLC $R_f$=0.31 (EtOAc/hexane 1: 4). MS ($DCl/NH_3$) m/e 304 $(M+H)^+$. $^1H$ NMR (DMSO-$d_6$, 300 MHz) δ: 7.41-7.27 (m, 5H), 5.75-5.64 (m, 1H), 5.11-4.96 (m, 4H), 3.62-3.28 (m, 5H), 2.92 major conformer and 2.84 minor conformer (dd, J=14.0 Hz, 7.0 Hz, 1H), 2.59-2.50

(m, 1H partially buried under DMSO), 2.13-1.94 (m, 2H), 1.86-1.77 (m, 2H).

32b. 1-Benzyloxycarbonyl-2-(3-hydroxypropyl)-2-(methoxycarbonyl)pyrrolidine

The product from Example 32a (21.0 g, 69.4 mmol) was dissolved in THF (70 mL) and 1.0 M borane THF complex (45 mL, 45 mmol) and was added dropwise to the reaction vessel under a nitrogen atmosphere over a 40 minute period. The reaction was allowed to stir for one hour, then ~10 mL of water was carefully added followed by the successive addition of 3N NaOH (16.2 mL, 48.5 mmol) and 30% hydrogen peroxide (5.5 mL, 48.5 mmol). The mixture was allowed to stir for one hour and then was poured into a separatory funnel containing ~250 mL of water and ~15 mL 10% $Na_2S_2O_3$. The aqueous solution was extracted with methylene chloride, (X3) and the organics were combined and washed in succession with saturated sodium bicarbonate and brine. The organic phase was dried over sodium sulfate and then concentrated. The resulting residue was subject to flash chromatography on silica gel using EtOAc/hexane (1:4 to 1:2 to 1:1 to EtOAc) as the elutant to give 12.3 g of a clear viscous oil, 55% yield. TLC $R_f$=0.18 (EtOAc/hexane 1:1). MS ($DCl/NH_3$) m/e 322 $(M+H)^+$. $^1H$ NMR ($CDCl_3$, 300 MHz) δ: 7.37-7.29 (m, 5H), 5.18-5.06 (m, 2H), 3.82-3.45 (m, 7H), 2.41-1.20 (m, 8H).

32c. 1-Benzyloxycarbonyl-2-(methoxycarbonyl)-2-(3-methylsulfonyloxypropyl)-pyrrolidine

The product from Example 32b (10.7 g, 33.2 mmol) and triethylamine (4.9 mL, 34.9 mmol) were combined in THF (133 mL) and cooled to 0°C under $N_2$. Methanesulfonyl chloride (2.70 mL, 34.9 mmol) was added dropwise, and the reaction was stirred for 30 minutes at 0°C. Water was added, and the reaction vessel contents were poured into a separatory funnel. The mixture was washed successively with 10% citric acid solution and saturated sodium bicarbonate solution. The organic phase was dried over sodium sulfate, concentrated, and the residue was subjected to flash chromatography on silica gel using EtOAc/hexane (1:1 to 1:2) as the elutant to give 11.0 g of a clear oil, 83% yield. TLC $R_f$=0.25 (EtOAc/hexane 1:1). MS ($DCl/NH_3$) m/e 400 $(M+H)^+$. $^1H$ NMR ($CDCl_3$, 300 MHz) δ: 7.36-7.29 (m, 5H), 5.17-5.05 (m, 2H), 4.27-4.07 (m, 2H), 3.80-3.46 (m, 5H), 2.99 major conformer and 2.93 minor conformer (s, 3H), 2.35-1.59 (m, 8H).

32d. 8-(Methoxycarbonyl)pyrrolizidine

The product from Example 32c (11.0 g, 27.6 mmol) was exposed to hydrogen gas at a pressure of 4 atm in the presence of 11.0 g of dry 10% palladium on carbon in methanol for ~48 hours, monitored by TLC. The reaction was filtered when complete and the filtrate evaporated. The remaining crude was purified by flash chromatography on silica gel using 2% MeOH in $CHCl_3$ to 5% MeOH in $CHCl_3$ to 10% MeOH in $CHCl_3$ to give 4.23 g of a yellow oil, 90% yield. TLC $R_f$=0.53 (10% MeOH in $CHCl_3$). MS ($DCl/NH_3$) m/e 170 $(M+H)^+$. $^1H$ NMR ($CDCl_3$, 300 MHz) δ: 3.72 (s, 3H), 3.23 (ddd, J=9.9 Hz, 5.9 Hz, 5.9 Hz, 2H), 2.72 (ddd, J=9.9 Hz, 6.6 Hz, 6.6 Hz, 2H), 2.30 ("qt", J=6.0 Hz, 2H), 1.85-1.24 (m, 6H).

32e. 8-Hydroxypyrrolizidine

Lithium aluminum hydride (20.6 mL, 20.6 mmol) 1.0 M in THF was added dropwise to a solution of the product from Example 32d (2.9 g, 17.1 mmol), and the reaction was allowed to stir overnight at ambient temperature. A standard workup was done as that found in **Fieser and Fieser,** Vol. 1, p.584. The resulting crude was subject to a kugelrohr distillation (~55°C at 0.3mm) to give 1.93 g of a viscous clear oil, 80% yield. TLC $R_f$=0.40 ($CHCl_3$:MeOH:$NH_4OH$ 10: 4:1). MS ($DCl/NH_3$) m/e 142 $(M+H)^+$. $^1H$ NMR ($CDCl_3$, 300 MHz) δ: 3.27 (s, 2H), 3.03-2.95 (m, 2H), 2.66-2.58 (m, 2H), 1.85-1.64 (m, 6H), 1.61-1.52 (m, 2H).

32f. 6-Chloro-3-(1-(8-pyrrolizidinyl)methyloxy)pyridazine

The product from Example 32e (201 mg, 1.43 mmol) was added to a stirring slurry of sodium hydride (80% dispersion in mineral oil, 43 mg, 1.43 mmol) at room temperature. After 15 minutes 1,6 dichloropyridazine (320 mg, 2.14 mmol) was added and the reaction was stir for 16 hr. A solution of 2N HCl was added, then the mixture was extracted with EtOAc. The aqueous phase was basified with 2N NaOH solution and extracted with $CHCl_3$ (X2). The organics were combined, dried over $NaSO_4$, concentrated and subjected to flash chromatography using 10% MeOH in $CHCl_3$ to give 16.5 mg of a white solid, 4.5% yield. TLC $R_f$=0.10 (10% MeOH in $CHCl_3$). MS ($DCl/NH_3$) m/e 254 $(M+H)^+$. $^1H$ NMR ($CDCl_3$, 300 MHz) δ: 7.35 (d, J=9.0 Hz, 1H), 7.02 (d, J=9.0 Hz, 1H), 4.36 (s, 2H), 3.28-3.17 (m, 2H), 2.77-2.68 (m, 2H), 2.05-1.85 (m, 6H), 1.72-1.65 (m, 2H).

32g. 6-Chloro-3-(1-(8-pyrrolizidinyl)methyloxy)pyridazine oxalate salt

The product from Example 32f (16.5 mg, 0.065 mmol) and oxalic acid (6.5 mg, 0.068 mmol) were combined in THF at 0°C. After 30 minutes of stirring, the solvent was removed, and the remaining residue was triturated with diethyl ether (X3). The solid/oil was dried *in vacuo*. MS ($DCl/NH_3$) mle 254 $(M+H)^+$. $^1H$ NMR ($D_2O$, 300 MHz) δ: 7.76 (d, J=9.2 Hz, 1H), 7.38 (d, J=9.2 Hz, 1H), 4.62 (s, 2H), 3.70-3.61 (m, 2H), 3.32-3.24 (m, 2H), 2.37-2.07 (m, 8H). Anal. calc. for $C_{14}H_{18}ClN_3O_5$•$0.6C_2H_2O_4$•0.3•$H_2O$: C, 45.28; H, 4.95; N, 10.42. Found: C, 45.34; H, 5.02; N, 10.26.

Example 33

3-(1-(8-pyrrolizidinyl)methyloxy)pyrazine hydrochloride salt

33a. 3-(1-(8-pyrrolizidinyl)methyloxy)pyrazine

Sodium hydride (80% dispersion in mineral oil, 15.6 mg, 0.52 mmol) was slowly added to a solution of Example 32e (70.0 mg, 0.50 mmol) in THF (1.0 mL) at ambient temperature. The reaction was allowed to stir for 15 minutes, then 2-iodopyrazine (123 mg, 0.60 mmol) was added to the reaction vessel neat. The reaction was heated for 3 hours and then allowed to cool to ambient temperature. The reaction vessel contents were next partitioned between chloroform and saturated sodium bicarbonate solution. The phases were separated, and the aqueous phase was extracted with chloroform. The organics were combined , washed with brine, dried over $MgSO_4$, concentrated and subjected to flash chromatography on silica gel. The product was eluted with 10% MeOH in $CHCl_3$ to 0.2% $NH_4OH$ in 10% MeOH in $CHCl_3$ to give 37 mg of a gelatinous material, 34% yield. MS ($DCl/NH_3$) m/e 220 $(M+H)^+$. $^1H$ NMR ($CDCl_3$, 300 MHz) δ: 8.29 (d, J=1.1 Hz, 1H), 8.13 (d, J=2.6 Hz, 1H), 8.06 (dd, J=2.6 Hz, 1.1 Hz, 1H), 4.32 (s, 2H), 3.50-3.35 (m, 2H), 2.87-2.75 (m, 2H), 2.15-1.90 (m, 6H), 1.83- 1.73 (m, 2H).

33b. 3-(1-(8-pyrrolizidinyl)methyloxy)pyrazine hydrochloride salt

The product from Example 33a (32.0 mg, 0.15 mmol) was dissolved in diethyl ether and cooled to 0°C. Diethyl ether saturated with dry HCl was added to the reaction vessel dropwise with stirring. After 30 minutes of stirring, the solvent was removed, and the remaining white solid was triturated with diethyl ether and dried *in vacuo* to give 29.1 mg, 78% yield, of the title product. mp = 195-198°C dec. MS ($DCl/NH_3$) m/e 220 $(M+H)^+$. $^1H$ NMR ($D_2O$, 300 MHz) δ: 8.36 (s, 1H), 8.25-8.22 (m, 2H), 4.54 (s, 2H), 3.72-3.64 (m, 2H), 3.34-3.26 (m, 2H), 2.37-2.10 (m, 8H). Anal. calc. for $C_{12}H_{18}ClN_3O$: C, 56.35; H, 7.09; N, 16.43. Found: C, 56.11; H, 7.14; N, 16.15.

Example 34

2-(1-(8-pyrrolizidinyl)methyloxy)thiazole hydrochloride salt

34a. 2-(1-(8-pyrrolizidinyl)methyloxy)thiazole

The product from Example 32e (142.0 mg, 1.0 mmol) in THF (1mL) was added dropwise to sodium hydride (80% dispersion in mineral oil, 30 mg, 1.0 mmol) slurried in THF (2 mL). The slurry was allowed to stir for 15 minutes at ambient temperature, then 2-bromothiazole was added neat to the reaction vessel. The reaction was allowed to stir for 48 hours and then quenched with 2 N HCl. The mixture was extracted with EtOAc, and the aqueous phase was basified with aqueous 2 N sodium hydroxide solution. The basic solution was extracted with chloroform, and the solvent was removed *in vacuo.* The resulting crude product was purified by flash chromatography using 10% MeOH in $CHCl_3$ as the elutant to give 65 mgs of an oil, 29% yield. TLC $R_f$=0.14 (10% MeOH in $CHCl_3$). MS ($DCl/NH_3$) m/e 225 $(M+H)^+$. $^1H$ NMR ($CDCl_3$, 300 MHz) δ: 7.10 (d, J=4.0 Hz, 1H), 6.66 (d, J=4.0 Hz, 1H), 4.18 (s, 2H), 3.14-3.07 (m, 2H), 2.73 (ddd, J=10.3 Hz, 6.6 Hz, 6.6 Hz, 2H), 2.01-1.92 (m, 2H), 1.88-1.77 (m, 4H), 1.67-1.58 (m, 2H).

34b. 2-(1-(8-pyrrolizidinyl)methyloxy)thiazole hydrochloride salt

The product from Example 34a (60.0 mg, 0.27 mmol) was dissolved in diethyl ether and cooled to 0°C. Diethyl ether saturated with dry HCl was added dropwise to the reaction vessel with stirring. After 30 minutes the ether was decanted, and the remaining white solid was triturated with diethyl ether and dried *in vacua* to give 61 mg of the title product, 83% yield. mp = 109-111°C. MS ($DCl/NH_3$) m/e 225 $(M+H)^+$. $^1H$ NMR ($D_2O$, 300 MHz) δ: 7.19 (d, J=4.0 Hz, 1H), 7.00 (d, J=4.0 Hz,1H), 4.58 (s, 2H), 3.68-3.61 (m, 2H), 3.32-3.24 (m, 2H), 2.32-2.07 (m, 8H). Anal. calc. for $C_{11}H_{16}N_2OS\cdot 1.5\ HCl$: C, 47.35; H, 6.32; N, 10.04. Found: C, 47.55; H, 6.36; N, 10.01.

Example 35

(1R,4S)-3-(R)-(2-Thiazoloxymethyl)-N-methyl-2-azabicyclo[2.2.1]heptane hydrochloride

35a. (1R, 4S)-3-(R)-Carboethox-2-azabicyclo[2.2.1]heptane hydrochloride

A mixture of (1 R,4S)-3-(R)-carboethoxy-N-(S)-(-)-α-methylbenzyl-2-azabicyclo[2.2.1]hept-5-ene (2.40 g, 8.8 mmol, prepared according to the method of Stella et al., *Tetrahedron Lett.*, 31: 2603, **1990)** in ethanol (100 mL) and 20% Pd/C (dry, 1.2 g) was reduced under 4 atm of $H_2$ at room temperature for 12 hr. The solution was filtered and concentrated *in vauco* to give the free base as an oil (1.33 g). MS (DCl/$NH_3$) m/e: 170 $(M+H)^+$. $^1H$ NMR ($CDCl_3$, 300 MHz) δ: 4.18 (q, 2H), 3.57 (br. s, 1H), 3.34 (s, 1H), 2.63 (br. s, 1H), 2.12 (m, 2H), 1.68-1.28 (m, 5H), 1.28 (t, 3H). The resultant oil was dissolved in methylene chloride (ca. 20 mL) and upon addition of HCl/diethyl ether (ca. 6.25 M) a white solid precipitated and was collected. The solid was then recrystallized from EtOH/Et2O and dried under vacuum at 50°C to give the *title compound* (0.94 g, 52% yield). mp >200 °C.

35b. (1R.4S)-3-(R)-Carboethoxy-N-*t*-butylcarboxy-2-azabicyclo[2.2.1]heptane

To the compound of step 35a (1.50 g, 7.3 mmol) in methylene chloride (20 mL) at room temperature under nitrogen was added triethylamine (1.0 mL, 7.3 mmol). After 5 min di-*t*-butyldicarbonate (1.84 mL, 8.0 mmol) was added, and the reaction was stirred for 18 hr. The reaction was quenched by the addition of aqueous pH 4 buffer, and the mixture was extracted with diethyl ether (2 x 25 mL). The combined organic layers were dried over anhydrous sodium sulfate and concentrated *in vacua.* The crude product was purified by flash chromatography on silica gel (2000 g, ethyl acetate/hexane 1:4; $R_f$ = 0.45) to yield the title compound (1.49 g, 76%) as an oil. MS (DCl/$NH_3$) m/e: 270 $(M+H)^+$, 287 $(M+NH_4)^+$. $^1H$ NMR ($CDCl_3$, 300 MHz) δ: 4.28 (br. d, 1H), 4.18 (m, 2H), 3.78 (d, 1H), 2.67 (br. s, 1H), 1.94 (br. d, 1H), 1.80-1.40 (m, 5H), 1.44 (d, 9H), 1.28 (t, 3H).

35c. (1R,4S)-3-(R)-(Hydroxymethyl)-N-methyl-2-azabicyclo[2.2.1]heptane

To the compound of step 35b (1.40 g, 5.2 mmol) in anhydrous tetrahydrofuran (30 mL) under nitrogen was added lithiumaluminum hydride (0.60 g, 1.56 mmol). The resultant solution was heated at reflux for 2 hr, cooled to room temperature and quenched by the careful addition of sodium sulfate decahydrate. Diethyl ether (50 mL) and celite were added, and the mixture was stirred at room temperature for 1 hr and filtered thru a pad of celite (*ca.* 2 cm). The filtrate was concentrated *in vacuo* to give the title compound (0.79 g, 100%) as a white solid, mp 76-77°C. MS (DCl/$NH_3$) m/e: 142 $(M+H)^+$. $^1H$ NMR ($CDCl_3$, 300 MHz) δ: 3.42 (dd, 1H), 3.41 (dd, 1H), 3.21 (br s, 1H), 2.36 (s, 3H), 2.15 (d, 1H), 1.98 (t, 1H), 1.94-1.85 (m, 1H), 1.80-1.70 (m, 1H), 1.60-1.52 (m, 1H), 1.38-1.20 (m, 3H).

35d. (1R,4S)-3-(R)-(2-Thiazoloxymethyl)-*N*-methyl-2-azabicyclo[2.2.1]heptane hydrochloride

To the compound from step 35c (0.2 g, 1.4 mmol) in anhydrous tetrahydrofuran (10 mL) under nitrogen was added sodium hydride (60% dispersion, 0.06 g, 1.5 mmol) and 2-bromothiazole (0.19 mL, 2.1 mmol). The solution was allowed to reflux for 18 h, quenched by the addition of water (5 mL), and extracted with diethyl ether (2 x 50 mL) and ethyl acetate (50 mL). The combined organic layers were dried over anhydrous sodium sulfate and concentrated *in vacuo.* The crude product was purified by flash chromatography on silica gel (2500 g, ethanol/ethyl acetate 1:9; $R_f$ = 0.37) to give an oil, which was dissolved in ethyl acetate and precipitated by the addition of HCl/diethyl ether (6.25 M, 5 mL). The product was collected and dried under vacuum at 50°C to give the title compound (0.154 g, 42%) as a white solid, mp 121-123°C. MS (CDl/$NH_3$) m/e: 225 $(M+H)^+$. $^1H$ NMR (DMSO-$d_6$, 300 MHz) δ: 10.4 (br. s, 1H), 7.20 (d, 1H), 7.13 (d, 1H), 4.66-4.46 (m, 2H), 3.94 (br. s, 1H), 3.39-3.32 (m, 1H), 2.82 (d, 3H), 2.54 (br. s, 1H), 2.02-1.90 (m, 2H), 1.74-1.57 (m, 3H). IR (KBr) 3440, 2600, 2520, 1580, 1530, 1310, 1220, 980, 610 $cm^{-1}$. Anal. calc. for $C_{11}H_{17}ClN_2OS \cdot 0.5\ HCl \cdot 0.3\ H_2O$ : C, 46.45; H, 6.41; N, 9.85. Found: C, 46.57; H, 6.11; N, 9.86. $[\alpha]^{23}D$ = -29.2°. (c=0.25, MeOH).

Example 36

(1S,4R)-3-(S)-(2-Thiazoloxymethyl)-N-methyl-2-azabicyclo[2.2.1]heptane hydrochloride

36a. (1S,4R)-3-(S)-Carboethoxy-2-azabicyclo[2.2.1]hept-2-ene hydrochloride

(1S,4R)-3-(S)-(Carboethoxy)-N-(R)-(+)-α-methylbenzyl-2-azabicyclo[2.2.1]hept-5-ene (11.0 g, 40.5 mmol,

prepared by the method of Stella et al., *Tetrahedron Lett*., 31: 2603, **1990)** was reacted according to the method described in the preparation of the compound of Example 37a, to give the title compound (5.7 g, 68%) as a crystalline solid. mp >200°C.

36b. (1S,4R)-3-(S)-(Carboethoxy)-N-t-butylcarboxy-2-azabicyclo[2.2.1]heptane

The compound from step 36a (5.0 g, 24.4 mmol), di-*t*-butyldicarbonate (5.8 g, 26.8 mmol) and triethylamine (3.4 g, 24.4 mmol) were reacted according to the. method described in Example 37b to give the title compound (5.4 g, 82%) as an oil. MS ($DCl/NH_3$) m/e: 270$(M+H)^+$, 287$(M+NH_4)^+$. $^1$H NMR ($CDCl_3$, 300 MHz) δ: 4.28 (d, 1H), 4.18 (m, 2H), 3.75 (d, 1H), 2.67 (br. s, 1H), 1.94 (m, 1H), 1.80-1.40 (m, 5H), 1.44 (d, 9H), 1.28 (t, 3H).

36c. (1S,4R)-3-(S)-(Hydroxymethyl)-N-methyl-2-azabicyclo[2.2.1]heptane

The compound from step 36b (3.0 g, 11.1 mmol) and lithiumaluminum hydride (1.27 g, 33.4 mmol) were reacted according to the method described in Example 37c, gave the title compound (1.43 g, 92%) as a white solid. mp 75-76°C. MS($DCl/NH_3$) m/e: 142$(M+H)^+$, 140 $(M-H)^+$. $^1$H NMR ($CDCl_3$, 300 MHz) δ: 3.5-3.3 (m, 2H), 3.19 (br. s, 1H), 2.70 (br. s, 1H), 2.35 (s, 3H), 2.14 (d, 1H), 1.95 (t, 1H), 1.94-1.85 (m, 1H), 1.77-1.70 (m, 1H), 1.62-1.52 (m, 1H), 1.37-1.18 (m, 3H).

36d. (1S.4R)-3-(S)-(2-Thiazoxymethyl)-*N*-methyl-2-azabicyclo[2.2.1]heptane hydrochloride

The compound from step 36c (0.35 g, 2.5 mmol), 2-bromothîazole (0.33 mL, 3.7 mmol) and sodium hydride (60% dispersion, 0.11 g, 2.8 mmol) were reacted according to the procedure of Example 37d to give the title compound (154 mg, 23%) as a white solid. mp 120-123 °C. MS ($DCl/NH_3$) m/e: 225 $(M+H)^+$. $^1$H NMR (DMSO-$d_6$, 300 MHz) δ: 10.8 (br. s, 1H), 7.20 (d, 1H), 7.10 (d, 1H), 4.66-4.47 (m, 2H), 3.93 (br. s, 1H), 3.38-3.32 (m, 1H), 2.82 (d, 3H), 2.54 (br. s, 1H), 2.03-1.92 (m, 2H), 1.73-1.58 (m, 3H). IR (KBr) 3440, 2510, 1570, 1520, 1230, 990, 610 $cm^{-1}$. Anal. calc. for $C_{11}H_{17}ClN_2OS \cdot 0.4\ HCl \cdot 0.1\ H_2O$: C, 47.67; H, 6.40; N, 10.11. Found: C, 47.69; H, 6.20; N, 10.08. $[\alpha]^{23}_D$ = +28.5°. (c=0.25, MeOH).

Example 37

(1S,4R)-3-(S)-(3-Pyridyloxymethyl)-N-methyl-2-azabicyclo[2.2.1]heptane dihydrochloride

To a solution of triphenylphosphine (1.78 g, 6.8 mmol) in tetrahydrofuran (40 mL) under nitrogen was added *t*-butylazodicarboxylate (1.56 g, 6.8 mmol). The solution was stirred at 0°C for 20 min, and a THF solution (10 mL) of containing 3-hydroxypyridine (0.64 g, 6.8 mmol) and the compound (1S,4R)-3-(S)-(hydroxymethyl)-N-methyl-2-azabicyclo[2.2.1]heptane (0.64 g, 4.5 mmol, from Example 38c) was added. The reaction was allowed to warm to room temperature, stirred for 72 hr, then concentrated *in vacuo*. The residue was taken up in an aqueous solution of hydrochloric acid (10%, 100 mL) containing a small amount (*ca*.20 mL) of methylene chloride and stirred for 2 hr. The solution was washed with methylene chloride (1 x 50 mL). The aqueous layer was adjusted to pH 12 with 10% aqueous sodium hydroxide and exacted with methylene chloride (4 x 50 mL). The combined organic extracts were dried over anhydrous sodium sulfate and concentrated *in vauco.* The crude product was purified by flash chromatography on silica gel (2000g, methylene chloride/ethyl acetate/methanol/ammonium hydroxide 50:50:4:1; $R_f$ = 0.31) to give an oil (80 mg, 8.0%). The oil was dissolved in methylene chloride, and addition of HCl/diethyl ether (6.25M, 15mL) gave a white solid which was collected and dried under vacuum at 50°C to give the title compound as a deliquescent white solid. MS ($DCl/NH_3$) m/e: 219 $(M+H)^+$. $^1$H NMR (DMSO-$d_6$, 300MHz) δ: 11.35 (br. s, 1H), 8.75 (d, 1H), 8.53 (d, 1H), 8.18 (m, 1H), 7.38 (dd, 1H), 4.48 (m, 2H), 3.88 (br. s, 1H), 3.43-3.36 (m, 1H), 2.85 (d, 3H), 2.45 (br. s, 1H), 2.15 (d, 1H), 1.95 (m, 1H), 1.80-1.60 (m, 4H). IR (KBr) 3260, 3180, 1650, 1550, 1380, 1000, 810 cm-1; Anal. calc. for $C_{13}H_{20}Cl_2N_2O \bullet 0.7\ NH_4Cl \bullet 0.3H_2O$: C, 46.74 H, 7.06; N, 11.32. Found : C, 46.90; H, 7.33; N, 11.24.

Example 38

(1R,4S)-3-(R)-(3-Pyridyloxymethyl)-N-methyl-2-azabicyclo[2.2.1]heptane dihydrochloride

Following the procedure of Example 37, replacing (1S,4R)-3-(S)-(hydroxymethyl)-N-methyl-2-azabicyclo [2.2.1]heptane with (1R,4S)-3-(R)-(hydroxymethyl)-N-methyl-2-azabicyclo[2.2.1]heptane (from Example 37c), the title compound is prepared.

Example 39

5-Chloro-3-(2-(R)-pyrrolidinylmethyloxy)pyridine dihydrochloride

39a. (R)-1-t-butoxycarbonyl-2-pyrrolidinemethanol

N-t-BOC-(R)-proline was treated as in Example 14a. The resulting material was carried on without any further purification.

39b. 5-Chloro-3-(N-t-butoxycarbonyl-2-(R)-pyrrolidinylmethyloxy)pyridine

Starting with the material from step 39a, and following the procedure of Example 15b, the title compound was prepared. TLC $R_f$ 0.75 (1:1 EtOAc/Hex). MS ($DCI/NH_3$) m/e: 313 $(M + H)^+$ with $^{35}Cl$ and m/e: 315 $(M + H)^+$ with $^{37}Cl$.

39c. 5- Chloro-3-(2-(R)-pyrrolidinylmethyloxy)pyridine dihydrochloride

The product of Example 39b was treated according to the procedures of Examples 2b and 1b to give the title product. The MS and $^1H$ NMR ($D_2O$, 300 MHz) were similar to Example 15d. Anal. Calc. for $C_{10}H_{13}N_2OCl{\cdot}2.00$ HCl: C, 42.06; H, 5.29; N, 9.81; Found C, 42.91; H, 5.44; N, 9.86. $[\alpha]^{25}_D$=-11.15° (c=1, MeOH).

Example 40

5-Chloro-3-(1-methyl-2-(R)-pyrrolidinylmethyloxy)pyridine dihydrochloride

40a. 5-Chloro-3-(1-methyl-2-(R)-pyrrolidinylmethyloxy)pyridine

Replacing 5-chloro-3-(N-t-butoxycarbonyl-2-(S)-pyrrolidinylmethyloxy)-pyridine with 5-chloro-3-(N-t-butoxycarbonyl-2-(R)-pyrrolidinylmethyloxy)pyridine (prepared from the (R)-isomer of the starting material as in Example 15a), the title compound was prepared in a manner similar to Example 16a TLC $R_f$=.23 (10%MeOH/$CHCl_3$). MS and $^1H$ NMR ($CDCl_3$, 300 MHz) are similar to 16a.

40b. 5-Chloro-3-(1-methyl-(R)-pyrrolidinylmethyloxy)pyridine dihydrochloride

The product of Example 40a was treated with HCl and isolated as described in Example 1b to give a white powder. MS and $^1H$ NMR ($D_2O$, 300 Hz) are similar to 16b. . Anal. Calc for $C_{11}H_{15}N_2OCl{\cdot}2.00$ HCl: C, 44.10; H, 5.20; N, 9.35; Found C, 43.98; H, 5.81; N, 9.33. $[\alpha]^{25}_D$=$^+$5.59°(c=1, MeOH).

Example 41

2-Methyl-3-(2-(R)-pyrrolidinylmethyloxy)pyridine dihydrochloride

41a. 2-Methyl-3-(2-(R)-pyrrolidinylmethyloxy)pyridine

Replacing (5)-1-t-butoxycarbonyl-2-pyrrolidinemethanol with (R)-1-t-butoxycarbonyl-2-pyrrolidinemethanol (Aldrich Chemical Co.), the title compound was prepared in a manner similar to Example 17, steps a and b. MS (DCI/$NH_3$) and $^1H$ NMR ($CDCl_3$, 300 MHz) are similar to 17a

41 b. 2-Methyl-3-(2-(R)-pyrrolidinylmethyloxy)pyridine dihydrochloride

The product of Example 41a was treated with HCl and isolated as described in Example 1b to give a white powder. MS and $^1H$ NMR (D20, 300 Hz) are similar to 17b. Anal. Calc for $C_{11}H_{16}N_2O{\cdot}2.00$ HCl: C, 48.82; H, 6.84; N, 10.56; Found C, 49.55; H, 6.95; N, 10.52. $[\alpha]^{25}_D$=$^-$27.01° (c=1, MeOH).

Example 42

6-Methyl-3-(1-melhyl-2-(R)-pyrrolidinylmethyloxy)pyridine dihydrochloride

42a. 6-Methyl-3-(1-t-butoxycarbonyl-2-(R)-pyrrolidinylmethyloxy)pyridine

Replacing 6-methyl-3-(1-t-butoxycarbonyl-2-(S)-pyrrolidinylmehtyloxy)-pyridine with 6-methiyl-3-(1-t-butoxycarbonyl-2-(R)-pyrrolidinylmethyloxy)pyridine, prepared from (R)-1-t-butoxycarbonyl-2-pyrrolldinemethanol (Aldrich Chemical Co.) as described in Example 19a, the title compound was prepared in a manner similar to Example 20a TLC $R_f$=.42 (1:1 EtOAc/Hex.).

42b. 6-Methyl-3-(1-methyl-2-(R)-pyrrolidinylmethyloxy)pyridine

The title compound was prepared in a manner similar to 20b TLC $R_f$=.17 (10%MeOH/$CHCl_3$). MS (DCl/$NH_3$) and $^1$H NMR ($CDCl_3$, 300 MHz) are similar to 20b

42c. 6-Methyl-3-(1-methyl-2-(R)-pyrrolidinylmethyloxy)pyridine dihydrochloride

The product of Example 42b was treated with HCl and isolated as described in Example 1b to give a white powder. MS and $^1$H NMR ($D_2O$, 300 Hz) are similar to 20c. Anal. Calc for $C_{12}H_{18}N_2O$·2.00 HCl: C, 51.62; H, 7.22; N, 10.03; Found C, 51.36; H, 7.53; N, 9.93. $[\alpha]^{25}_D$=$^+$6.22° (c=1, MeOH).

Example 43

6-Methyl-3-(2-(R)-pyrrolidinylmethyloxy)pyridine dihydrochloride

The product of Example 45a was treated as described in Examples 19a and 19b to give a white powder. MS (DCl/$NH_3$) and d$^1$H NMR ($D_2O$, 300 Mz) are similar to 19b Analysis calculated for $C_{11}H_{16}N_2O$·2.00 HCl: C, 49.82; H, 6.84; N, 10.56; Found C, 49.89; H, 6.59; N, 10.33. $[\alpha]^{25}_D$=-10.47° (c=1, MeOH).

Example 44

3-(1-Ethyl-2(S)-pyrrolidinylmethyloxy)pyridine dihydrochloride

44a. 3-(1-Ethyl-2-oxo-5-(S)-pyrrolidinylmethyloxy)pyridine

To a solution of the compound from example 22a (1.50 g, 7.8 mmol) in anhydrous THF at 0°C was added NaH (60% yield dispersion, 0.625g, 15.6 mmol) was added, and the reaction mixture was stirred for 20 minutes at this temperature. The reaction was then warmed to room temperature, and iodoethane (1.25 ml, 15.6 mmol) was added via syringe. After starting material was consumed, $NaHCO_3$ was added to the reaction followed by $CHCl_3$. The desired compound was extracted from the aqueous phase, and the organic layer was subjected to a brine wash (2X). The organic layer was dried over $MgSO_4$. The residue was purified by silica gel flash chromatography (5% yieldMeOH/$CHC_3$) to give 0.23 g (13% yield) of the title compound as an oil. MS (DCl/$NH_3$) m/e 221 $(M + H)^+$ and 238 $(M + NH_4^+)^+$. $^1$H NMR ($CDCl_3$, 300 MHz) δ: 8.39-8.33 (m, 1H), 8.33-8.28 (m, 1H), 7.39-7.34 (m, 2H), 4.20-4.00 (m, 3H), 3.78-3.64 (m, 1H), 3.22-3.07 (m, 1H), 2.63-2.49 (m, 1H), 2.47-2.23 (m, 2H), 2.06-1.91 (m, 1H).

44b. 3-(1-Ethyl-2-(S)-pyrrolidinylmethyloxy)pyridine

To the product of Example 44a (222 mg, 1.01 mmol) in 3 mL of THF was added, under nitrogen and dropwise over a period of 5 minutes, 3.03 ml. (3.03 mmol) of a 1 M solution of borane in THF. After stirring under reflux for 3 hours, methanol was added dropwise, and the reaction was stirred for an additional 30 minutes. The solvent was removed *in vacua,* affording a white solid borane complex. This solid was dissolved in anhydrous ethanol. Cesium fluoride (0.347g, 3.03 mmol) was added, and the resultant solution was stirred under reflux for 16 hr. Evaporation of the solvent provided a white solid which was purified on a silica gel column, eluting with chloroform:methanol (10:1) to give 162 mg of the desired methyl compound as an oil in 78% yield. MS (DCl/$NH_3$) m/e 207 $(M + H)^+$, $^1$H NMR ($CDCl_3$, 300 MHz) δ: 8.36-8.31 (m, 1H), 8.28-8.21 (m, 1H), 7.26-7.20 (m, 2H), 4.30-4.10 (m 1H), 4.02-3.90 (m, 1H), 3.43-3.23 (m, 1H), 3.20-2.90 (m, 2H), 2.68-2.28 (m, 2H), 2.16-1.76 (m, 2H), 1.72-1.44 (m, 1H).

44c. 3-(1-Ethyl-2-(S)-pyrrolidinylmethyloxy)pyridine dihydrochloride

The free base from example 44b was dissolved in diethyl ether and brought to 0°C with stirring. The solution was treated with diethyl ether saturated with HCl. The solvent was removed *in vacuo*. The resulting salt was triturated with diethyl ether (2X) and dried under vacuum to give a white powder. MS ($DCl/NH_3$) m/e207 $(M + H)^+$. $^1H$ NMR ($D_2O$, 300 MHz) δ: 8.49 (d, J=3.0 Hz, 1H), 8.39 (dd, J=1.0,5.1 Hz, 1H), 8.02 (m, 1H), 7.85 (dd, J=8.70, 5.40 Hz, 1H), 4.61 (dd, J=11.0, 3.3 Hz, 1H), 4.44 (dd, J=11.0, 6.6 Hz, 1H), 4.10-4.01 (m, 1H), 3.79-3.72 (m, 1H), 3.65-3.51 (m, 2H), 3.32-3.18 (m, 2H), 2.44-2.33 (m, 1H), 2.27-2.05 (m, 2H), 1.37 (t, J=7.5 Hz, 3H). Anal. Calc. for $C_{12}H_{18}N_2O \cdot 2.0\ HCl$: C, 51.57; H, 7.16; N, 10.02; Found C, 51.43; H, 7.39; N, 9.96. $[\alpha]_D$ = -1.5° (c 0.46, MeOH).

Example 45

5-Chloro-3-(2-(S)-azetidinylmethyloxy)pyridine dihydrochloride

45a. 5-Chloro-3-(N-*t*-Butoxycarbonyl-2-(S)-azetidinylmethyloxy)pyridine

An ice-cooled solution of the product from Example 7b (0.242g, 1.20 mmol) was allowed to react with 3-chloro-5-hydroxypyridine (0.187 g, 1.40 mmol) under the conditions of Example 2a, except that DEAD was replaced with di-*t*-butylazodicarbonate, to yield the title compound (0.137 g, 88%) after purification on silica gel (ethyl acetate/hexane 2:1). MS ($DCl/NH_3$) m/e: 299 $(M+H)^+$. $^1H$ NMR ($CDCl_3$, 300 MHz) δ: 8.25 (d, J=1.38 Hz, 1H), 8.21 (br. s, 1H), 7.29 (t, J=2.2 Hz, 1H), 4.53-4.51 (m, 1H), 4.34-4.33 (m, 1H), 4.13 (dd, J=10.3, 2.9 Hz, 1H), 3.91-3.86 (m, 2H), 2.51 (s, 3H), 2.38-2.29 (m, 2H), 1.43 (s, 9H).

45b. 5-Chloro-3-(2-(S)-azetidinylmethyloxy)pyridine dihydrochloride

The product from step 45a (0.130 g, 0.44 mmol) was treated with saturated ethanolic HCl (5 mL) for 16 hr. The volatiles were removed *in vacuo,* and the dihydrochloride was recrystallized ($EtOH/Et_2O$) to yield the title compound (0.094g, 80%) as a white solid. mp 156-157°C. MS ($DCl/NH_3$) m/e: 199 $(M+H)^+$, 216 $(M+NH_4)^+$. $^1H$ NMR ($D_2O$, 300 MHz) δ: 8.41 (d, J=5.1 Hz, 1H), 8.39 (d, J=4.4 Hz, 1H), 7.94 (t, J=2.1 Hz, 1H), 5.01-4.93 (m, 1H), 4.50 (d, J=4.0 Hz, 2H), 4.20-4.03 (m, 2H), 2.69 (q, J=8.45 Hz, 2H). Anal. calc. for $C_9H_{13}Cl_3N_2O \cdot 0.5H_2O$: C, 38.53; H, 5.03; N, 9.98 Found: C, 38.51; H, 5.16 N, 9.96. $[\alpha]^D{}_{23}$ =-3.23° (c=0.16 in MeOH).

Example 46

4-Methyl-3-(2-(S)-azetidinylmethyloxy)pyridine dihydrochloride

46a. 4-Methyl-3-(N-*t*-Butoxycarbonyl-2-(S)-azetidinylmethyloxy)pyridine

An ice cooled solution of the product from Example 7b (0.232g, 1.24 mmol) was allowed to react with 3-hydroxy-6-methylpyridine (0.142 g, 1.30 mmol) under the conditions of Example 2a, except that DEAD was replaced with di-*t*-butylazodicarbonate to yield the title compound (0.123 g, 36%) after purification on silica gel (ethyl acetate/hexane 2:1). MS ($DCl/NH_3$) m/e: 279 $(M+H)^+$. $^1H$ NMR ($CDCl_3$, 300 MHz) δ: 8.23-8.22 (d, J=2.6 Hz, 1H), 7.20 (dd, J=8.5, 3.0 Hz, 1H), 7.08 (d, J=8.5 Hz, 1H), 4.51-4.49 (m, 1H), 4.30-4.28 (m, 1H), 4.13 (dd, J=9.9 ,2.9 Hz, 1H), 3.89 (t, J=7.75 Hz, 2H), 2.51 (s, 3H), 2.37-2.28 (m, 2H), 1.41 (s, 9H).

46b. 4-Methyl-3-(2-(S)-azetidinylmethyloxy)pyridine dihydrochloride

The product from step 46a (0.123 g, 0.44 mmol) was treated with saturated ethanolic HCl (5 mL) for 18 hr. The volatiles were removed *in vacuo*, and the dihydrochloride was washed with $Et_2O$ (3 x 20 mL), evaporated to dryness and recrystallized ($EtOH/Et_2O$) to yield the title compound (0.074g, 63%) as a white solid. mp 141-144°C. MS ($DCl/NH_3$) m/e: 179 $(M+H)^+$. $^1H$ NMR ($D_2O$, 300 MHz) δ: 8.33 (d, J=2.9 Hz, 1H), 7.89 (dd, J=9.0, 2.8 Hz, 1 H), 7.64 (d J=8.8 Hz, 1H), 5.01-4.93 (m, 1H), 4.48 (d, J=4.4 Hz, 2H), 4.21-4.04 (m, 2H), 2.70 (q, J=8.5 Hz, 2H), 2.62 (s, 3H). Anal. calc. for $C_{10}H_{16}Cl_2N_2O \cdot 1.0H_2O$: C, 44.62; H, 6.74; N, 10.41. Found: C, 44.55; H, 7.02; N, 10.50. $[\alpha]^D$24-7.89° (c=0.19 in MeOH).

Example 47

2-Methyl-3-(2-(R)-azetidinylmethyloxy)pyridine dihydrochloride

47a. 1-*t*-Butoxycarbonyl-2-(R)-hydroxymethylazetidine

An ice cooled solution of 2-(R)-azetidine carboxylic acid (0.400 g, 3.96 mmol, preparation as described by Miyoshi *et al., Chemistry Lett.*, **1973**: 5) was allowed to react under the conditions described in Example 7a to yield the protected acid (0.237 g, 30%) which was used without further purfication. This crude product was allowed to reaction under the conditions described in Example 7b to yield the title compound, which was used without further purification. MS (CDI/$NH_3$) m/e: 188 $(M+H)^+$, 205 $(M+NH_4)^+$. $^1H$ NMR ($CDCl_3$, 300 MHz) δ: 4.46-4.42 (m, 1H), 3.92-3.69 (m, 2H), 2.21-2.12 (m, 2H), 1.99-1.87 (m, 2H).

47b. 2-Methyl-3-(N-*t*-Butoxycarbonyl-2-(R)-azetidinylmethyloxy)pyridine

An ice cooled solution of the product from step 47a (0.151g, 0.81 mmol) was allowed to react with 2-methyl-3-hydroxypyridine (0.0.092 g, 0.85 mmol) under the conditions of Example 2a, except that DEAD was replaced by di-*t*-butyl azodicarbonate, to yield the title compound (0.125 g, 55%). MS (DCl/$NH_3$) m/e: 279 $(M+H)^+$. $^1H$ NMR ($CDCl_3$, 300 MHz) δ: 8.11 (dd, J=4.6, 1.3 Hz, 1H), 7.20-7.10 (m, 2H), 4.54-4.53 (m, 1H), 4.36-4.35 (m, 1H), 4.09 (dd, J=10.0, 2.6 Hz, 1H), 3.95-3.88 (m, 2H), 2.55 (s, 3H), 2.42-2.30 (m, 2H), 1.40 (s, 9H).

47c. 2-Methyl-3-(2-(R)-azetidinylmethyloxy)pyridine dihydrochloride

The product from step 47b (0.121 g, 0.435 mmol) was treated with saturated ethanolic HCl (5 mL). After 16 hr, the volatiles were removed *in vacuo*, and the dihydrochloride was recrystallized (EtOH/$Et_2O$) to yield the title compound (0.098 g, 90%) as a hygroscopic oil. MS (DCl/$NH_3$) m/e: 179 $(M+H)^+$. $^1H$ NMR ($D_2O$, 300 MHz) δ: 8.16 (d, J=5.5 Hz, 1H), 7.79 (d, J=8.5 Hz. 1H), 7.62 (dd, J=8.5, 5.5 Hz, 1H), 5.00 (m, 1H), 4.57-4.47 (m, 2H), 4.24-4.12 (m, 2H), 2.78-2.71 (m, 2H). Anal. calc. for $C_{10}H_{16}ClN_2O \cdot 0.5H_2O$: C, 46.17 H, 6.59 N, 10.77. Found: C, 45.93; H, 6.61 N, 10.63. $[\alpha]^D 23$ -5.85° (c=0.21 in MeOH).

Example 48

3-(1-Methyl-2-(R)-pipecolinylmethyloxy) pyridine dihydrochloride

48a. (R)-N-(*t*-butyloxycarbonyl)-pipecolinic acid

A 10.44 g (80.9 mmol) sample of (R)-pipecolinic acid, previously resolved according to Hemingway,R. J., *J. Pharm. Pharmac.*, 20, 87-91, **(1968),** was dissolved in 70 mL of dioxane and 40 mL of $H_2O$, and 40 mL of 1M $K_2CO_3$ and 39 mL of di-t-butyldicarbonate were added. The reaction was stirred at room temperature for 16 hr, then an additional 10 mL of di-*t*-butyldicarbonate and 40 mL of 1 M $K_2CO_3$ were added and the reaction continued for another 24 hr. The solvents were removed on a rotary evaporator, 10% citric acid solution was added to the residue, and the mixture was extracted with $CHCl_3$. The extract was dried over $MgSO_4$, filtered and concentrated to give the title product as a white solid. MS: 247 $(M+NH_4)^+$, 230 $(M+H)^+$, 191 $(M-C_4H_8+NH_4)^+$.

48b. 3-(1-Methyl-2-(R)-hydroxymethylpiperidine

To the product from Example 48a (1.06 g, 4.63 mmol) in anhydrous THF (10 mL) under $N_2$ at 0°C was added $LiAlH_4$ (1 M in diethyl ether; 15 mL). The reaction was allowed to stir for 16 hr, and additional $LiAlH_4$ (8 mL) was added. The reaction was quenched by the addition of sodium sulfate decahydrate (60 mg), filtered through celite and washed with THF. The combined organics were concentrated *in vacuo* to yield the title compound as an oil (0.552 g, 93%). This material was carried forward without further purification. MS (CDI/$NH_3$) m/e: 130 $(M+H)^+$. 1H NMR ($CDCL_3$, 300 MHz) δ: 3.86 (dd, 1H), 3.41 (dd, 1H), 2.88 (m, 1H), 2.32 (s, 3H), 2.18 (m, 2H), 1.98 (m, 1H), 1.76 (m, 1H), 1.62 (m, 2H), 1.51 (m, 1H), 1.29 (m, 1H).

48c. 3-(1-Methyl-2-(R)-pipecolinylmethyloxy)pyridine

The product from step 48b (553 mg, 4.28 mmol) was allowed to react with 3-bromopyridine (0.43 mL, 4.50 mmol), cuprous bromide (0.165 g, 0.86 mmol), triphenylphosphine (0.449 g, 1.7 mmol) and potassium carbonate (0.592

g, 4.28 mmol). The reaction mixture was heated to 90°C and stirred for 120 hr, then cooled to 25 °C, acidified with HCl (1.5 M; 35 mL) and washed with ethyl acetate (4 x 50 mL). The aqueous layer was basified with saturated aqueous potassium carbonate, and the product was extracted with chloroform (6 x 50 mL), dried ($MgSO_4$) and concentrated *in vacuo* to an oil. The crude product was purified by chromatography on silica gel ($CH_2Cl_2$/EtOAc/MeOH/$NH_4OH$ 50:50: 4:1) to yield the free base of the title compound (0.048 g). The amine was treated as in example 1b to yield the title compound as a hygroscopic semisolid (0.024 g, 2%). MS (DCI/$NH_3$) m/e: 207 $(M+H)^+$. $^1H$ NMR ($D_2O$, 300 MHz) δ: 8.37-8.29 (m, 2H), 7.66 (dd, J=8.45,1.5 Hz, 1H), 7.59-7.57 (m, 1 H), 4.63 (dt, J=14.5, 2.4 Hz, 2H), 4.8 (dd, J=11.9, 1.8 Hz, 1H), 3.58-3.53 (m, 2H), 3.20 (m, 1H), 2.93 (s, 3H), 2.05-1.60 (m, 5H). Anal. calc. for $C_{12}H_{20}Cl_2N_2O \cdot 2.0\ H_2O$: C, 45.72; H, 7.67; N, 8.89. Found: C, 45.82; H, 7.93; N, 8.84. $[\alpha]_D^{23}$ -1.14 ° (c=0.71 in MeOH).

## Claims

**1.** A compound having the formula:

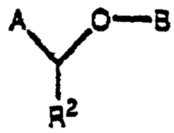

or a pharmacautically-acceptable salt or prodrug thereof, wherein:

A is selected from the group consisting of:

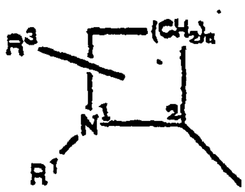

(i)

wherein n is 1, 2, or 3;

$R^1$ is H or $C_1$-$C_6$-alkyl; and
$R^3$ is H or, when n is 2, $R^3$ is any of H,
one substituent at the 4-position of the N- containing ring and is O-W, wherein W is H, $C_1$-$C_3$-alkyl, $CH_2OH$, $CH_2O$-methyl, Br, Cl, or F, or
two substituents, one of which being a substituent at the 4-position of the N- containing ring and being O-W wherein W is as defined above, and the other one being a substituent at the 5-position of the N-containing ring and being $C_1$-$C_3$-alkyl.

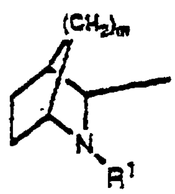

(ii) ,

wherein m is 1 or 2 and $R^1$ is as defined above;

(iii) 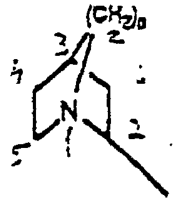,

wherein p is 1 or 2; 2,

(iv) 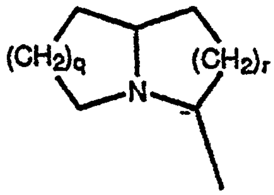,

wherein q and r are independently 0, 1 or 2, except that q and r may not concurrently be 0; and

(v) 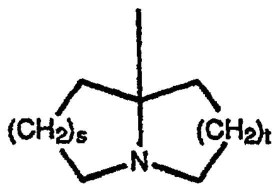,

wherein s and t are independently 0, 1 or 2, except that s and t may not concurrently be 0;

$R^2$ is H or $C_1$-$C_6$-alkyl; and
B is selected from the group consisting of:

(i) 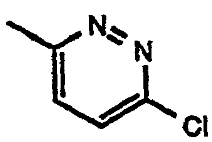;

(ii) 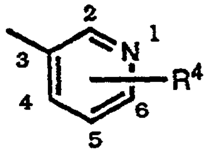,

wherein $R^4$ is H or is mono-substituted at the 2-, 4-, 5- or 6-position with hydroxyl, $C_1$-$C_3$-alkyl or $C_1$-$C_3$-alkoxy; or is mono-substituted at the 4-, 5- or 6- position with Br, Cl or F;

(iii) 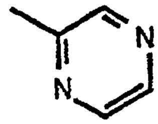 ;

(iv) 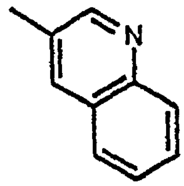 ;

(v) 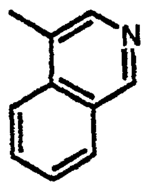 ;

(vi) 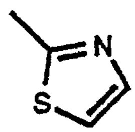 ;

and

(vii) 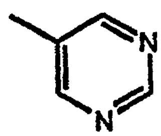 ;

with the provisos that when A is selected from option (iii), then B must be selected from options (ii) and (vi); or when B is selected from option (ii) with $R^4$ being 2-methyl, and A is selected from option (i), then $R^1$ must be H.

**2.** A compound according to Claim 1, wherein A is selected from options (i), (ii) and (v); B is selected from options (ii) and (iv); n is 1 or 2, $R^1$ and $R^2$ are H, or $R^1$ is methyl and $R^2$ is H.

**3.** A compound according to Claim 1, wherein A is selected from option (i), B is selected from option (ii), $R^3$ is H, n is 1 or 2, $R^1$ and $R^2$ are H, or $R^1$ is methyl and $R^2$ is H.

**4.** A compound according to Claim 1, which is:

3-(1-Methyl-2-(R)-pyrrolidinylmethyloxy)pyridine;
3-(2-(R)-Pyrrolidinylmethyloxy)pyridine;
2-(1-Methyl-2-(S)-pyrrolidinylmethyloxy)pyrazine;

3-(1-Methyl-2-(S)-pyrrolidinylmethyloxy)pyridine;
2-(1-Methyl-2-(R)-pyrrolidinylmethyloxy)-6-chloropyridazine;
1-(Methyl)-2-(S)-(1-(3'-pyridyloxy)ethyl)pyrrolidine;
2-(2-(S)-Azetidinylmethyloxy)pyrazine;
2-(1-Methyl-2-(S)-azetidinylmethyloxy)pyrazine;
3-(2-(S)-Azetidinylmethyloxy)pyridine;
3-(1-Methyl-2-(S)-azetidinylmethyloxy)pyridine;
2-(1-Methyl-2-(S)-pyrrolidinylmethyloxy)thiazole;
2-(1-Methyl-2-(S)-pyrrolidinylmethyloxy)-6-chloropyridazine;
6-Chloro-3-(1-Methy)-2(S)-methyloxyazetidinyl)pyridazine;
3-(2-(S)-Pyrrolidinylmethyloxy)pyridine;
5-Chloro-3-(2-(S)-pyrrolidinylmethyloxy)pyridine;
5-Chloro-3-(1-methyl-2-(S)-pyrrolidinylmethyloxy)pyridine;
2-Methyl-3-(2-(S)-pyrrolidinylmethyloxy)pyridine;
6-Methyl-3-(2-(S)-pyrrolidinylmethyloxy)pyridine;
6-Methyl-3-(1-methyl-2-(S)-pyrrolidinylmethyloxy)pyridine;
4-Bromo-3-(2-(S)-pyrrolidinylmethyloxy)pyridine;
4-Bromo-3-(1-methyl-2-(S)-pyrrolidinylmethyloxy)pyridine;
3-(1-Methyl-5-methyl-2-(S)-pyrrolidinylmethyloxy)pyridine;
3-(*trans*-1-Methyl-4-hydroxy-2(S)-pyrrolidinylmethyloxy)pyridine;
3-(*trans*-1,4-Dimethyl-2(S)-pyrrolidinylmethyloxy)pyridine;
3-(*trans*-1-Methyl-4-ethyl-2(S)-pyrrolidinylmethyloxy)pyridine;
3-(1-Methyl-2-plpecoilnylmethyloxy)pyridine;
4-Methyl-3-(2-(S)-pyrrolidinylmethyloxy)pyridine;
5-Bromo-3-(1-methyl-2-(S)-pyrrolidinylmethyloxy)pyridine;
2-Methyl-3-(2-(S)-azetidinylmethyloxy)pyridine;
3-(1-Methyl-2-(S)-pyrrolidinylmethyloxy)quinoline;
4-(1-Methyl-2-(S)-pyrrolidinylmethyloxy)isoquinoline;
6-Chloro-3-(1-(8-pyrrolizidinyl)methyloxy)pyridazine;
3-(1-(8-Pyrrolizidinyl)methyloxy)pyrazine;
2-(1-(8-Pyrrolizidinyl)methyloxy)thiazole;
(1R,4S)-3-(R)-(2-Thiazoloxymethyl)-N-methyl-2-azabicyclo[2.2.1]heptane;
(1S,4R)-3-(S)-(2-Thiazoloxymethyl)-N-methyl-2-azabicyclo[2.2.1]heptane;
(1S,4R)-3-(S)-(3-Pyridyloxymethyl)-N-methyl-2-azabicyclo[2.2.1]heptane;
(1R,4S)-3-(R)-(3-Pyridyloxymethyl)-N-methyl-2-azabicyclo[2.2.1]heptane;
5-Chloro-3-(2-(R)-pyrrolidinylmethyloxy)pyridine;
5-Chloro-3-(1-methyl-2-(R)-pyrrolidinylmethyloxy)pyridine;
2-Methyl-3-(2-(R)-pyrrolidinylmethyloxy)pyridine;
6-Methyl-3-(1-methyl-2-(R)-pyrrolidinylmethyloxy)pyridine;
6-Methyl-3-(2-(R)-pyrrolidinylmethyloxy)pyridine;
3-(1-Ethyl-2(S)-pyrrolidinylmethyloxy)pyridine;
5-Chloro-3-(2-(S)-azetidinylmethyloxy)pyridine;
4-Methyl-3-(2-(S)-azetidinylmethyloxy)pyridine;
2-Methyl-3-(2-(R)-azetidinylmethyloxy)pyridine; or
3-(1-Methyl-2-(R)-pipecolinylmethyloxy) pyridine.

**5.** A compound according to Claim 1, which is:

3-(1-Methyl-2-(R)-pyrrolidinylmethyloxy)pyridine;
3-(2-(R)-Pyrrolidinytmethyloxy)pyridine;
3-(1-Methyl-2-(S)-pyrrolidinylmethyloxy)pyridine;
1-(Methyl)-2-(S)-(1-(3'-pyridyloxy)ethyl)pyrrolidine;
3-(2-(S)-Azetidinylmethyloxy)pyridine;
3-(1-Methyl-2-(S)-azetidinylmethyloxy)pyridine;
3-(2-(S)-Pyrrolidinylmethyloxy)pyridine;
5-Chloro-3-(2-(S)-pyrrolidinylmethyloxy)pyridine;
5-Chloro-3-(1-methyl-2-(S)-pyrrolidinylmethyloxy)pyridine;
2-Methyl-3-(2-(S)-pyrrolidinylmethyloxy)pyridine;

2-Methyl-3-(1-methyl-2-(S)-pyrrolidinylmethyloxy)pyridine;
6-Methyl-3-(2-(S)-pyrrolidinylmethyloxy)pyridine;
6-Methyl-3-(1-methyl-2-(S)-pyrrolidinylmethyloxy)pyridine;
4-Bromo-3-(1-methyl-2-(S)-pyrrolidinyimethyloxy)pyridine;
3-(1-Methyl-5-methyl-2-(S)-pyrrolidinylmethyloxy)pyridine;
3-(*trans*-1,4-Dimethyl-2(S)-pyrrolidinylmethyloxy)pyridine;
3-(*trans*-1-Methyl-4-ethyl-2(S)-pyrrolidinylmethyloxy)pyridine;
4-Methyl-3-(2-(S)-pyrrolidinylmethyloxy)pyridine;
5-Bromo-3-(1-methyl-2-(S)-pyrrolidinylmethyloxy)pyridine;
2-Methyl-3-(2-(S)-azetidinylmethyloxy)pyridine;
3-(1-Methyl-2-(S)-pyrrolidinylmethyloxy)quinoline;
(1S,4R)-3-(S)-(3-Pyridyloxymethyl)-N-methyl-2-azabicyclo[2.2.1]heptane;
(1R,4S)-3-(R)-(3-Pyridyloxymethyl)-N-methyl-2-azabicyclo[2.2.1]heptane;
5-Chloro-3-(2-(R)-pyrrolidinylmethyloxy)pyridine;
5-Chloro-3-(1-methyl-2-(R)-pyrrolidinylmethyloxy)pyridine;
6-Methyl-3-(1-methyl-2-(R)-pyrrolidinylmethyloxy)pyridine;
6-Methyl-3-(2-(R)-pyrrolidinylmethyloxy)pyridine;
3-(1-Ethyl-2(S)-pyrrolidinylmethyloxy)pyridine; or
5-Chloro-3-(2-(S)-azetidinylmethyloxy)pyridine.

**6.** A compound according to Claim 1, which is:

3-(2-(R)-Pyrrolidinylmethyloxy)pyridine;
3-(1-Methyl-2-(S)-pyrrolidinylmethyloxy)pyridine;
3-(2-(S)-Azetidinylmethyloxy)pyridine;
3-(1-Methyl-2-(S)-azetidinylmethyloxy)pyridine;
3-(2-(S)-Pyrrolidinylmethyloxy)pyridine;
5-Chloro-3-(2-(S)-pyrrolidinylmethyloxy)pyridine;
5-Chloro-3-(1-methyl-2-(S)-pyrrolidinylmethyloxy)pyridine;
6-Methyl-3-(2-(S)-pyrrolidinylmethyloxy)pyridine;
6-Methyl-3-(1-methyl-2-(S)-pyrrolidinylmethyloxy)pyridine;
5-Bromo-3-(1-methyl-2-(S)-pyrrolidinylmethyloxy)pyridine;
2-Methyl-3-(2-(8)-azetidinylmethyloxy)pyridine;
5-Chloro-3-(2-(R)-pyrrolidinylmethyloxy)pyridine;
6-Methy(-3-(2-(R)-pyrrolidinylmethyloxy)pyridine; or
5-Chloro-3-(2-(S)-azetidinylmethyloxy)pyridine.

**7.** A pharmaceutical composition for treating cognitive, neurological, and mental disorders **characterized by** decreased cholinergic function, comprising a pharmaceutically-acceptable carrier and a therapeutically-effective amount of a compound according to Claim 1.

**8.** Use of compound according to claim 1, for manufacturing a medicament for selectively activating neuronal nicotinic acetylcholine receptors in a patient in need of such treatment.

**9.** Use of a compound according to Claim 1, for manufacturing a medicament for treating dementias, attention-deficit disorder, anxiety associated with cognitive impairment or substance abuse withdrawal **characterized by** decreased cholinergic function in a patient in need of such treatment.

**Patentansprüche**

**1.** Eine Verbindung mit der Formel:

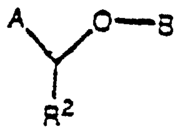

oder ein pharmazeutisch verträgliches Salz oder Prodrug davon, worin:

A gewählt ist aus der Gruppe bestehend aus:

(I) 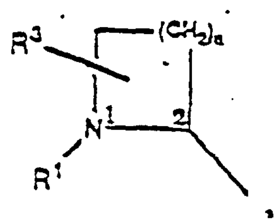 ,

worin n 1, 2 oder 3 ist;

$R^1$ H oder $C_1$-$C_6$-Alkyl ist; und
$R^3$ H ist, oder, wenn n 2 ist, ist $R^3$ irgendeines von H,
einem Substituenten an der 4-Position des N-enthaltenden Ringes, der O-W ist, worin W H, $C_1$-$C_3$-Alkyl, $CH_2OH$, $CH_2O$-Methyl, Br, Cl oder F ist,
oder zwei Substiuenten, wobei einer ein Substituent an der 4-Position des N-enthaltenden Ringes und O-W ist, worin W wie oben definiert ist, und der andere ein Substituent an der 5-Position des N- enthaltenden Ringes und $C_1$-$C_3$-Alkyl ist.

(II) 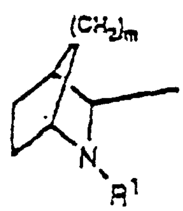 ,

worin m 1 oder 2, und $R^1$ wie oben definiert ist;

(III) 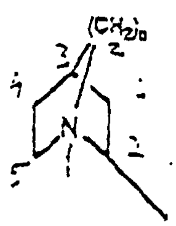 ,

worin p 1 oder 2 ist;

(iv) 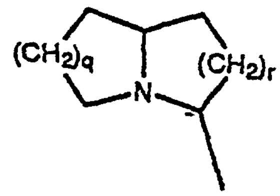 ,

worin q und r unabhängig voneinander 0, 1 oder 2 sind, außer, daß q und r nicht gleichzeitig 0 sein können; und

(v) 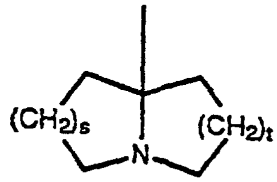 ,

worin s und t unabhängig voneinander 0, 1 oder 2 sind außer, daß s und t nicht gleichzeitig O sein können;

$R^2$ ist H oder $C_1$-$C_6$-Alkyl; und
B ist gewählt aus der Gruppe bestehend aus:

(i) 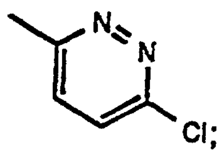

(ii) 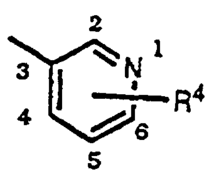 ,

worin $R^4$ H ist, oder mono-substituiert an der 2-, 4-, 5- oder 6- Position mit Hydroxyl, $C_1$-$C_3$-Alkyl oder $C_1$-$C_3$-Alkoxy; oder mono-substituiert an der 4-, 5- oder 6-Position mit Br, Cl oder F;

(iii) 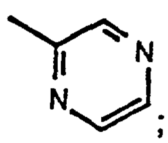

(iv) 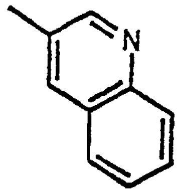 ;

(v) 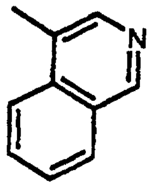 ;

(vi) 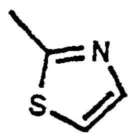 ;

und

(vii) 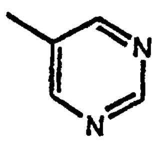 ;

unter den Bedingungen daß, wenn A aus Alternative (iii) gewählt ist, dann muß B aus den Alternativen (ii) und (vi) gewählt sein; oder wenn B aus der Alternative (ii) gewählt ist, wobei $R^4$ 2-Methyl ist, und A aus Alternative (i) gewählt ist, dann muß $R^1$ H sein.

2. Eine Verbindung gemäß Anspruch 1, worin A aus den Alternativen (i), (ii) und (v) gewählt ist; B ist gewählt aus den Alternativen (ii) und (iv); n ist 1 oder 2, $R^1$ und $R^2$ sind H, oder $R^1$ ist Methyl und $R^2$ ist H.

3. Eine Verbindung gemäß Anspruch 1, worin A aus Alternative (i) gewählt ist, B aus Alternative (ii) gewählt ist, $R^3$ ist H, n ist 1 oder 2, $R^1$ und $R^2$ sind H, oder $R^1$ ist Methyl und $R^2$ ist H.

4. Eine Verbindung gemäß Anspruch 1, die folgendermaßen ist:

3-(1-Methyl-2-(R)-pyrrolidinylmethyloxy)pyridin;
3-(2-(R)-Pyrrolidinylmethyloxy)pyridin;
2-(1-Methyl-2-(S)-pyrrolidinylmethyloxy)pyrazin;
3-(1-Methyl-2-(S)-pyrrolidinylmethyloxy)pyridin;
2-(1-Methyl-2-(R)-pyrrolidinylmethyloxy)-6-chlorpyridazin;
1-(Methyl)-2-(S)-(1-(3'-pyridyloxy)ethyl)pyrrolidin;
2-(2-(S)-Azetidinylmethyloxy)pyrazin;

2-(1-Methyl-2-(S)-azetidinylmethyloxy)pyrazin;
3-(2-(S)-Azetidinylmethyloxy)pyridin;
3-(1-Methyl-2-(S)-azetidinylmethyloxy)pyridin;
2-(1-Methyl-2-(S)-pyrrolidinylmethyloxy)thiazol;
2-(1-Methyl-2-(S)-pyrrolidinylmethyloxy)-6-chlorpyridazin;
6-Chlor-3-(1-methyl-2(S)-methyloxyazetidinyl)pyridazin;
3-(2-(S) -Pyrrolidinylmethyloxy)pyridin;
5-Chlor-3-(2-(S)-pyrrolidinylmethyloxy)pyridin;
5-Chlor-3-(1-methyl-2-(S)-pyrrolidinylmethyloxy)pyridin;
2-Methyl-3-(2-(S)-pyrrolidinylmethyloxy)pyridin;
6-Methyl-3-(2-(S)-pyrrolidinylmethyloxy)pyridin;
6-Methyl-3-(1-methyl-2-(S) -pyrrolidinylmethyloxy)pyridin;
4-Brom-3-(2-(S)-pyrrolidinylmethyloxy)pyridin;
4-Brom-3-(1-methyl-2-(S)-pyrrolidinylmethyloxy)pyridin;
3-(1-Methyl-5-methyl-2-(S)-pyrrolidinylmethyloxy)pyridin;
3-(*trans*-1-Methyl-4-hydroxy-2(S)-pyrrolidinylmethyloxy)pyridin;
3-(*trans*-1,4-Dimethyl-2(S)-pyrrolidinylmethyloxy)pyridin;
3-(*trans*-1-Methyl-4-ethyl-2(S)-pyrrolidinylmethyloxy)pyridin;
3-(1-Methyl-2-pipecolinylmethyloxy)pyridin,
4-Methyl-3-(2-(S)-pyrrolidinylmethyloxy)pyridin;
5-Brom-3-(1-methyl-2-(S)-pyrrolidinylmethyloxy)pyridin;
2-Methyl-3-(2-(S)-azetidinylmethyloxy)pyridin;
3-(1-Methyl-2-(S)-pyrrolidinylmethyloxy)chinolin;
4-(1-Methyl-2-(S)-pyrrolidinylmethyloxy)isochinolin;
6-Chlor-3-(1-(8-pyrrolizidinyl)methyloxy)pyridazin;
3-(1-(8-Pyrrolizidinyl)methyloxy)pyrazin;
2-(1-(8-Pyrrolizidinyl)methyloxy)thiazol;
(1R,4S)-3-(R)-(2-Thiazoloxymethyl)-N-methyl-2-azabicyclo[2.2.1]heptan;
(1S,4R)-3-(S)-(2-Thiazoloxymethyl)-N-methyl-2-azabicyclo[2.2.1] heptan;
(1S,4R)-3-(S)-(3-Pyridyloxymethyl)-N-methyl-2-azabicyclo[2.2.1]heptan;
(1R,4S)-3-(R)-(3-Pyridyloxymethyl)-N-methyl-2-azabicyclo[2.2.1]heptan;
5-Chlor-3-(2-(R)-pyrrolidinylmethyloxy)pyridin;
5-Chlor-3-(1-methyl-2-(R)-pyrrolidinylmethyloxy)pyridin;
2-Methyl-3-(2-(R)-pyrrolidinylmethyloxy)pyridin;
6-Methyl-3- (1-methyl-2-(R) -pyrrolidinylmethyloxy)pyridin;
6-Methyl-3-(2-(R)-pyrrolidinylmethyloxy)pyridin;
3-(1-Ethyl-2(S)-pyrrolidinylmethyloxy)pyridin;
5-Chlor-3-(2-(S)-azetidinylmethyloxy)pyridin;
4-Methyl-3-(2-(S)-azetidinylmethyloxy)pyridin;
2-Methyl-3-(2-(R)-azetidinylmethyloxy)pyridin; oder
3-(1-Methyl-2-(R)-pipecolinylmethyloxy)pyridin.

**5.** Eine Verbindung gemäß Anspruch 1, die folgendermaßen ist:

3-(1-Methyl-2-(R)-pyrrolidinylmethyloxy)pyridin;
3-(2-(R)-Pyrrolidinylmethyloxy)pyridin,
3-(1-Methyl-2-(S)-pyrrolidinylmethyloxy)pyridin;
1-(Methyl)-2-(S)-(1-(3'-pyridyloxy)ethyl)pyrrolidin;
3-(2- (S) -Azetidinylmethyloxy)pyridin;
3-(1-Methyl-2-(S)-azetidinylmethyloxy)pyridin;
3-(2-(S)-Pyrrolidinylmethyloxy)pyridin;
5-Chlor-3-(2-(S)-pyrrolidinylmethyloxy) pyridin;
5-Chlor-3-(1-methyl-2-(S)-pyrrolidinylmethyloxy)pyridin,
2-Methyl-3-(2-(S)-pyrrolidinylmethyloxy)pyridin;
2-Methyl-3-(1-methyl-2-(S)-pyrrolidinylmethyloxy)pyridin;
6-Methyl-3-(2-(S)-pyrrolidinylmethyloxy)pyridin;
6-Methyl-3-(1-methyl-2-(S)-pyrrolidinylmethyloxy)pyridin;
4-Brom-3-(1-methyl-2-(S)-pyrrolidinylmethyloxy)pyridin;

3- (1-Methyl-5-methyl-2-(S) -pyrrolidinylmethyloxy)pyridin;
3-(*trans*-1,4-Dimethyl-2(S) -pyrrolidinylmethyloxy) pyridin;
3-(*trans*-1-Methyl-4-ethyl-2(S)-pyrrolidinylmethyloxy)pyridin;
4-Methyl-3-(2-(S)-pyrrolidinylmethyloxy)pyridin;
5-Brom-3-(1-methyl-2-(S)-pyrrolidinylmethyloxy)pyridin;
2-Methyl-3-(2-(S)-azetidinylmethyloxy)pyridin;
3-(1-Methyl-2-(S)-pyrrolidinylmethyloxy)chinolin;
(1S,4R)-3-(S)-(3-Pyridyloxymethyl)-N-methyl-2-azabicyclo[2.2.1]heptan;
(1R,4S)-3-(R) - (3-Pyridyloxymethyl)-N-methyl-2-azabicyclo[2.2.1]heptan;
5-Chlor-3-(2-(R)-pyrrolidinylmethyloxy)pyridin;
5-Chlor-3-(1-methyl-2-(R)-pyrrolidinylmethyloxy)pyridin;
6-Methyl-3-(1-methyl-2-(R)-pyrrolidinylmethyloxy)pyridin;
6-Methyl-3-(2-(R)-pyrrolidinylmethyloxy)pyridin;
3-(1-Ethyl-2(S)-pyrrolidinylmethyloxy)pyridin; oder
5-Chlor-3-(2-(S)-azetidinylmethyloxy)pyridin.

**6.** Eine Verbindung gemäß Anspruch 1, die folgendermaßen ist:

3-(2-(R)-Pyrrolidinylmethyloxy)pyridin;
3-(1-Methyl-2-(S)-pyrrolidinylmethyloxy)pyridin;
3-(2-(S)-Azetidinylmethyloxy)pyridin;
3-(1-Methyl-2-(S)-azetidinylmethyloxy)pyridin;
3-(2-(S)-Pyrrolidinylmethyloxy)pyridin;
5-Chlor-3-(2-(S)-pyrrolidinylmethyloxy) pyridin;
5-Chlor-3-(1-methyl-2-(S)-pyrrolidinylmethyloxy)pyridin;
6-Methyl-3-(2-(S)-pyrrolidinylmethyloxy)pyridin;
6-Methyl-3-(1-methyl-2-(S)-pyrrolidinylmethyloxy)pyridin;
5-Brom-3-(1-methyl-2-(S)-pyrrolidinylmethyloxy)pyridin;
2-Methyl-3-(2-(S)-azetidinylmethyloxy)pyridin;
5-Chlor-3-(2-(R)-pyrrolidinylmethyloxy)pyridin;
6-Methyl-3-(2-(R) -pyrrolidinylmethyloxy)pyridin; oder
5-Chlor-3-(2-(S)-azetidinylmethyloxy)pyridin.

**7.** Eine pharmazeutische Zusammensetzung zur Behandlung von kognitiven, neurologischen und mentalen Erkrankungen **gekennzeichnet durch** verminderte cholinerge Funktion, die einen pharmazeutisch verträglichen Träger und eine therapeutisch wirksame Menge einer Verbindung gemäß Anspruch 1 umfaßt.

**8.** Verwendung einer Verbindung gemäß Anspruch 1, zur Herstellung eines Medikamentes zur selektiven Aktivierung neuronaler nikotinischer Acetylcholinrezeptoren in einem Patienten, der eine solche Behandlung benötigt.

**9.** Verwendung einer Verbindung gemäß Anspruch 1, zur Herstellung eines Medikamentes zur Behandlung von Demenzen, Konzentrationsstörungen, Angst aufgrund von kognitiver Beeinträchtigung oder Entzug bei Substanzenmißbrauch, charkterisiert durch verminderte cholinerge Funktion, in einem Patienten, der eine solche Behandlung benötigt.

**Revendications**

**1.** Composé de formule :

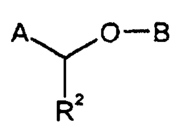

ou un sel ou promédicament acceptable en pharmacie d'un tel composé,
formule dans laquelle :

A est choisi dans l'ensemble constitué par :

(i) 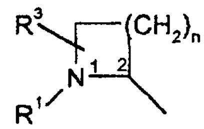

où n vaut 1, 2 ou 3 ;

$R^1$ est H ou un radical alkyle en $C_1$ à $C_6$ ; et
$R^3$ est H ou, quand n vaut 2, $R^3$ est l'un quelconque parmi H, un substituant en position 4 du cycle azoté et est O-W, où W est H, Br, Cl, F ou un radical alkyle en $C_1$ à $C_3$, $CH_2OH$ ou $CH_2O$-méthyle, ou deux substituants, l'un étant un substituant en position 4 du cycle azoté et était O-W où W est tel que défini ci-dessus, et l'autre étant un substituant en position 5 du cycle azote et étant un radical alkyle en $C_1$ à $C_3$ ;

(ii) 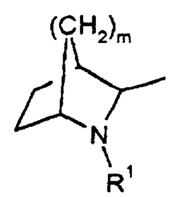

où m vaut 1 ou 2 et $R^1$ est tel que défini ci-dessus ;

(iii) 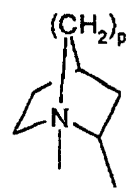

où p vaut 1 ou 2 ;

(iv) 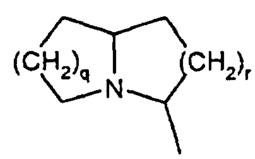

où q et r valent indépendamment 0, 1 ou 2, sauf que q et r ne peuvent pas être en même temps 0 ; et

(v) 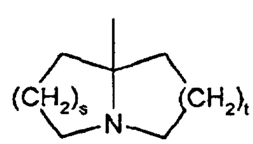

où s et t valent indépendamment 0, 1 ou 2, sauf que s et t ne peuvent pas être en même temps 0 ;

$R^2$ est H ou un radical alkyle en $C_1$ à $C_6$ ; et
B est choisi dans l'ensemble constitué par :

(i) 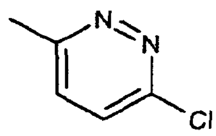

(ii) 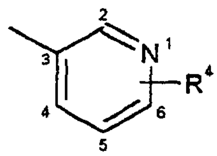

où $R^4$ est H ou est mono-substitué en position 2, 4, 5 ou 6 par un radical hydroxyle, alkyle en $C_1$ à $C_3$ ou alcoxy en $C_1$ à $C_3$ ; ou est mono-substitué en position 4, 5 ou 6 par Br, Cl ou F ;

(iii) 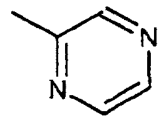

(iv) 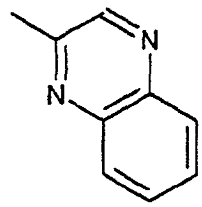

(v) 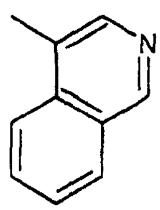

(vi) 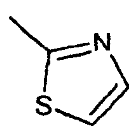

et

(vii) 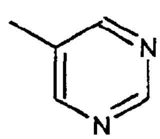

avec pour conditions que lorsque A est choisi parmi l'option (iii), alors B doit être choisi parmi les options (ii)

et (vi) ; ou lorsque B est choisi parmi l'option (ii) où $R^4$ est un radical 2-méthyle, et A est choisi parmi l'option (i), alors $R^1$ doit être H.

**2.** Composé selon la revendication 1, dans lequel A est choisi parmi les options (i), (ii) et (v) ; B est choisi parmi les options (ii) et (iv) ; n vaut 1 ou 2, $R^1$ et $R^2$ sont H, ou $R^1$ est le radical méthyle et $R^2$ est H.

**3.** Composé selon la revendication 1, dans lequel A est choisi parmi l'option (i), B est choisi parmi l'option (ii), $R^3$ est H, n vaut 1 ou 2, $R^1$ et $R^2$ sont H, ou $R^1$ est le radical méthyle et $R^2$ est H.

**4.** Composé selon la revendication 1, qui est l'un des suivants :

3-(1-méthyl-2-(R)-pyrrolidinylméthyloxy)pyridine ;
3-(2-(R)-pyrrolidinylméthyloxy)pyridine ;
2-(1-méthyl-2-(S)-pyrrolidinylméthyloxy)pyrazine ;
3- (1-méthyl-2-(S)-pyrrolidinylméthyloxy)pyridine ;
2-(1-méthyl-2-(R)-pyrrolidinylméthyloxy)-6-chloropyridazine ;
1-(méthyl)-2-(S)-(1-(3'-pyridyloxy) éthyl)pyrrolidine
2-(2-(S) -azétidinylméthyloxy)pyrazine ;
2-(1-méthyl-2-(S)-azétidinylméthyloxy)pyrazine ;
3-(2-(S)-azétidinylméthyloxy)pyridine ;
3-(1-méthyl-2-(S)-azétidinylméthyloxy)pyridine ;
2- (1-méthyl-2-(S)-pyrrolidinylméthyloxy)thiazole ;
2-(1-méthyl-2-(S)-pyrrolidinylméthyloxy)-6-chloropyridazine ;
6-chloro-3-(1-méthyl-2-(S)-méthyloxyazétidinyl)pyridazine ;
3-(2-(S)-pyrrolidinylméthyloxy)pyridine ;
5-chloro-3-(2-(S)-pyrrolidinylméthyloxy)pyridine ;
5-chloro-3-(1-méthyl-2-(S)-pyrrolidinylméthyloxy)pyridine;
2-méthyl-3-(2-(S)-pyrrolidinylméthyloxy)pyridine ;
6-méthyl-3-(2-(S)-pyrrolidinylméthyloxy)pyridine ;
6-méthyl-3- (1-méthyl-2-(S)-pyrrolidinylméthyloxy)pyridine ;
4-bromo-3-(2-(S)-pyrrolidinylméthyloxy)pyridine ;
4-(bromo-3-(1-méthyl-2-(S)-pyrrolidinylméthyloxy)pyridine ;
3-(1-méthyl-5-méthyl-2-(S)-pyrrolidinylméthyloxy)pyridine ;
3-(trans-1-méthyl-4-hydroxy-2-(S)-pyrrolidinylméthyloxy)pyridine ;
3-(trans-1,4-diméthyl-2-(S)-pyrrolidinylméthyloxy)pyridine ;
3-(trans-1-méthyl-4-éthyl-2(S)-pyrrolidinylméthyloxy)pyridine ;
3-(1-méthyl-2-pipécolinylméthyloxy)pyridine ;
4-méthyl-3-(2-(S)-pyrrolidinylméthyloxy)pyridine ;
5-bromo-3-(1-méthyl-2-(S)-pyrrolidinylméthyloxy)pyridine;
2-méthyl-3-(2-(S)-azétidinylméthyloxy)pyridine;
3-(1-méthyl-2-(S)-pyrrolidinylméthyloxy)quinoline ;
4-(1-méthyl-2-(S)-pyrrolidinylméthyloxy)isoquinoline ;
6-chloro-3-(1-(8-pyrrolizidinyl)méthyloxy)pyridazine ;
3-(1-(8-pyrrolizidinyl)méthyloxy)pyrazine ;
2-(1-(8-pyrrolizidinyl)méthyloxy)thiazole ;
(1R, 4S)-3- (R) - (2-thiazoloxyméthyl)-N-méthyl-2-azabicyclo[2.2.1]heptane ;
(1S,4R)-3-(S)-(2-thiazoloxyméthyl)-N-méthyl-2-azabicyclo[2.2.1]heptane ;
(1S, 4R) -3- (S) - (3-pyridyloxyméthyl)-N-méthyl-2-azabicyclo[2.2.1]heptane ;
(1R,4S)-3-(R)-(3-pyridyloxyméthyl)-N-méthyl-2-azabicyclo[2.2.1]heptane ;
5-chloro-3-(2-(R)-pyrrolidinylméthyloxy)pyridine ;
5-chloro-3-(1-méthyl-2-(R)-pyrrolidinylméthyloxy)pyridine ;
2-méthyl-3-(2-(R)-pyrrolidinylméthyloxy)pyridine ;
6-méthyl-3-(1-méthyl-2-(R)-pyrrolidinylméthyloxy)pyridine ;
6-méthyl-3-(2-(R)-pyrrolidinylméthyloxy)pyridine ;
3-(1-éthyl-2-(S)-pyrrolidinylméthyloxy)pyridine ;
5-chlore-3-(2-(S)-azétidinylméthyloxy)pyridine ;
4-méthyl-3-(2-(S)-azétidinylméthyloxy)pyridine ;
2-méthyl-3-(2- (R)-azétidinylméthyloxy)pyridine ; ou

3- (1-méthyl-2-(R) -pipécolinylméthyloxy) pyridine.

**5.** Composé selon la revendication 1, qui est l'un des suivants :

3-(1-méthyl-2-(R)-pyrrolidinylméthyloxy)pyridine ;
3-(2-(R)-pyrrolidinylméthyloxy)pyridine ;
3-(1-méthyl-2-(S)-pyrrolidinylméthyloxy)pyridine ;
1-(méthyl)-2-(S)-(1-(3'-pyridyloxy)éthyl)pyrrolidine ;
3-(2-(S)-azétidinylméthyloxy)pyridine ;
3-(1-méthyl-2-(S)-azétidinylméthyloxy)pyridine ;
3-(2-(S) -pyrrolidinylméthyloxy)pyridine;
5-chloro-3-(2-(S)-pyrrolidinylméthyloxy)pyridine ;
5-chloro-3-(1-méthyl-2-(S)-pyrrolidinylméthyloxy)pyridine ;
2-méthyl-3-(2-(S)-pyrrolidinylméthyloxy)pyridine ;
2-méthyl-3-(1-méthyl-2-(S)-pyrrolidinylméthyloxy)pyridine ;
6-méthyl-3-(2-(S)-pyrrolidinylméthyloxy)pyridine ;
6-méthyl-3-(1-méthyl-2-(S)-pyrrolidinylméthyloxy)pyridine ;
4-bromo-3-(1-méthyl-2-(S)-pyrrolidinylméthyloxy)pyridine ;
3-(1-méthyl-5-méthyl-2-(S)-pyrrolidinylméthyloxy)pyridine ;
3-(trans-1,4-diméthyl-2-(S)-pyrrolidinylméthyloxy)pyridine ;
3- (trans-1-méthyl-4-éthyl-2-(S)-pyrrolidinylméthyloxy)pyridine ;
4-méthyl-3-(2-(S)-pyrrolidinylméthyloxy)pyridine ;
5-bromo-3-(1-méthyl-2-(S)-pyrrolidinylméthyloxy)pyridine ;
2-méthyl-3-(2-(S)-azétidinylméthyloxy)pyridine ;
3-(1-méthyl-2-(S)-pyrrolidinylméthyloxy)quinoline ;
(1S, 4R) -3-(S)-(3-pyridyloxyméthyl)-N-méthyl-2-azabicyclo[2.2.1]heptane ;
(1R, 4S)-3-(S)-(3-pyridyloxyméthyl)-N-méthyl-2-azabicyclo[2.2.1]heptane ;
5-chloro-3-(2-(R)-pyrrolidinylméthyloxy) pyridine ;
5-chloro-3-(1-méthyl-2-(R)-pyrrolidinylméthyloxy)pyridine ;
6-méthyl-3-(1-méthyl-2-(R)-pyrrolidinylméthyloxy)pyridine ;
6-méthyl-3- (2-(R)-pyrrolidinylméthyloxy)pyridine ;
3-(1-éthyl-2-(S)-pyrrolidinylméthyloxy)pyridine ; ou 5-chloro-3-(2-(S)-azétidinylméthyloxy)pyridine.

**6.** Composé selon la revendication 1, qui est l'un des suivants :

3-(2-(R)-pyrrolidinylméthyloxy)pyridine ;
3-(1-méthyl-2-(S)-pyrrolidinylméthyloxy)pyridine ;
3-(2-(S)-azétidinylméthyloxy)pyridine ;
3-(1-méthyl-2-(S)-azétidinylméthyloxy)pyridine;
3-(2-(S)-pyrrolidinylméthyloxy)pyridine ;
5-chloro-3-(2- (S) -pyrrolidinylméthyloxy) pyridine;
5-chloro-3-(1-méthyl-2-(S)-pyrrolidinylméthyloxy)pyridine ;
6-méthyl-3-(2-(S)-pyrrolidinylméthyloxy)pyridine ;
6-méthyl-3-(1-méthyl-2-(S)-pyrrolidinylméthyloxy)pyridine ;
5(bromo-3-(1-méthyl-2-(S)-pyrrolidinylméthyloxy)pyridine ;
2-méthyl-3-(2- (S)-azétidinylméthyloxy)pyridine ;
5(chloro-3-(2-(R)-pyrrolidinylméthyloxy)pyridine ;
6-méthyl-3-(2- (R) -pyrrolidinylméthyloxy)pyridine ; ou
5-chloro-3-(2-(S) -azétidinylméthyloxy)pyridine.

**7.** Composition pharmaceutique pour traiter des troubles cognitifs, neurologiques et mentaux, **caractérisés par** une diminution de la fonction cholinergique, comprenant un véhicule acceptable en pharmacie et une quantité efficace, du point de vue thérapeutique, d'un composé selon la revendication 1.

**8.** Utilisation d'un composé selon la revendication 1, pour fabriquer un médicament destiné à activer sélectivement des récepteurs d'acétylcholine nicotiniques neuronaux chez un patient nécessitant un tel traitement.

**9.** Utilisation d'un composé selon la revendication 1, pour fabriquer un médicament destiné à traiter des démences,

un trouble de déficit de l'attention, une anxiété associée à une défaillance cognitive ou un sevrage d'une drogue, **caractérisés par** une diminution de la fonction cholinergique, chez un patient nécessitant un tel traitement.